# EUROPEAN PATENT APPLICATION

(11) **EP 3 682 833 A1**
(43) Date of publication of application: **22.07.2020**
(21) Application number: 18852893.9
(22) Date of filing: 10.09.2018
(51) Int. Cl.: A61B 18/14

(54) **HIGH-FREQUENCY TREATMENT IMPLEMENT, HIGH-FREQUENCY TREATMENT IMPLEMENT KNIFE, AND HIGH-FREQUENCY TREATMENT IMPLEMENT TIP END PROCESSING IMPLEMENT**

(30) Priority: 11.09.2017 JP 2017174238; 11.09.2017 JP 2017174239; 12.04.2018 JP 2018076776; 12.04.2018 JP 2018076777
(71) Applicant: Sumitomo Bakelite Co., Ltd., Shinagawa-ku Tokyo 140-0002 (JP)
(72) Inventor: ITAMI Yasuhito, Akita-shi Akita 011-8510 (JP); IKEDA Masao, Akita-shi Akita 011-8510 (JP); ISHII Yasuhisa, Akita-shi Akita 011-8510 (JP)
(74) Representative: Ter Meer Steinmeister & Partner
(86) International application number: PCT/JP2018/033372
(87) International publication number: WO 2019/050025

(57) **Abstract**

There is provided a medical high frequency treatment device (200) . A distal portion of the high frequency treatment device (200) includes a high frequency treatment device knife (100) having a pair of scissors (10) so as to incise a biological tissue. The pair of scissors (10) is configured to be capable of shearing the biological tissue by pivoting in a direction closer to each other. Each of the pair of scissors (10) has a blade surface (13). Each surface of the pair of scissors (10) includes a formation region of a non-conductive layer (insulating film (12)) and an electrode region (19) where the non-conductive layer is not formed on the surface of the blade surface (13). With regard to at least one of the pair of scissors (10), a width dimension of the electrode region (19) in a plate thickness direction of the scissors (10) varies depending on a position of the scissors (10) in a longitudinal direction.

## Description

### TECHNICAL FIELD

The present invention relates to a high frequency treatment device, a high frequency treatment device knife, and a high frequency treatment device distal treatment instrument.

### BACKGROUND ART

A high frequency treatment device used by being inserted into a forceps hole of an endoscope is known as a medical device for performing an incising treatment on a biological tissue inside a body cavity (including an excising treatment on a lesion site) .

For example, Patent Document 1 discloses a high frequency treatment device, a distal portion of which includes a pair of openable and closable scissors.

In addition, Patent Document 1 discloses the high frequency treatment device, the distal portion of which includes a pair of openable and closable shearing scissors.

In addition, Patent Document 1 discloses the high frequency treatment device, the distal portion of which includes a distal treatment instrument including a pair of opening and closing portions (high frequency treatment device distal treatment instrument).

### RELATED DOCUMENT

### PATENT DOCUMENT

[Patent Document 1] Pamphlet of International Publication No. 2011-043340

### SUMMARY OF THE INVENTION

### TECHNICAL PROBLEM

However, according to studies of the present inventor, a technique disclosed in Patent Document 1 still has room for improvement in hemostatic capability for a biological tissue.

In addition, according to the studies of the present inventor, the technique disclosed in Patent Document 1 does not always have sufficient reliability when the biological tissue is gripped using the pair of shearing scissors.

In addition, according to the studies of the present inventor, in the technique disclosed in Patent Document 1, when the distal treatment instrument is projected from a distal end of the endoscope and the pair of opening and closing portions are opened to perform a treatment, the pair of opening and closing portions is likely to interfere with the distal end of the endoscope (for example, a distal end of a hood).

First and sixth aspects of the present invention are made in view of the above-described problem, and aim to provide a high frequency treatment device having a structure having more satisfactory hemostatic capability for the biological tissue.

In addition, a second aspect of the present invention is made in view of the above-described problem, and aims to provide a high frequency treatment device knife having a structure capable of more reliably gripping the biological tissue. Furthermore, the second aspect of the present invention is made in view of the above-described problem, and aims to provide a medical high frequency treatment device, a distal portion of which has the high frequency treatment device knife having the structure capable of more reliably gripping the biological tissue.

In addition, a fourth aspect of the present invention is made in view of the above-described problem, and aims to provide a high frequency treatment device distal treatment instrument having a structure capable of suppressing interference with the distal end of the endoscope. Furthermore, a fifth aspect of the present invention is made in view of the above-described problem, and aims to provide a medical high frequency treatment device having the structure capable of suppressing the interference with the distal end of the endoscope.

### SOLUTION TO PROBLEM

According to a first aspect of the present invention, there is provided a medical high frequency treatment device, a distal portion of which including a high frequency treatment device knife having a pair of scissors so as to incise a biological tissue.

Each of the pair of scissors is formed in an elongated plate shape.

Proximal portions of the pair of scissors are axially supported by each other in a pivot shaft intersecting a plate surface direction of the scissors.

The pair of scissors is configured to be capable of shearing the biological tissue by pivoting in a direction closer to each other.

Each of the pair of scissors has a blade surface.

Each surface of the pair of scissors includes a formation region of a non-conductive layer, and an electrode region where the non-conductive layer is not formed on a surface of the blade surface.

With regard to at least one of the pair of scissors, a width dimension of the electrode region in a plate thickness direction of the scissors varies depending on a position of the scissors in a longitudinal direction.

According to a second aspect of the present invention, there is provided a high frequency treatment device knife disposed in a distal portion of the medical high frequency treatment device, and used by being inserted into a forceps hole of an endoscope so as to incise a biological tissue.

The high frequency treatment device knife includes a pair of shearing scissors axially supported by a common rotary shaft, capable of opening and closing each other, and each having a blade portion for shearing the biological tissue.

Each of the pair of shearing scissors has a proximal piece formed on a proximal side of the shearing scissors and axially supported by the rotary shaft, a distal claw portion formed in a distal end of the shearing scissors, and a blade portion formed between the distal claw portion and the proximal piece in the shearing scissors.

An electrode is formed in the blade portion.

Based on a virtual straight line connecting an axis center of the rotary shaft and a bottom of the blade portion to each other, a height of a highest position of the formation region of the electrode in the blade portion is lower than a height of the distal claw portion.

In addition, according to a third aspect of the present invention, there is provided a medical high frequency treatment device, a distal portion of which has a high frequency treatment device knife of the present invention, and a proximal side of which has an operation unit for performing an opening and closing operation on the pair of shearing scissors.

According to a fourth aspect of the present invention, there is provided a high frequency treatment device distal treatment instrument disposed in a distal portion of a medical high frequency treatment device and used by being inserted into a forceps hole of an endoscope so as to incise a biological tissue.

The high frequency treatment device distal treatment instrument includes a distal treatment unit having a pair of opening and closing portions each having a line-shaped electrode, axially supported by a common rotary shaft, capable of opening and closing each other, performing high frequency excision by shearing or pinching the biological tissue.

In a state where the pair of opening and closing portions is closed, a shape of a distal side portion of the distal treatment unit when viewed in an axial direction of the rotary shaft is a shape which is narrowed after being widened from a distal end toward a proximal end.

In addition, according to a fifth aspect of the present invention, there is provided a medical high frequency treatment device, a distal portion of which has a high frequency treatment device distal treatment instrument of the present invention, and a proximal side of which has an operation unit for performing an opening and closing operation on a pair of opening and closing portions.

According to a sixth aspect of the present invention, there is provided a medical high frequency treatment device, a distal portion of which including a high frequency treatment device knife having a pair of scissors so as to incise a biological tissue.

Each of the pair of scissors is formed in an elongated plate shape.

Proximal portions of the pair of scissors are axially supported by each other in a pivot shaft intersecting a plate surface direction of the scissors.

The pair of scissors is configured to be capable of shearing the biological tissue by pivoting in a direction closer to each other.

Each of the pair of scissors has a blade surface, a sliding contact surface that comes into sliding contact with each other, an outer surface that is a rear surface with respect to the sliding contact surface, and an inclined surface that is located between the outer surface and the blade surface.

The inclined surface is inclined from the sliding contact surface side toward the outer surface side in a direction away from the other scissor.

Each surface of the pair of scissors includes a formation region of a non-conductive layer, and an electrode region where the non-conductive layer is not formed.

With regard to at least one of the pair of scissors, the electrode region is formed on the blade surface and the inclined surface.

### ADVANTAGEOUS EFFECTS OF INVENTION

According to the first and sixth aspects of the present invention, the hemostatic capability for the biological tissue is more satisfactorily achieved.

According to the second and third aspects of the present invention, the biological tissue can be more reliably gripped.

According to the fourth and fifth aspects of the present invention, it is possible to suppress the interference between the pair of opening and closing portions of the high frequency treatment device distal treatment instrument and the distal end of the endoscope.

### BRIEF DESCRIPTION OF THE DRAWINGS

The above-described objects, other objects, features, and advantages will become more apparent from the preferred embodiments described below and the following drawings accompanying thereto.
Fig. 1 is a schematic view illustrating an overall structure of a high frequency treatment device according to a first embodiment.
Fig. 2 is a side view of a high frequency treatment device knife provided in a distal portion of the high frequency treatment device according to the first embodiment, and illustrates a state where a pair of scissors is closed.
Fig. 3 is a side view of the high frequency treatment device knife provided in the distal portion of the high frequency treatment device according to the first embodiment, and illustrates a state where the pair of scissors is open.
Fig. 4 is a plan view of the high frequency treatment device knife provided in the distal portion of the high frequency treatment device according to the first embodiment, and illustrates a state where the pair of scissors is closed.
Fig. 5(a) is a side view illustrating an outer surface of the scissors of the high frequency treatment device according to the first embodiment, and Fig. 5(b) is a side view illustrating an inner surface (sliding contact surface) of the scissors of the high frequency treatment device according to the first embodiment.
Fig. 6 (a) is a plan view of the scissors of the high frequency treatment device according to the first embodiment, and Fig. 6(b) is a perspective view of the scissors of the high frequency treatment device according to the first embodiment.
Fig. 7 is a sectional end view taken along line A-A in Fig. 2.
Fig. 8(a) is a side view illustrating an outer surface of scissors of a high frequency treatment device according to a second embodiment, and Fig. 8(b) is a plan view of the scissors of the high frequency treatment device according to the second embodiment.
Fig. 9(a) is a perspective view of the scissors of the high frequency treatment device according to the second embodiment, and Fig. 9 (b) is a perspective view when the scissors of the high frequency treatment device according to the second embodiment are viewed from a proximal side.
Fig. 10(a) is a side view illustrating an outer surface of scissors of a high frequency treatment device according to a third embodiment, and Fig. 10(b) is a plan view of the scissors of the high frequency treatment device according to the second embodiment.
Fig. 11(a) is a perspective view of the scissors of the high frequency treatment device according to the third embodiment, and Fig. 11(b) is a perspective view when the scissors of the high frequency treatment device according to the third embodiment are viewed from the proximal side.
Fig. 12 is a schematic view illustrating an overall structure of a medical high frequency treatment device according to a fourth embodiment.
Fig. 13 is a side view of a high frequency treatment device knife provided in a distal portion of the medical high frequency treatment device according to the fourth embodiment, and illustrates a state where a pair of shearing scissors is closed.
Fig. 14 is a side view of the high frequency treatment device knife provided in the distal portion of the medical high frequency treatment device according to the fourth embodiment, and illustrates a state where the pair of shearing scissors is open.
Fig. 15 is a plan view of the high frequency treatment device knife provided in the distal portion of the medical high frequency treatment device according to the fourth embodiment, and illustrates a state where the pair of shearing scissors is closed.
Fig. 16(a) is a side view illustrating an inner surface of the shearing scissors of the high frequency treatment device knife according to the fourth embodiment, and Fig. 16(b) is a side view illustrating an outer surface of the shearing scissors of the high frequency treatment device knife according to the fourth embodiment.
Fig. 17(a) is a perspective view of the shearing scissors of the high frequency treatment device knife according to the fourth embodiment, and Fig. 17 (b) is a sectional end view of the shearing scissors, which is taken along line A-A in Fig. 16(b).
Fig. 18 is a side view illustrating a state where the high frequency treatment device knife provided in the distal portion of the medical high frequency treatment device according to the fourth embodiment projects from a hood disposed in a distal end of an endoscope.
Fig. 19 is a side view of a high frequency treatment device knife provided in a distal portion of a medical high frequency treatment device according to a fifth embodiment, and illustrates a state where a pair of shearing scissors is closed.
Fig. 20 is a side view of the high frequency treatment device knife provided in the distal portion of the medical high frequency treatment device according to the fifth embodiment, and illustrates a state where the pair of shearing scissors is open.
Fig. 21 is a side view of a high frequency treatment device knife provided in a distal portion of a medical high frequency treatment device according to a sixth embodiment, and illustrates a state where a pair of shearing scissors is open.
Fig. 22 is a side view of the high frequency treatment device knife provided in the distal portion of the medical high frequency treatment device according to the sixth embodiment, and illustrates a state where the pair of shearing scissors is closed, distal claw portions of the pair of shearing scissors start to overlap each other, and mutually corresponding intermediate high step portions in the pair of shearing scissors are in contact with each other.
Fig. 23 is a side view of the high frequency treatment device knife provided in the distal portion of the medical high frequency treatment device according to the sixth embodiment, and illustrates a state where the pair of shearing scissors is closed.
Fig. 24 is a schematic view illustrating an overall structure of a medical high frequency treatment device according to a seventh embodiment.
Fig. 25 is a side view of a high frequency treatment device distal treatment instrument provided in a distal portion of the medical high frequency treatment device according to the seventh embodiment, and illustrates a state where a pair of opening and closing portions is closed.
Fig. 26 is a side view of the high frequency treatment device distal treatment instrument provided in the distal portion of the medical high frequency treatment device according to the seventh embodiment, and illustrates a state where the pair of opening and closing portions is open.
Fig. 27 is a plan view of the high frequency treatment device distal treatment instrument provided in the distal portion of the medical high frequency treatment device according to the seventh embodiment, and illustrates a state where the pair of opening and closing portions is closed.
Fig. 28 is a sectional end view taken along line A-A in Fig. 25.
Fig. 29 is a side view illustrating a state where the high frequency treatment device distal treatment instrument provided in the distal portion of the medical high frequency treatment device according to the seventh embodiment projects from a hood disposed in a distal end of an endoscope.
Fig. 30 is a side view of a high frequency treatment device distal treatment instrument provided in a distal portion of a medical high frequency treatment device according to an eighth embodiment, and illustrates a state where a pair of opening and closing portions is closed.
Fig. 31 is a side view of the high frequency treatment device distal treatment instrument provided in the distal portion of the medical high frequency treatment device according to the eighth embodiment, and illustrates a state where the pair of opening and closing portions is open.
Fig. 32 is a perspective view of a distal portion of one opening and closing portion of the high frequency treatment device distal treatment instrument according to the eighth embodiment.
Fig. 33 is a schematic view illustrating an overall structure of a high frequency treatment device according to a ninth embodiment.
Fig. 34 is a side view of a high frequency treatment device knife provided in a distal portion of the high frequency treatment device according to the ninth embodiment, and illustrates a state where a pair of scissors is closed.
Fig. 35 is a side view of the high frequency treatment device knife provided in the distal portion of the high frequency treatment device according to the ninth embodiment, and illustrates a state where the pair of scissors is open.
Fig. 36 is a plan view of the high frequency treatment device knife provided in the distal portion of the high frequency treatment device according to the ninth embodiment, and illustrates a state where the pair of scissors is closed.
Fig. 37(a) is a side view illustrating an outer surface of scissors of the high frequency treatment device according to the ninth embodiment, and Fig. 37 (b) is a side view illustrating an inner surface (sliding contact surface) of the scissors of the high frequency treatment device according to the ninth embodiment.
Fig. 38 (a) is a plan view of the scissors of the high frequency treatment device according to the ninth embodiment, and Fig. 38 (b) is a perspective view of the scissors of the high frequency treatment device according to the ninth embodiment.
Fig. 39(a) is a perspective view when the scissors of the high frequency treatment device according to the ninth embodiment are viewed from a distal side, and Fig. 39(b) is a perspective view when the scissors of the high frequency treatment device according to the ninth embodiment are viewed from a proximal side.
Fig. 40 is a sectional end view taken along line A-A in Fig. 34.
Fig. 41 is a perspective view when a high frequency treatment device knife of a high frequency treatment device according to a tenth embodiment is viewed from a proximal side.
Fig. 42(a) is a plan view of scissors of a high frequency treatment device according to an eleventh embodiment, and Fig. 42 (b) is a perspective view of the scissors of the high frequency treatment device according to the eleventh embodiment.

### DESCRIPTION OF EMBODIMENTS

Hereinafter, embodiments of the present invention will be described with reference to the drawings. In all of the drawings, the same reference numerals will be given to the same configuration elements, and description thereof will not be repeated.

Various configuration elements of a medical high frequency treatment device according to the present embodiment do not need to be independently present, and allow the followings. A plurality of the configuration elements are formed as one member. One configuration element is formed of a plurality of members. A certain configuration element is a part of other configuration elements. A part of a certain configuration element overlaps a part of other configuration elements.

### [First Embodiment]

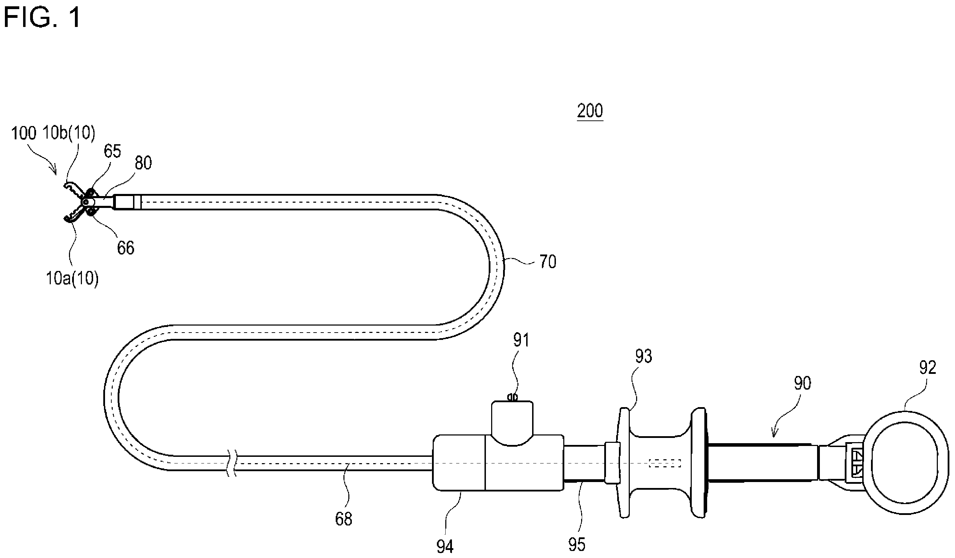

First, a first embodiment will be described with reference to Figs. 1 to 7.

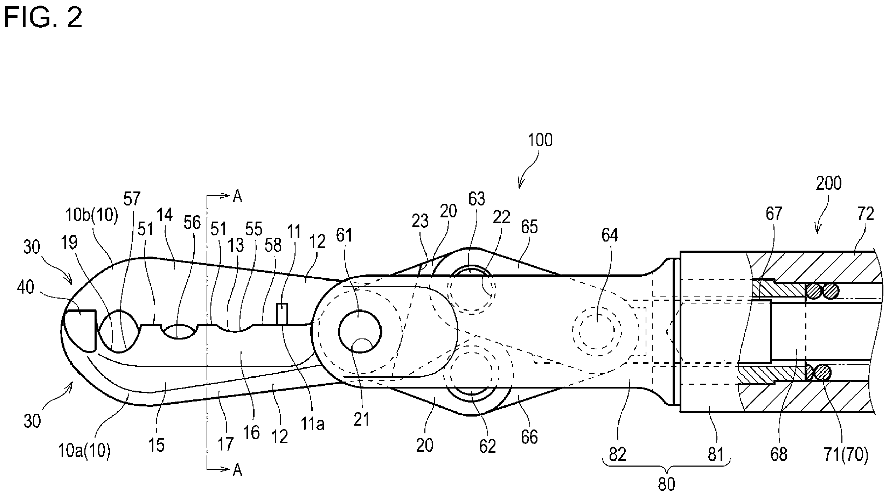

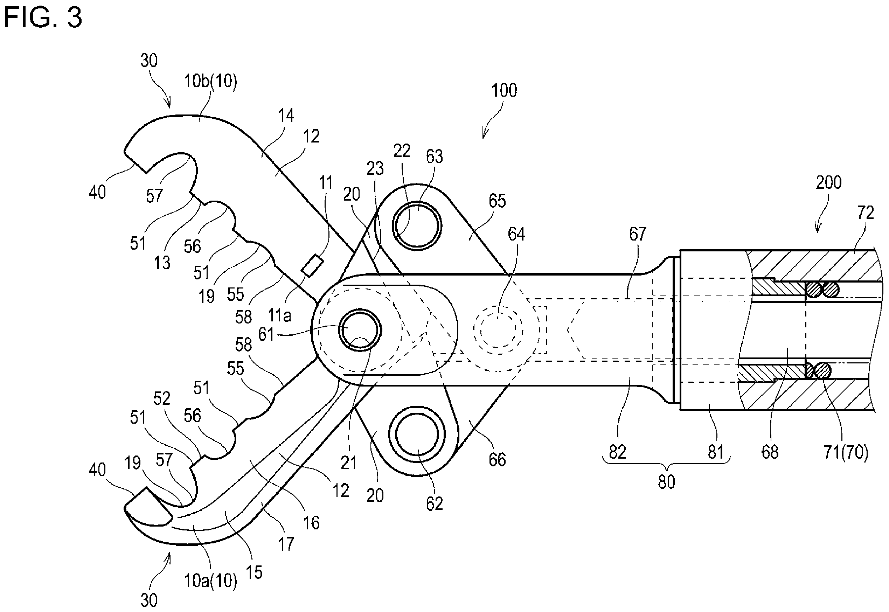

In side views illustrated in Figs. 2 and 3, a proximal side portion in an illustrated range in a sheath 70 indicates a side cross section taken along a center line.

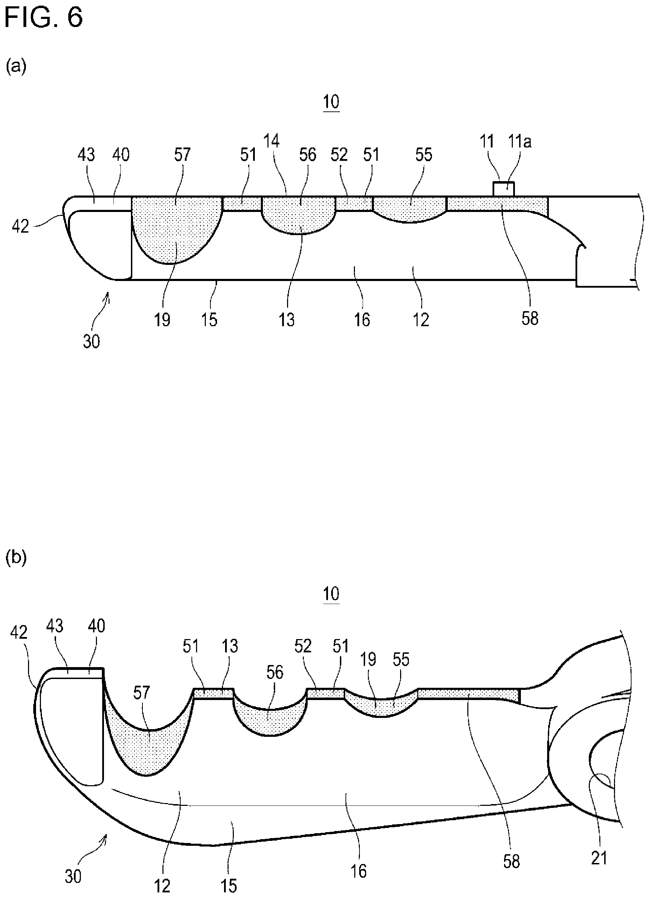

In each of Figs. 6 (a) and 6(b), an electrode formation region (electrode region 19) in scissors 10 is hatched in a dot shape. In each of Fig. 6 (a) and Fig. 6(b), a region which is not hatched in the dot shape is a formation region of an insulating film 12 (non-conductive layer). However, the insulating film 12 may not be formed inside a first shaft support hole 21 and inside a second shaft support hole 22.

As illustrated in Fig. 1, a high frequency treatment device 200 according to the present embodiment is a medical high frequency treatment device 200. In the high frequency treatment device 200, a distal portion of the high frequency treatment device 200 includes a high frequency treatment device knife 100 having a pair of scissors 10 so as to incise a biological tissue.

The high frequency treatment device 200 is used by inserting the high frequency treatment device knife 100 of the high frequency treatment device 200 into a forceps hole of an endoscope (not illustrated).

One of the pair of scissors 10 will be referred to as scissors 10a, and the other will be referred to as scissors 10b.

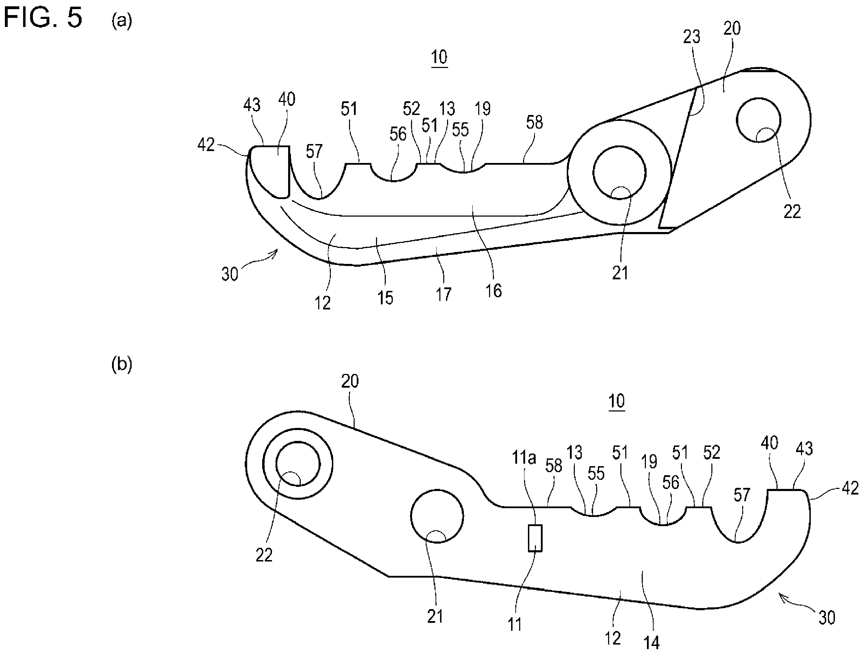

Each of the pair of scissors 10 is formed in an elongated plate shape (refer to Figs. 5(a) and 5(b)).

As illustrated in Figs. 2 to 4, proximal portions of the pair of scissors 10 are axially supported by each other in a pivot shaft (shaft member 61) intersecting a plate surface direction of the scissors 10.

The pair of scissors 10 is configured to be capable of shearing the biological tissue by pivoting in a direction closer to each other.

Each of the pair of scissors 10 has a blade surface 13.

Each surface of the pair of scissors 10 includes the formation region of the non-conductive layer (insulating film 12) and the electrode region 19 where the non-conductive layer is not formed on a surface of the blade surface 13.

Then, with regard to at least one of the pair of scissors 10, a width dimension of the electrode region 19 in a plate thickness direction of the scissors 10 varies depending on a position of the scissors 10 in a longitudinal direction (Fig. 6(a)) . Here, the width dimension of the electrode region 19 is the width dimension of the electrode region 19 in a direction parallel to the pivot shaft of the scissors 10 (width dimension of the electrode region 19 in a thickness direction of the scissors 10) . In a case of the present embodiment, with regard to each of the pair of scissors 10, the width dimension of the electrode region 19 in the plate thickness direction of the scissors 10 varies depending on the position of the scissors 10 in the longitudinal direction.

According to the high frequency treatment device 200 in the present embodiment, with regard to at least one of the pair of scissors 10, the width dimension of the electrode region 19 in the plate thickness direction of the scissors 10 varies depending on the position of the scissors 10 in the longitudinal direction. Therefore, a current flowing from the electrode region 19 to the biological tissue can be sufficiently secured. Accordingly, hemostatic capability can be satisfactorily achieved. In addition, the electrode region 19 is formed on the blade surface 13. Accordingly, an intended site in the biological tissue can be more reliably and selectively cauterized.

As illustrated in Figs. 5(a) and 5(b), each of the pair of scissors 10 has a proximal piece 20 which is a proximal side portion in the scissors 10, and a distal piece 30 which is a distal side portion in the scissors 10.

A distal portion of the proximal piece 20 has the first shaft support hole 21 penetrating the proximal piece 20 in the thickness direction. A common shaft member 61 (Figs. 2 to 4) is inserted into the first shaft support holes 21 of the pair of scissors 10, and the pair of scissors 10 are axially supported.

The distal piece 30 is a distal side portion of the first shaft support hole 21 in the scissors 10.

A proximal portion of the proximal piece 20 has the second shaft support hole 22 penetrating the proximal piece 20 in the thickness direction.

As illustrated in Fig. 1, the high frequency treatment device 200 includes an elongated operation wire 68, a high frequency treatment device knife 100 disposed in a distal end of the operation wire 68, a flexible sheath 70 that accommodates the operation wire 68, and a hand operation unit 90 disposed on a proximal side of the sheath 70 and connected to a proximal end of the operation wire 68.

The sheath 70 is an elongated and tubular member that accommodates the operation wire 68. In a case of the present embodiment, the sheath 70 is configured to have a metal coil 71 (Figs. 2 and 3) manufactured by tightly winding a conductive wire such as a stainless wire. The insulating film 72 (Figs. 2 and 3) is tightly disposed on an outer surface of the sheath 70. However, as the sheath 70, an insulating tubular member (tube) may be used instead of the metal coil 71.

The hand operation unit 90 is disposed to perform an opening and closing operation on the pair of scissors 10, and is located on the proximal side in the high frequency treatment device 200.

For example, the hand operation unit 90 includes a shaft portion 95 into which the operation wire 68 is inserted, a finger ring 92 disposed in the proximal portion of the shaft portion 95, a slider 93 to which the proximal end of the operation wire 68 is connected and which moves forward and rearward with respect to the shaft portion 95, and a rotational operation unit 94. The operation wire 68 is slidably inserted into the shaft portion 95. For example, a user inserts a thumb into the finger ring 92, and pinches the slider 93 with other two fingers, thereby driving the slider 93 to move forward and rearward along the longitudinal direction of the shaft portion 95. In this manner, the operation wire 68 moves forward or rearward with respect to the hand operation unit 90. The proximal end of the sheath 70 is fixed to the hand operation unit 90, and the operation wire 68 is inserted into the sheath 70 to be movable forward and rearward. Accordingly, the distal end of the operation wire 68 moves forward or rearward with respect to the sheath 70 in conjunction with the forward and rearward movement of the slider 93. In this manner, as will be described later, a forward and rearward movement portion 67 (Figs. 2 and 3) of the high frequency treatment device knife 100 is driven to move forward and rearward, and the pair of scissors 10 is opened and closed.

An axial direction of the rotary shaft of the pair of scissors 10 is a direction perpendicular to a plate surface of the scissors 10 (thickness direction of the scissors 10). Sliding contact surfaces 14 of the pair of scissors 10 slide when the pair of scissors 10 is opened and closed.

As illustrated in Fig. 1, the hand operation unit 90 includes a power supply unit 91. The power supply unit 91 is a terminal for applying a high frequency current to the pair of scissors 10. A high frequency power source (not illustrated) is connected to the power supply unit 91 through a power cable. The pair of scissors 10, link pieces 65 and 66 (to be described below), and the forward and rearward movement portion 67 (to be described below), which configure the high frequency treatment device knife 100, are all manufactured using a conductive metal material. In addition, the operation wire 68 is also manufactured using the conductive metal material. Therefore, the high frequency current input to the power supply unit 91 is applied to the pair of scissors 10.

The operation wire 68 is connected to the rotational operation unit 94, and the rotational operation unit 94 is axially rotated around the shaft portion 95. In this manner, the operation wire 68 whose proximal end is fixed to the slider 93 is rotated inside the sheath 70. In this manner, the high frequency treatment device knife 100 can be oriented in a desired direction.

The rotational operation unit 94 is rotatably attached to the power supply unit 91, and the rotational operation unit 94 can be operated to rotate around the shaft portion 95 on a state where a power cable (not illustrated) connecting the power supply unit 91 and a high frequency power source (not illustrated) to each other is hung downward.

Instead of the present embodiment, the slider 93 may be configured to be axially rotatable around the shaft portion 95, and the slider 93 may also function as the rotational operation unit 94. That is, a configuration may be adopted as follows. The slider 93 is driven to move forward and rearward along the longitudinal direction of the shaft portion 95. In this manner, the operation wire 68 is moved forward and rearward to perform the opening and closing operation on the high frequency treatment device knife 100. In addition, the slider 93 is axially rotated around the shaft portion 95. In this manner, the high frequency treatment device knife 100 is rotated and oriented in a desired direction.

In addition, a configuration may be adopted in which the rotational operation unit 94 is disposed in the shaft portion 95 to be rotatable with respect to the power supply unit 91. In this case, the slider 93 may be configured to be rotatable around the shaft portion 95.

As illustrated in Figs. 2 to 4, the high frequency treatment device knife 100 includes the pair of plate-shaped scissors 10, the shaft member 61 that axially supports the scissors 10 to be openable and closable, the two link pieces 65 and 66, the forward and rearward movement portion 67, and a holding frame 80.

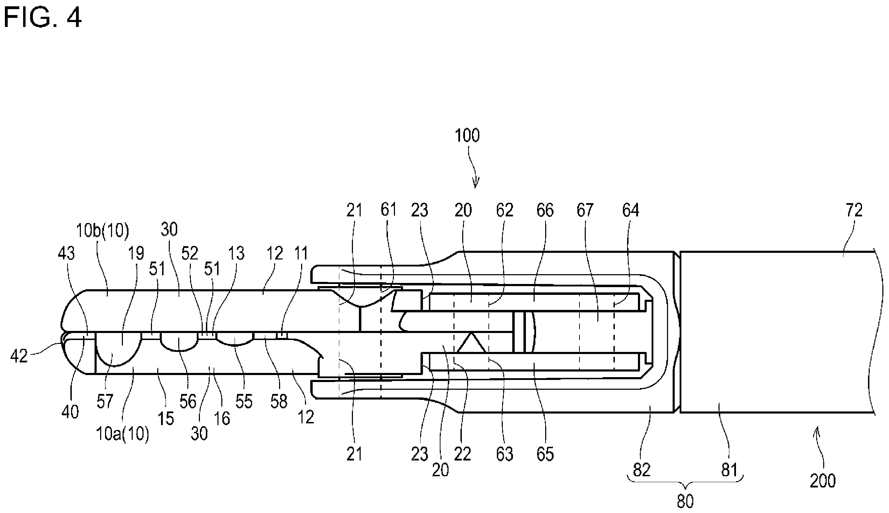

The axial direction of the shaft member 61 is a direction perpendicular to a paper surface in Figs. 2 and 3, and is an upward-downward direction in Fig. 4. In addition, the axial direction of the shaft member 61 is a direction in which the pair of scissors 10 overlaps each other. In other words, the axial direction of the shaft member 61 is the thickness direction of the pair of scissors 10.

The pair of scissors 10 is driven to be opened and closed by pushing and pulling the operation wire 68. The operation wire 68 is manufactured using a conductive metal material such as stainless steel.

The forward and rearward movement portion 67 is integrally connected to the operation wire 68 in the distal end of the operation wire 68. The proximal portion of the two link pieces 65 and 66 is pivotally connected to the forward and rearward movement portion 67 by a shaft member 64. Furthermore, the proximal piece 20 of one of the scissors 10 (scissors 10a) is pivotally connected to the distal portion of the link piece 65 by a shaft member 63. That is, the shaft member 63 is inserted into the second shaft support hole 22 of one scissors 10a and the distal portion of the link piece 65. In this manner, the scissors 10a and the link piece 65 are rotatably and axially supported by each other. Similarly, the proximal piece 20 of the other scissors 10 (scissors 10b) is pivotally connected to the distal portion of the link piece 66 by a shaft member 62. That is, the shaft member 62 is inserted into the second shaft support hole 22 of the other scissors 10b and the distal portion of the link piece 66. In this manner, the scissors 10b and the link piece 66 are rotatably and axially supported by each other.

The axial direction of the respective shaft members 62, 63, and 64 is a direction parallel to the axial direction of the shaft member 61.

The pair of scissors 10 and the link pieces 65 and 66 relatively pivot in a plane illustrated in Figs. 2 and 3 (in a plane perpendicular to the axial direction of the shaft member 61) .

The proximal piece 20 of the pair of scissors 10 and the link pieces 65 and 66 configure a four-joint link having a rhomboid shape.

The shaft members 62 and 63 are located on the distal side of the shaft member 64, and the shaft member 61 is located on the distal side of the shaft members 62 and 63.

As illustrated in Fig. 5(a), a step portion 23 is formed on an outer surface of the proximal piece 20. In the proximal piece 20, a proximal side portion from the step portion 23 is thinner than a distal side portion from the step portion 23.

As illustrated in Fig. 4, a thickness difference between the proximal side portion and the distal side portion from the step portion 23 in the proximal piece 20 is set to be slightly larger than the thickness of the link pieces 65 and 66, or is set to be equal to the thickness of the link pieces 65 and 66.

The holding frame 80 is fixed to the distal end of the sheath 70.

The holding frame 80 includes a proximal portion 81 fixed to the distal end of the sheath 70, and a pair of brackets 82 projecting to the distal side from the proximal portion 81.

Each of the pair of brackets 82 is formed in a plate shape, for example.

The pair of scissors 10 is axially supported by the shaft member 61 with respect to the distal portion of the pair of brackets 82. That is, the shaft member 61 is inserted into the first shaft support hole 21 of each proximal piece 20 of the pair of scissors 10 and the pair of brackets 82. In this manner, the proximal piece 20 of the pair of scissors 10 is axially supported by the pair of brackets 82.

In a gap between the pair of brackets 82, the proximal piece 20 of the pair of scissors 10 and the link pieces 65 and 66 are respectively rotatable. A portion projecting to the distal side from the sheath 70 in the forward and rearward movement portion 67 is movable forward and rearward.

Furthermore, the bracket 82 is rotatable around the axis of the sheath 70 with respect to the proximal portion 81, or the bracket 82 is rotatable around the axis of the sheath 70 with respect to the sheath 70.

As illustrated in Fig. 2, when the operation wire 68 and the forward and rearward movement portion 67 are pulled toward the proximal side (rightward in Fig. 2), the pair of scissors 10 is in a closed state. Conversely, as illustrated in Fig. 3, when the operation wire 68 and the forward and rearward movement portion 67 are pushed to the distal side (leftward in Fig. 3), the pair of scissors 10 is in an open state.

The insulating film 12 (non-conductive layer) is formed on each surface of the pair of scissors 10. The insulating film 12 is formed on a whole surface of the distal piece 30 except for at least the formation region of the electrode region 19.

For example, the insulating film 12 can be formed by coating the surface of the scissors 10 with an insulating material such as a fluororesin, polyether ether ketone (PEEK), diamond-like carbon (DLC), or a ceramic material (ceramic material such as titanium oxide or silicon).

The electrode region 19 is a linear portion where the insulating film 12 is not formed in the distal piece 30. The pair of scissors 10 serves as a monopolar high frequency electrode when a high frequency voltage in the same phase is applied thereto from the power supply unit 91. The high frequency current is applied to the pair of scissors 10 in a state where the biological tissue is gripped using the pair of scissors 10. In this manner, the biological tissue is cauterized and incised. Instead of the present embodiment, a bipolar high frequency treatment device 200 may be used in which one of the pair of scissors 10 is used as an active electrode and the other is used as a return electrode.

The shapes of the pair of scissors 10 may be the same as each other, or may be different from each other. In a case of the present embodiment, the pair of scissors 10 has mutually the same shape.

Hereinafter, the shape of the scissors 10 will be described in detail with reference to Figs. 5(a) to 7.

The scissors 10 have a blade surface 13, a sliding contact surface 14 that comes into sliding contact with each other, an outer surface 15 that is a rear surface with respect to the sliding contact surface 14, an inclined surface 16 located between the outer surface 15 and the blade surface 13, and a rear surface 17 that is a surface opposite to the blade surface 13.

For example, the sliding contact surface 14 and the outer surface 15 are located parallel to each other.

In a case of the present embodiment, for example, the blade surface 13 is perpendicular to both the sliding contact surface 14 and the outer surface 15. That is, the blade surface 13 is located parallel to the axial direction of the shaft member 61 which is the rotary shaft of the scissors 10.

The inclined surface 16 is inclined with respect to both the blade surface 13 and the outer surface 15.

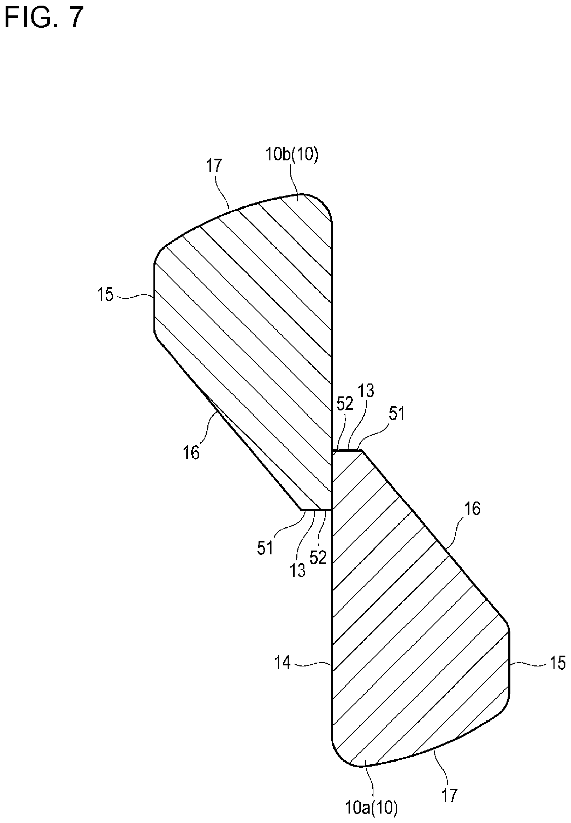

The inclined surface 16 is inclined from the sliding contact surface 14 side toward the outer surface 15 side in a direction away from the other scissors 10. That is, as illustrated in Fig. 7, the inclined surface 16 of the scissors 10a is inclined downward from the sliding contact surface 14 side (left side in Fig. 7) of the scissors 10a toward the outer surface 15 side (right side in Fig. 7). The inclined surface 16 of the scissors 10b is inclined upward from the sliding contact surface 14 side (the right side in Fig. 7) of the scissors 10b toward the outer surface 15 (the left side in Fig. 7).

In other words, the inclined surface 16 is inclined from the blade surface 13 side toward the outer surface 15 side in a direction away from the other scissors 10. That is, as illustrated in Fig. 7, the inclined surface 16 of the scissors 10a is inclined downward from the blade surface 13 side of the scissors 10a toward the outer surface 15 side. The inclined surface 16 of the scissors 10b is inclined upward from the blade surface 13 side of the scissors 10b toward the outer surface 15 side.

Each surface of the pair of scissors 10 includes the formation region of the non-conductive layer (insulating film 12) and the electrode region 19 where the non-conductive layer is not formed.

A distal claw portion 40 is formed in the distal portion of the distal piece 30 (left end portion of the distal piece 30 in Fig. 5(a)) of the scissors 10. The distal claw portion 40 projects in a closing direction. The closing direction is a direction from one scissors 10 to the other scissors 10b, and a direction opposite thereto will be referred to as an opening direction. The distal claw portion 40 projects upward in Fig. 5(a).

The distal claw portion 40 is a projection that is bitten into the biological tissue.

The blade surface 13 is formed along an end edge (edge) on a side in the closing direction in a portion on the proximal side (right side in Fig. 5(a)) of the distal claw portion 40 in the distal piece 30, that is, along an upper edge of the distal piece 30 in Fig. 5(a).

In a state where the biological tissue is pinched by the distal claw portion 40 of the pair of scissors 10 to suppress the falling of the biological tissue, the biological tissue can be sheared and incised by the blade surface 13 of the pair of scissors 10.

For example, the insulating films 12 are respectively formed on a distal surface 42 which is a surface facing the distal side in the distal claw portion 40, a top surface 43 of the distal claw portion 40, and a side surface of the distal claw portion 40. The electrode region 19 is not formed on the distal surface 42, the top surface 43, and the side surface of the distal claw portion 40.

On the other hand, the proximal surface which is a surface facing the proximal side in the distal claw portion 40 is a portion of the surface of the recessed portion 57. The insulating film 12 is not formed on the proximal surface of the distal claw portion 40, and the electrode region 19 is formed.

In a case of the present embodiment, as illustrated in Fig. 5(a), the scissors 10 have a projecting portion 51 projecting toward the other scissors 10 in an intermediate portion in the longitudinal direction of the scissors 10, and recessed portions 55, 56, and 57 located adjacent to the projecting portion 51 in the longitudinal direction of the scissors 10 and recessed toward a side away from the other scissors.

The blade surface 13 includes a top surface 52 of the projecting portion 51 and surfaces of the recessed portions 55, 56, and 57 (refer to Figs. 6(a) and 6(b)).

Then, the electrode regions 19 are respectively formed on the top surface 52 of the projecting portion 51 and the surfaces of the recessed portions 55, 56, and 57.

More specifically, the electrode regions 19 are formed in an entire region of the top surface 52 of the projecting portion 51 and an entire region of the surfaces of the recessed portions 55, 56, and 57.

More specifically, the scissors 10 have a plurality of the recessed portions 55, 56, and 57 located with the projecting portion 51 as a boundary, and the width dimension of the electrode region 19 increases toward the recessed portions on the distal side (refer to Fig. 6(a)).

In a case of the present embodiment, the scissors 10 have two projecting portions 51 and three recessed portions 55, 56, and 57. Out of the three recessed portions 55, 56, 57, the recessed portion 55 is located on the most proximal side, and the recessed portion 57 is located on the most distal side. The width dimension (maximum width dimension) of the electrode region 19 in the recessed portion 56 is larger than the width dimension (maximum width dimension) of the electrode region 19 in the recessed portion 55, and the width dimension (maximum width dimension) of the electrode region 19 in the recessed portion 56 is larger than the width dimension (maximum width dimension) of the electrode region 19 in the recessed portion 57.

More specifically, the widths of the electrode regions 19 on the top surface 52 of the respective projecting portions 51 are equal to each other.

That is, the scissors 10 have a plurality of the projecting portions 51 located at mutually different positions in the longitudinal direction of the scissors 10, and the widths of the electrode regions 19 on the top surfaces 52 of the plurality of projecting portions 51 are equal to each other.

Here, in the distal piece 30 of the scissors 10, a portion which is located adjacent to the proximal side of the recessed portion 55 on the most proximal side and which is in a higher step than the recessed portion 55 will be referred to as a proximal side high step portion 58.

That is, in the end edge on the side in the closing direction of the distal piece 30, the distal claw portion 40, the recessed portion 57, the projecting portion 51, the recessed portion 56, the projecting portion 51, the recessed portion 55, and the proximal side high step portion 58 are located sequentially from the distal side of the distal piece 30.

The distal claw portion 40 is located adjacent to the distal side of the recessed portion 57 located on the most distal side.

The distal claw portion 40 projects in the closing direction (to the other scissors 10 side) from the respective projecting portions 51 and the proximal side high step portion 58.

For example, the top surface 52 of the respective projecting portion 51 is a flat surface. More specifically, the top surface 52 is a plane parallel to both a distal-proximal direction of the distal piece 30 and the rotary shaft of the scissors 10.

When the pair of scissors 10 is closed, at a position (height) in the opening and closing direction, it is preferable that the height of the top surface 43 of the distal claw portion 40 is higher than the height of the top surface 52 of the projecting portion 51, and that the height of the top surface 52 of the projecting portion 51 is higher than the height of the proximal side high step portion 58. In this way, when the pair of scissors 10 is closed, the high frequency treatment device knife 100 is configured so that the height positions of the projecting portions in which the positions of the scissors 10 in the distal-proximal direction are different from each other vary. In this manner, the tissue can be more easily gripped. From a similar viewpoint, with regard to the height (height in the opening and closing direction) of the projecting portions 51, it is preferable that the height of the top surface 52 of the projecting portion 51 located on the distal side is higher than the height of the top surface 52 of the projecting portion 51 located on the proximal side. However, the present embodiment is not limited to this example. The heights of the top surfaces 52 of the projecting portions 51 may be equal to each other, or the heights of the top surfaces 52 of the projecting portions 51 and the proximal side high step portion 58 may be equal to each other.

In a case of the present embodiment, the blade surface 13 is continuously formed over the recessed portion 57, the projecting portion 51 adjacent to the proximal side of the recessed portion 57, the recessed portion 56, the projecting portion 51 adjacent to the proximal side of the recessed portion 56, the recessed portion 55, and the proximal side high step portion 58. In addition, for example, the electrode region 19 is also formed over the entire region in the width direction of the proximal side high step portion 58 on the blade surface 13 of the distal side portion of the proximal side high step portion 58.

In a case of the present embodiment, the recessed portions 55, 56, and 57 are recessed in an arc shape.

More specifically, the recessed portion 57 located on the most distal side is recessed most deeply, and the recessed portion 55 located on the most proximal side is recessed most shallowly.

In the respective recessed portions 55, 56, and 57, the thickness dimension of the scissors 10 increases as a portion is recessed deeper. Therefore, in the respective recessed portions 55, 56, and 57, the width dimension of the electrode region 19 increases as a portion is recessed deeper.

The width dimension of the respective recessed portions 55, 56, 57, that is, the width dimension of the electrode region 19 in the respective recessed portions 55, 56, 57 is minimized in the proximal end and the distal end of the respective recessed portions 55, 56, 57, and is maximized in the intermediate portion between the proximal end and the distal end.

The surface of the respective recessed portions 55, 56, 57 is a recessed curved surface. In a case of the present embodiment, the surface of the respective recessed portions 55, 56, 57 is located parallel to the rotary shaft of the scissors 10. Therefore, in Figs. 5 (a) and 5(b), the surface of the respective recessed portions 55, 56, and 57 is not visible.

In a case of the present embodiment, for example, the width dimension of the top surface 52 of the projecting portion 51, that is, the width dimension of the electrode region 19 on the top surface 52 is constant over the entire region of the projecting portion 51 in the distal-proximal direction.

Furthermore, for example, the width dimension of the electrode region 19 on the top surface 52 is equal to the width dimension of the portion where the width dimension of the electrode region 19 is smallest in the recessed portions 55, 56, and 57, and is equal to the width dimension of the portion where the width dimension of the electrode region 19 is smallest in the proximal side high step portion 58.

The present embodiment is not limited to this example. The width dimension of the electrode region 19 on the top surface 52 may be smaller than the width dimension of the portion where the width dimension of the electrode region 19 is smallest in the recessed portions 55, 56, and 57, and may be smaller than the width dimension of the portion where the width dimension of the electrode region 19 is smallest in the proximal side high step portion 58.

That is, the width of the electrode region 19 on the top surface 52 of the projecting portion 51 is equal to or smaller than the width of the narrowest portion in the electrode region 19 other than the projecting portion 51.

According to this configuration, the biological tissue is more easily gripped by the high frequency treatment device knife 100.

In a case of the present embodiment, the electrode region 19 is not formed in a portion other than the blade surface 13 in the distal piece 30. That is, the insulating films 12 are formed on the entire surface of the distal piece 30 except for the surface of the respective recessed portions 55, 56, and 57, the top surface 52 of the respective projecting portions 51, and the surface of the proximal side high step portion 58.

A stopper portion 11 is formed in at least one scissors 10 of the pair of scissors 10. A closing operation of the pair of scissors 10 is restricted by the stopper portion 11 coming into contact with the blade surface 13 (for example, the proximal side high step portion 58) of the other scissors 10.

In a case of the present embodiment, the stopper portion 11 is formed in each of the pair of scissors 10, and the stopper portion 11 of the scissors 10a comes into contact with the blade surface 13 of the scissors 10b, and the stopper portion 11 of the scissors 10b comes into contact with the blade surface 13 of the scissors 10a, thereby restricting the closing operation of the pair of scissors 10.

The stopper portion 11 is formed on the sliding contact surface 14 illustrated in Fig. 5(b), in the scissors 10. In the stopper portion 11, a portion facing the other scissors 10 side is a flat surface 11a.

For example, the flat surface 11a is located parallel to the proximal side high step portion 58. Then, when the pair of scissors 10 is closed, the flat surface 11a comes into surface contact with the proximal side high step portion 58 of the other scissors 10, thereby restricting the closing operation of the pair of scissors 10.

According to the first embodiment as described above, with regard to at least one of the pair of scissors 10, the width dimension of the electrode region 19 in the plate thickness direction of the scissors 10 varies depends on the position of the scissors 10 in the longitudinal direction. Therefore, a current flowing from the electrode region 19 to the biological tissue can be sufficiently secured. Accordingly, hemostatic capability can be satisfactorily achieved. In addition, the electrode region 19 is formed on the blade surface 13. Accordingly, an intended site in the biological tissue can be more reliably and selectively cauterized.

Moreover, the outer surface 15 that is likely to touch the biological tissue is covered with the insulating film 12. Accordingly, the outer surface 15 is sufficiently insulated, and it is possible to preferably suppress a possibility that the biological tissue may be erroneously cauterized by the outer surface 15.

### [Second Embodiment]

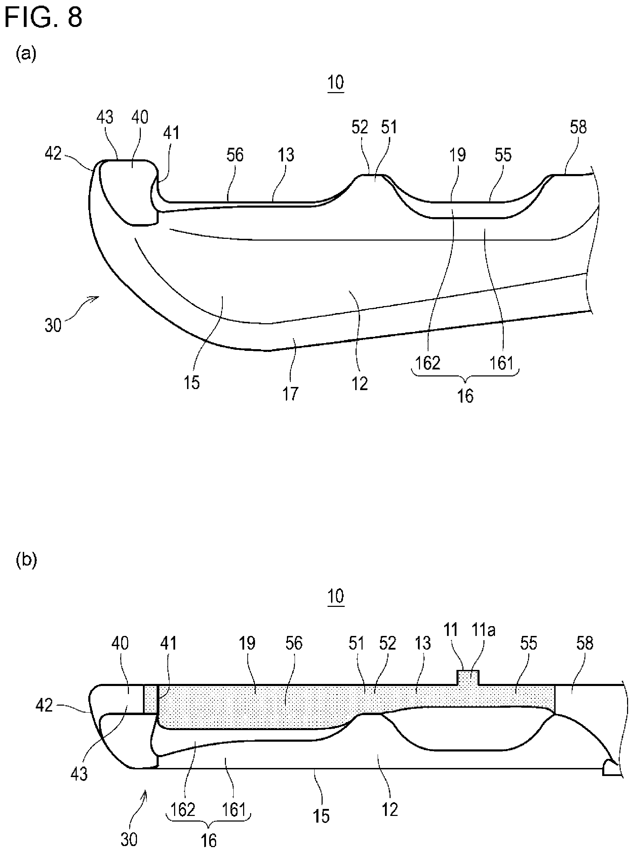

Next, a second embodiment will be described with reference to Figs. 8(a) to 9(b).

A high frequency treatment device according to the present embodiment is different from the high frequency treatment device 200 according to the first embodiment in the following points. Other points are configured to be the same as those of the high frequency treatment device 200 according to the first embodiment.

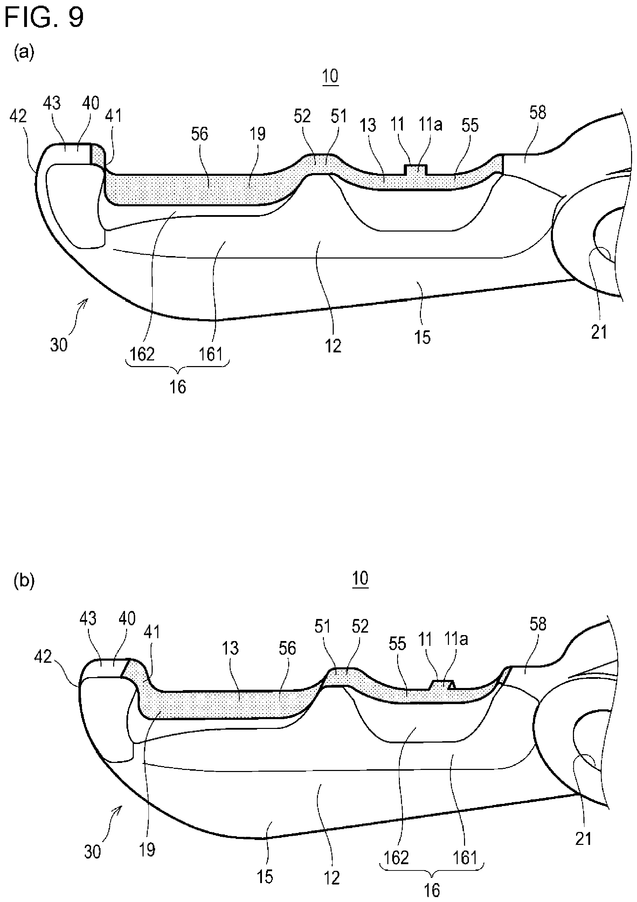

In each of Figs. 8(b), 9(a), and 9(b), the electrode formation region (electrode region 19) in the scissors 10 is hatched in a dot shape. In each of Figs. 5(a), 6(a), 6(b), 7(a), and 7(b), a region which is not hatched in the dot shape is the formation region of the insulating film 12 (non-conductive layer) . However, the insulating film 12 may not be formed inside the first shaft support hole 21.

In a case of the present embodiment, unlike the first embodiment, the number of the projecting portions 51 in the scissors 10 is one. In addition, the recessed portions 55 and 56 are respectively located adjacent to the proximal side and the distal side of the projecting portion 51. That is, unlike the first embodiment, the number of recessed portions is two.

In a case of the present embodiment, the distal claw portion 40, the recessed portion 56, the projecting portion 51, the recessed portion 55, and the proximal side high step portion 58 are sequentially located from the distal side of the distal piece 30 in the end edge on the side in the closing direction of the distal piece 30.

In a case of the present embodiment, the blade surface 13 includes the top surface 52 of the projecting portion 51 and the surfaces of the recessed portions 55 and 56. More specifically, in a case of the present embodiment, the blade surface 13 is continuously formed over the recessed portion 56, the projecting portion 51, and the recessed portion 55.

The electrode region 19 is formed over the entire region of the blade surface 13 (entire region of the surface of the recessed portion 55, the top surface 52 of the projecting portion 51, and the surface of the recessed portion 56).

Each of the distal claw portion 40, the recessed portion 56, the projecting portion 51, the recessed portion 55, and the proximal side high step portion 58 has a predetermined width in the thickness direction of the scissors 10.

Each of the recessed portion 56 and the recessed portion 55 is formed in an elongated shape in the distal-proximal direction of the distal piece 30 (rightward-leftward direction in Figs. 8(a) and 8(b)).

For example, the blade surfaces 13 are respectively formed to be flat on the bottom surface of the recessed portion 55, the bottom surface of the recessed portion 56, and the top surface 52 of the projecting portion 51. For example, the bottom surface of the recessed portion 55, the bottom surface of the recessed portion 56, and the top surface 52 of the projecting portion 51 are located substantially parallel to each other. The bottom surface of the recessed portion 56 and the bottom surface of the recessed portion 55 extend in the distal-proximal direction of the distal piece 30.

In a case of the present embodiment, the inclined surface 16 is configured to include a first inclined surface 161 located on the outer surface 15 side and a second inclined surface 162 located on the blade surface 13 side.

An angle formed between the blade surface 13 and the first inclined surface 161 is larger than an angle formed between the blade surface 13 and the first inclined surface 161. In addition, an angle formed between the outer surface 15 and the first inclined surface 161 is larger than an angle formed between the outer surface 15 and the second inclined surface 162.

For example, the second inclined surface 162 is formed between the recessed portion 56 and the outer surface 15 and between the recessed portion 55 and the outer surface 15, and is not formed between the top surface 52 and the outer surface 15. That is, for example, only the first inclined surface 161 is formed between the top surface 52 and the outer surface 15.

On the other hand, the first inclined surface 161 is continuously present between the recessed portion 56 and the outer surface 15, between the top surface 52 and the outer surface 15, and between the recessed portion 55 and the outer surface 15.

As illustrated in Fig. 8(b), the electrode region 19 is formed to be wider toward the distal side of the scissors 10. That is, when the length of the electrode region 19 in the distal-proximal direction of the distal piece 30 is equally divided into two, an average width dimension of the electrode region 19 on the distal side is larger than an average width dimension of the electrode region 19 on the proximal side.

In the present embodiment, the electrode region 19 may be wider toward the distal side of the scissors 10 in a stepwise manner, or the width dimension of the electrode region 19 may be continuously wider toward the distal side.

More specifically, the width dimension of the recessed portion 56 (width dimension of the electrode region 19 in the recessed portion 56) is larger than the width dimension of the recessed portion 55 (width dimension of the electrode region 19 in the recessed portion 55).

The distal side portion of the scissors 10 is used to excise the biological tissue by entering a mucous membrane when the biological tissue is excised. Accordingly, this configuration can meet requirements for achieving an advantageous effect of improving hemostatic capability by increasing a current flowing from the electrode region 19 to the biological tissue.

In addition, the scissors 10 have the projecting portion 51 projecting toward the other scissors 10 in an intermediate portion in the longitudinal direction of the scissors 10. The electrode region 19 is formed to be wider in the distal side portion (recessed portion 56) of the projecting portion 51 on the blade surface 13 than in the proximal side portion (recessed portion 55) of the projecting portion 51.

As illustrated in Fig. 8(b), the blade surface 13 is wider on the distal side of the scissors 10 than on the proximal side, and the electrode region 19 is wider in a relatively wide portion on the blade surface 13 than in a relatively narrow portion on the blade surface 13.

More specifically, the electrode region 19 is formed in the entire region in the width direction of the blade surface 13. Accordingly, the width dimension of the electrode region 19 is the same as the width dimension of the blade surface 13.

In addition, the scissors 10 have the projecting portion 51 projecting toward the other scissors 10 in an intermediate portion in the longitudinal direction of the scissors 10. The blade surface 13 is formed to be wider in the distal side portion (recessed portion 56) of the projecting portion 51 than in the proximal side portion (recessed portion 55) of the projecting portion 51.

In addition, the width dimensions of the blade surface 13 are substantially constant in the distal side portion (recessed portion 56) of the projecting portion 51 and the proximal side portion (recessed portion 55) of the projecting portion 51.

That is, the width dimension of the recessed portion 56 and the width dimension of the blade surface 13 are substantially constant over the entire region in the distal-proximal direction of the recessed portion 56, and the width dimension of the recessed portion 55 and the width dimension of the blade surface 13 are substantially constant over the entire region in the distal-proximal direction of the recessed portion 55.

Even in a case of the present embodiment, the insulating films 12 are respectively formed on the distal surface 42 which is a surface facing the distal side in the distal claw portion 40, and on the side surface of the distal claw portion 40. The electrode region 19 is not formed on the distal surface 42 and the side surface of the distal claw portion 40.

However, the insulating film 12 is not formed in a portion (portion on the recessed portion 56 side) of the top surface 43 of the distal claw portion 40, and the electrode region 19 is formed therein (refer to Figs. 9(a) and 9(b)). In addition, the insulating film 12 is not formed on the proximal surface 41 which is a surface facing the proximal side in the distal claw portion 40, and the electrode region 19 is formed therein (refer to Fig. 9(b)) .

Even in a case of the present embodiment, the stopper portion 11 is formed in at least one scissors 10 of the pair of scissors 10, and the closing operation of the pair of scissors 10 is restricted by the stopper portion 11 coming into contact with the blade surface 13 of the other scissors 10.

More specifically, the stopper portion 11 is formed in each of the pair of scissors 10. The stopper portion 11 of the scissors 10a comes into contact with the blade surface 13 of the scissors 10b, and the stopper portion 11 of the scissors 10b comes into contact with the blade surface 13 of the scissors 10a, thereby restricting the closing operation of the pair of scissors 10.

In a case of the present embodiment, for example, the stopper portion 11 is located in the intermediate portion in the longitudinal direction of the recessed portion 55. For example, the flat surface 11a is located to be flush with the bottom surface of the recessed portion 55. Then, when the pair of scissors 10 is closed, the flat surface 11a comes into surface contact with the bottom surface of the recessed portion 55 of the other scissors 10, thereby restricting the closing operation of the pair of scissors 10.

For example, the insulating film 12 is not formed on the flat surface 11a, and the flat surface 11a is also a portion of the electrode region 19.

However, the flat surface 11a of the stopper portion 11 is not included in the blade surface 13.

### [Third Embodiment]

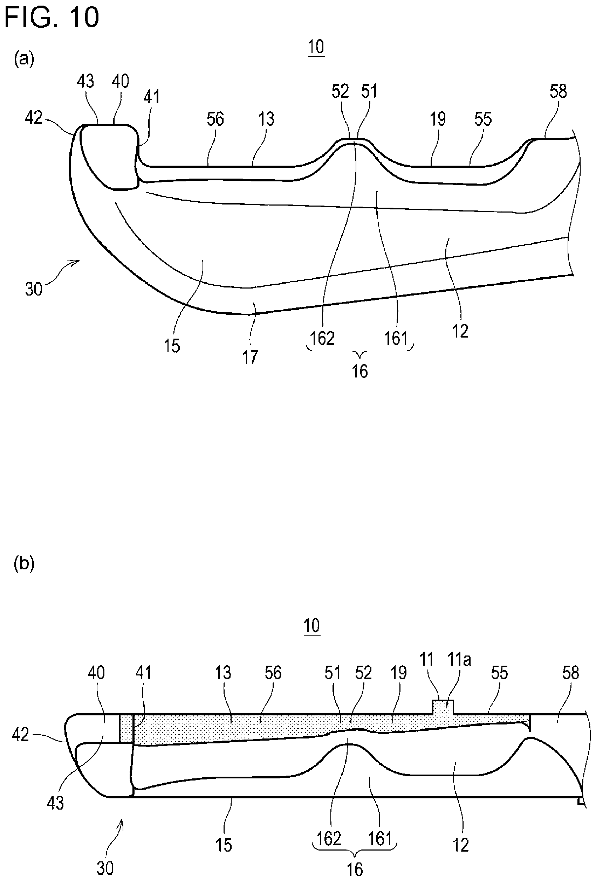

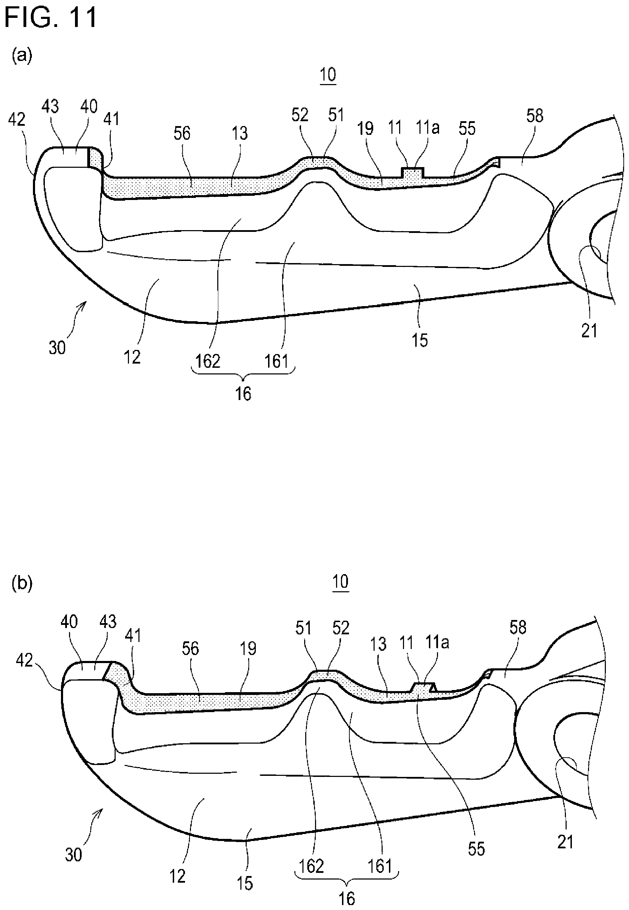

Next, a third embodiment will be described with reference to Figs. 10(a) to 11(b).

A high frequency treatment device according to the present embodiment is different from the high frequency treatment device according to the above-described second embodiment in the following points. Other points are configured to be the same as those of the high frequency treatment device according to the above-described second embodiment.

As illustrated in Fig. 10(b), in a case of the present embodiment, the width dimension of the blade surface 13 is changed to be continuously wider from the proximal side toward the distal side of the scissors 10.

More specifically, the width dimension of the blade surface 13 is changed to be continuously wider from the proximal side toward the distal side of the scissors 10 in substantially the entire region in the distal-proximal direction of the blade surface 13.

Therefore, the electrode region 19 is wider toward the distal side on the surface (top surface 52) of the projecting portion 51.

In a case of the present embodiment, the second inclined surface 162 is formed between the top surface 52 of the projecting portion 51 and the outer surface 15. Then, the second inclined surface 162 is continuously present between the recessed portion 56 and the outer surface 15, between the top surface 52 and the outer surface 15, and between the recessed portion 55 and the outer surface 15 (Figs. 10(a) to 11(b)).

Hitherto, the first to third embodiments have been described with reference to the drawings. However, these are examples of the first aspect of the present invention, and various configurations other than those examples described above can be adopted.

For example, in the above-described first embodiment, an example has been described in which the surfaces of the recessed portions 55, 56, and 57 are located parallel to the rotary shaft of the scissors 10. However, the first aspect of the present invention is not limited to this example. At least a portion of the surfaces of the recessed portions 55, 56, 57 may be inclined downward from the sliding contact surface 14 side toward the outer surface 15 side.

That is, in addition to the blade surface 13, each of the pair of scissors 10 has the sliding contact surface 14 that comes into sliding contact with each other, and the outer surface 15 that is the rear surface with respect to the sliding contact surface 14. The surface of the recessed portions 55, 56, and 57 (at least a portion of the surfaces of the recessed portions 55, 56, and 57) may be inclined from the sliding contact surface 14 side toward the outer surface 15 side in a direction away from the other scissors 10.

In addition, in the above-described second and third embodiments, an example has been described in which the number of the projecting portions 51 of the scissors 10 is one. However, the number of the projecting portions 51 of the scissors 10 may be two or more. Then, the electrode region 19 may be wider toward the distal side by using each of the projecting portions 51 as a boundary.

That is, the scissors 10 may have the plurality of projecting portions 51 located at mutually different positions in the longitudinal direction of the scissors 10, and the electrode region 19 may be formed to be wider toward the distal side in a stepwise manner from each of the plurality of projecting portions 51 serving as the boundary.

In addition, the above-described first to third embodiments can be appropriately combined with each other within the scope not departing from the gist of the first aspect of the present invention.

At least one of the first to third embodiments includes the following technical concept.
(1) There is provided a medical high frequency treatment device, a distal portion of which including a high frequency treatment device knife having a pair of scissors so as to incise a biological tissue.
   Each of the pair of scissors is formed in an elongated plate shape.
   Proximal portions of the pair of scissors are axially supported by each other in a pivot shaft intersecting a plate surface direction of the scissors.
   The pair of scissors is configured to be capable of shearing the biological tissue by pivoting in a direction closer to each other.
   Each of the pair of scissors has a blade surface.
   Each surface of the pair of scissors includes a formation region of a non-conductive layer, and an electrode region where the non-conductive layer is not formed on a surface of the blade surface.
   With regard to at least one of the pair of scissors, a width dimension of the electrode region in a plate thickness direction of the scissors varies depending on a position of the scissors in a longitudinal direction.
(2) In the high frequency treatment device according to (1), the scissors have a projecting portion projecting toward the other scissors side in an intermediate portion in the longitudinal direction of the scissors, and
   a recessed portion located adj acent to the projecting portion in the longitudinal direction of the scissors, and recessed toward a side away from the other scissors.
   The blade surface includes a top surface of the projecting portion and a surface of the recessed portion.
   The electrode regions are respectively formed on the top surface of the projecting portion and the surface of the recessed portion.
(3) In the high frequency treatment device according to (2), the recessed portion is recessed in an arc shape.
(4) In the high frequency treatment device according to (2) or (3), in addition to the blade surface, each of the pair of scissors has a sliding contact surface that comes into sliding contact with each other, and an outer surface that is a rear surface with respect to the sliding contact surface.
   The surface of the recessed portion is inclined from the sliding contact surface side to the outer surface side in a direction away from the other scissors.
(5) In the high frequency treatment device according to (3) or (4), the scissors have a plurality of recessed portions located with the projecting portion as a boundary.
   The recessed portion on the distal side has a larger width dimension of the electrode region.
(6) In the high frequency treatment device according to any one of (2) to (5), the scissors have a plurality of projecting portions disposed at mutually different positions in the longitudinal direction of the scissors.
   The widths of the electrode regions on the top surfaces of the plurality of projecting portions are equal to each other.
(7) In the high frequency treatment device according to any one of (2) to (6), a width of the electrode region on the top surface of the projecting portion is equal to or smaller than a width of a narrowest portion in the electrode region other than the projecting portion.
(8) In the high frequency treatment device according to any one of (1) to (5), the electrode region is formed to be wider toward the distal side of the scissors.
(9) In the high frequency treatment device according to (8), the scissors have a projecting portion projecting toward the other scissors side in an intermediate portion in the longitudinal direction of the scissors.
   The electrode region is formed to be wider in a distal side portion of the projecting portion than in a proximal side portion of the projecting portion on the blade surface.
(10) In the high frequency treatment device according to (9), the scissors have a plurality of the projecting portions located at mutually different positions in the longitudinal direction of the scissors.
   The electrode region is formed to be wider toward the distal side in a stepwise manner from each of the plurality of projecting portions serving as a boundary.
(11) In the high frequency treatment device according to (9) or (10), the electrode region is wider toward the distal side on the surface of the projecting portion.
(12) In the high frequency treatment device according to any one of (1) or (11), the blade surface is wider on the distal side than on the proximal side of the scissors.
   The electrode region is wider in a relatively wide portion on the blade surface than in a relatively narrow portion on the blade surface.
(13) In the high frequency treatment device according to (12), the scissors have a projecting portion projecting toward the other scissors side in an intermediate portion in the longitudinal direction of the scissors.
   The blade surface is formed to be wider in a distal side portion of the projecting portion than in a proximal side portion of the projecting portion.
(14) In the high frequency treatment device according to (13), the width dimensions of the blade surfaces are respectively and substantially constant in the distal side portion of the projecting portion and in the proximal side portion of the projecting portion.
(15) In the high frequency treatment device according to (12) or (13), the width dimension of the blade surface is changed to be continuously wider from the proximal side toward the distal side of the scissors.

### [Fourth Embodiment]

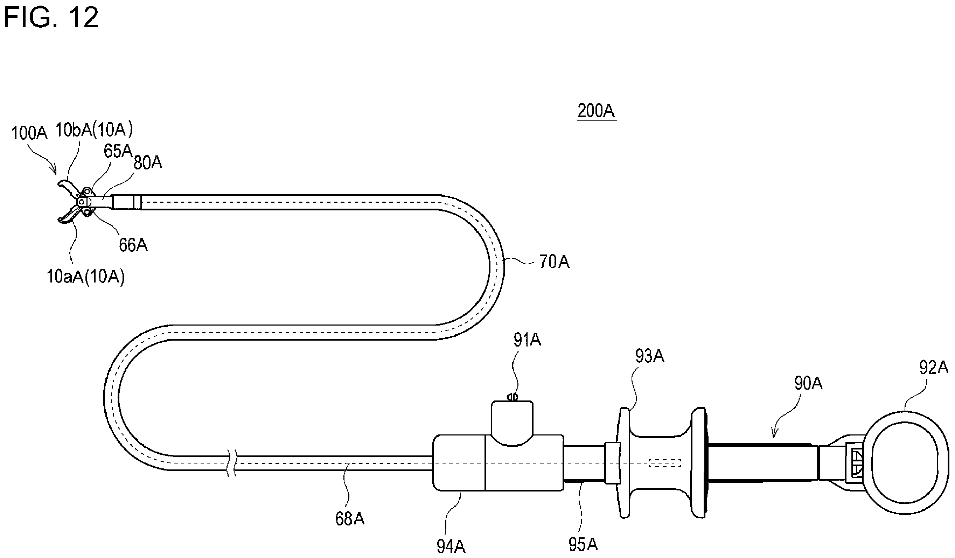

First, a fourth embodiment will be described with reference to Figs. 12 to 18.

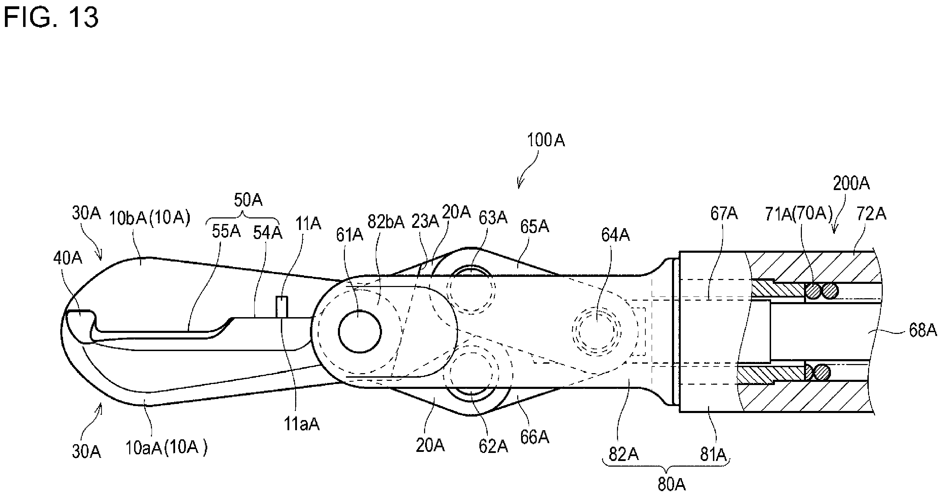

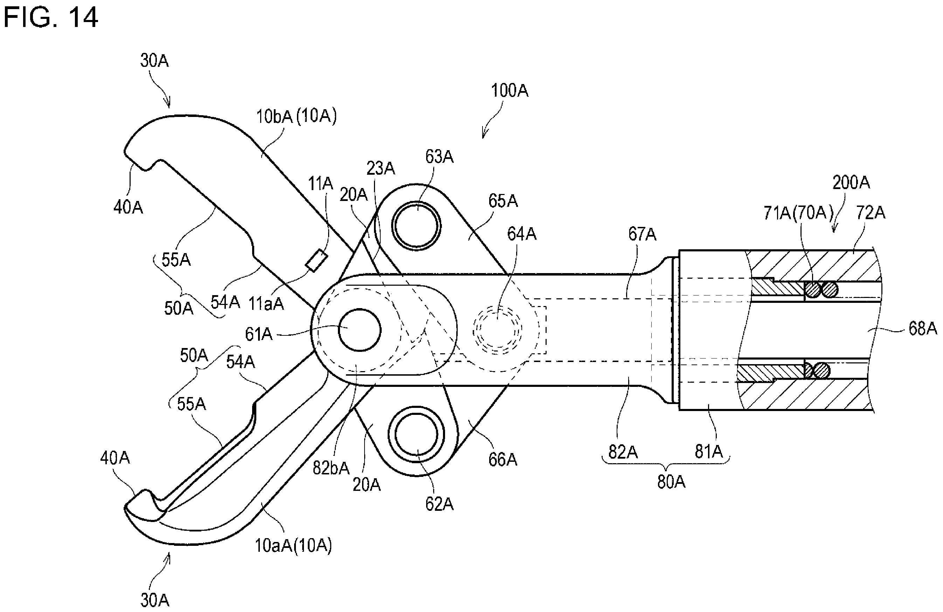

In side views illustrated in Figs. 13 and 14, a proximal side portion in an illustrated range in a sheath 70A indicates a side cross section taken along a center line.

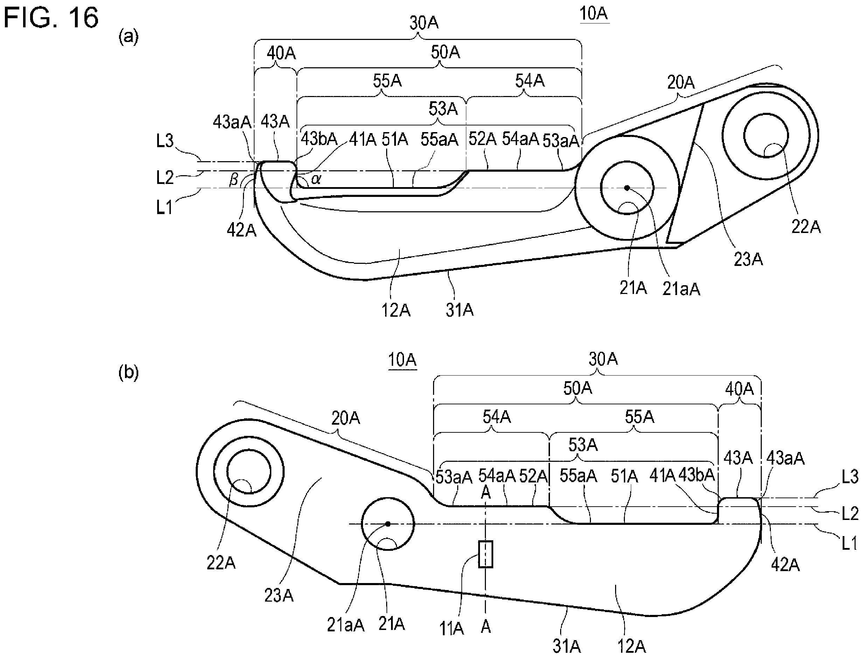

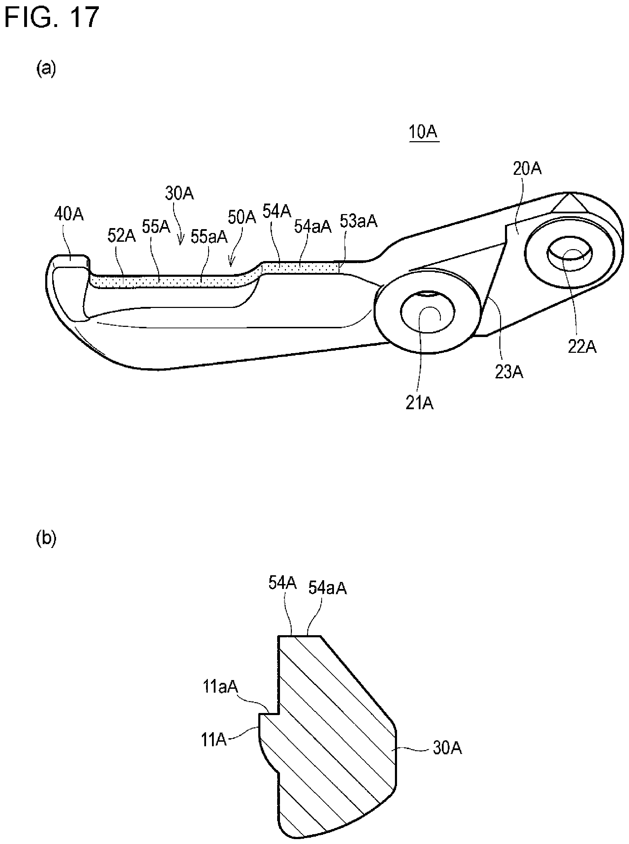

In Fig. 17(a), a formation region of an electrode 52A (electrode formation region 53 (Figs. 16(a) and 16(b))) in the blade portion 50A is hatched in a dot shape.

The electrode 52A is continuously formed over electrode proximal positions 53aA located in proximal portions of a proximal side edge side 41A, a low step portion 55A, and a high step portion 54A in a distal claw portion 40A (to be described later).

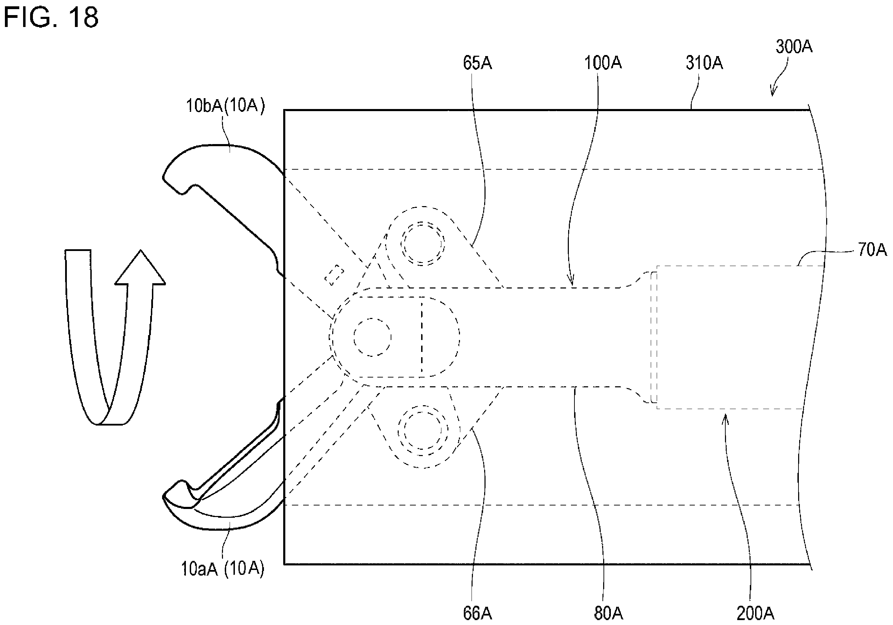

A high frequency treatment device knife 100A according to the present embodiment is disposed in a distal portion of a medical high frequency treatment device 200A (hereinafter, simply referred to as a high frequency treatment device 200A in some cases), and is the high frequency treatment device knife 100A used by being inserted into a forceps hole (not illustrated) of an endoscope 300A (Fig. 18 illustrates a distal side portion in a hood 310A of the distal portion of the endoscope 300A) to incise a biological tissue.

The high frequency treatment device knife 100A includes a pair of shearing scissors 10A axially supported by a common rotary shaft (shaft member 61A illustrated in Figs. 13 and 14), capable of opening and closing each other, and respectively having a blade portion 50A for shearing the biological tissue.

As illustrated in Figs. 16(a), 16(b), and 17(a), each of the pair of shearing scissors 10A has a proximal piece 20A formed on the proximal side of the shearing scissors 10A and axially supported by the above-described the rotary shaft, a distal claw portion 40A formed in the distal end of the shearing scissors 10A, and a blade portion 50A formed between the distal claw portion 40A and the proximal piece 20A in the shearing scissors 10A.

An electrode 52A is formed in the blade portion 50A. Based on a virtual straight line L1 connecting an axis center 21aA of the above-described rotary shaft and a bottom 51A of the blade portion 50A to each other, a height of a highest position of a formation region (electrode formation region 53A) of the electrode 52A in the blade portion 50A is lower than a height of the distal claw portion 40A.

Fig. 16 (a) illustrates a straight line L2 and a straight line L3 which are respectively parallel to the virtual straight line L1. Out of the lines, the straight line L2 is a straight line passing through the highest position in the formation region (electrode formation region 53A) of the electrode 52A in the blade portion 50A. The straight line L3 is a straight line passing through the highest position in the distal claw portion 40A.

Based on the virtual straight line L1, the height of the highest position in the formation region of the electrode 52A in the blade portion 50A is lower than the height of the distal claw portion 40A. In other words, this means that a distance between the virtual straight line L1 and the straight line L2 is shorter than a distance between the virtual straight line L1 and the straight line L3.

In addition, the distal portion of the medical high frequency treatment device 200A according to the present embodiment has the high frequency treatment device knife 100A according to the present embodiment. The proximal side has hand operation unit (operation unit) 90A for performing the opening and closing operation on the pair of shearing scissors 10A.

According to the present embodiment, based on the virtual straight line L1, the height of the highest position in the formation region (electrode formation region 53A) of the electrode 52A in the blade portion 50A is lower than the height of the distal claw portion 40A. Accordingly, when the pair of shearing scissors 10A is closed, an operation of the blade portion 50A for pushing back the biological tissue is suppressed. Therefore, when the pair of shearing scissors 10A is closed, the distal claw portion 40A can be quickly bitten into the biological tissue, and the biological tissue can be more reliably gripped by the pair of shearing scissors 10A.

As illustrated in Fig. 12, the high frequency treatment device 200A includes an elongated operation wire 68A, the high frequency treatment device knife 100A disposed in the distal end of the operation wire 68A, and a flexible sheath 70A that accommodates the operation wire 68A, and a hand operation unit 90A which is disposed on the proximal side of the sheath 70A and to which the proximal end of the operation wire 68A is connected.

The sheath 70A is an elongated and tubular member that accommodates the operation wire 68A. In a case of the present embodiment, the sheath 70A is a metal coil 71A (Figs. 13 and 14) manufactured by tightly winding a conductive wire such as a stainless wire. An insulating film 72A is tightly disposed on the outer surface of the sheath 70A. However, as the sheath 70A, an insulating tubular member (tube) may be used instead of the metal coil 71A.

The hand operation unit 90A illustrated in Fig. 12 includes a shaft portion 95A into which the operation wire 68A is inserted, a finger ring 92A disposed in the proximal portion of the shaft portion 95A, and a slider 93A to which the proximal end of the operation wire 68A is connected and which moves forward and rearward with respect to the shaft portion 95A. The operation wire 68A is slidably inserted into the shaft portion 95A. For example, a user inserts a thumb into the finger ring 92A, and pinches the slider 93A with other two fingers, thereby driving the slider 93A to move forward and rearward along the longitudinal direction of the shaft portion 95A. In this manner, the operation wire 68A moves forward or rearward with respect to the hand operation unit 90A. The proximal end of the sheath 70A is fixed to the hand operation unit 90A, and the operation wire 68A is inserted into the sheath 70A to be movable forward and rearward. Accordingly, the distal end of the operation wire 68Amoves forward or rearward with respect to the sheath 70A in conjunction with the forward and rearward movement of the slider 93A. In this manner, as will be described later, a forward and rearward movement portion 67A (Figs. 13 and 14) of the high frequency treatment device knife 100A is driven to move forward and rearward, and the pair of shearing scissors 10A is opened and closed.

The axial direction of the rotary shaft of the pair of shearing scissors 10A is a direction perpendicular to the plate surface of the shearing scissors 10A. When the pair of shearing scissors 10A is opened and closed, the inner surfaces illustrated in Fig. 16(b) slide on each other.

As illustrated in Fig. 12, the hand operation unit 90A includes a power supply unit 91. The power supply unit 91A is a terminal for applying a high frequency current to the pair of shearing scissors 10A, and a high frequency power source (not illustrated) is connected thereto through a power cable. The pair of shearing scissors 10A, link pieces 65A and 66A, and the forward and rearward movement portion 67A, which configure the high frequency treatment device knife 100A, are all manufactured using a conductive metal material. In addition, the operation wire 68A is also manufactured using the conductive metal material. Therefore, the high frequency current input to the power supply unit 91A is applied to the pair of shearing scissors 10A.

As illustrated in Figs. 13 and 14, the high frequency treatment device knife 100A includes the pair of plate-shaped shearing scissors 10A, a shaft member 61A that axially supports the shearing scissors 10A to be openable and closable, the two link pieces 65A and 66A, a forward and rearward movement portion 67A, and a holding frame 80A.

The axial direction of the shaft member 61A is a direction perpendicular to the paper surface in Figs. 13 and 14. The axial direction of the shaft member 61A is a direction in which the pair of shearing scissors 10A overlaps each other, in other words, the thickness direction of the pair of shearing scissors 10A.

The pair of shearing scissors 10A is driven to be opened and closed by pushing and pulling the operation wire 68A. The operation wire 68A is manufactured using a conductive metal material such as stainless steel.

The forward and rearward movement portion 67A is integrally connected to the distal end of the operation wire 68A. The two link pieces 65A and 66A are pivotally connected to the forward and rearward movement portion 67A by a shaft member 64A. Furthermore, the proximal piece 20A of one shearing scissors 10A (hereinafter, shearing scissors 10aA) is pivotally connected to the link piece 65A by a shaft member 63A, and the proximal piece 20A of the other shearing scissors 10A (hereinafter, shearing scissors 10bA) is pivotally connected to the link piece 66A by a shaft member 62A.

The axial direction of the respective shaft members 62A, 63A, and 64A is a direction parallel to the axial direction of the shaft member 61A.

The pair of shearing scissors 10A and the link pieces 65A and 66A relatively pivot in a plane illustrated in Figs. 13 and 14 (in a plane perpendicular to the axial direction of the shaft member 61A).

The proximal piece 20A of the pair of shearing scissors 10A and the link pieces 65A and 66A configure a four-joint link having a rhomboid shape.

The shaft members 62A and 63A are located on the distal side of the shaft member 64A, and the shaft member 61A is located on the distal side of the shaft members 62A and 63A.

The holding frame 80A is fixed to the distal end of the sheath 70A.

The holding frame 80A includes a proximal portion 81A fixed to the distal end of the sheath 70A, and a pair of brackets 82A projecting to the distal side from the proximal portion 81A.

For example, the bracket 82A is formed in a plate shape.

The pair of shearing scissors 10A is axially supported by the shaft member 61A with respect to the distal portion of the pair of brackets 82A.

In a gap between the pair of brackets 82A, the proximal piece 20A of the pair of shearing scissors 10A and the link pieces 65A and 66A are respectively rotatable. A portion projecting to the distal side from the sheath 70A in the forward and rearward movement portion 67A is movable forward and rearward.

As illustrated in Fig. 13, when the operation wire 68A and the forward and rearward movement portion 67A are pulled toward the proximal side (rightward in Fig. 13), the pair of shearing scissors 10A is in a closed state. Conversely, as illustrated in Fig. 14, when the operation wire 68A and the forward and rearward movement portion 67A are pushed to the distal side (leftward in Fig. 14), the pair of shearing scissors 10A is in an open state.

Each of the pair of shearing scissors 10A has a substantially L-shape (that is, a sickle shape) that is shallowly bent in a pivot plane in the vicinity of the shaft member 61A. The proximal piece 20A is a proximal side portion of the shaft member 61A in each of the pair of shearing scissors 10A. On the other hand, in each of the pair of shearing scissors 10A, a distal side portion of the shaft member 61A will be referred to as a distal piece 30A.

The shapes of the pair of shearing scissors 10A may be the same as each other, or may be different from each other. In a case of the present embodiment, the pair of shearing scissors 10A has mutually the same shape.

Hereinafter, the shape of the shearing scissors 10A will be described in detail with reference to Figs. 16(a), 16(b), and 17(a).

The distal claw portion 40A is formed in the distal end of the distal piece 30A of the shearing scissors 10A. The distal claw portion 40A projects in a closing direction. The closing direction is a direction from one shearing scissors 10A to the other shearing scissors 10A, and a direction opposite thereto will be referred to as an opening direction.

The distal claw portion 40A is a projection that is bitten into the biological tissue.

The blade portion 50A is formed along an end edge (edge) on the side in the closing direction in the proximal side portion of the distal claw portion 40A in the distal piece 30A.

The biological tissue can be incised by the blade portion 50A of the pair of shearing scissors 10A in a state where the biological tissue is picked and held by the distal claw portion 40 of the pair of shearing scissors 10A so as to prevent the falling of the biological tissue.

An insulating film 12A (Figs. 16(a) and 16(b)) is formed on each surface of the pair of shearing scissors 10A. The insulating film 12A is formed on the entire surface of the distal piece 30A except for at least the formation region of the electrode 52A.

The insulating film 12A can be formed by coating the surface of the shearing scissors 10A with an insulating material such as a fluororesin.

The electrode 52A is a linear portion exposed from the insulating film 12A in the blade portion 50A. The pair of shearing scissors 10A serves as a monopolar high frequency electrode when a high frequency voltage in the same phase is applied thereto from the power supply unit 91A. The high frequency current is applied to the pair of shearing scissors 10A in a state where the biological tissue is gripped by the pair of shearing scissors 10A. In this manner, the biological tissue is cauterized and incised. Instead of the present embodiment, a bipolar high frequency treatment device 200A may be used in which one of the pair of shearing scissors 10A is used as an active electrode and the other is used as a return electrode.

In a case of the present embodiment, a high step portion 54A and a low step portion 55A having a notch shape recessed toward a rear side of the high step portion 54A are formed in the blade portion 50A. In other words, the blade portion 50A has the high step portion 54A having a relatively high height based on the virtual straight line L1 and the low step portion 55A having a relatively low height.

The electrode 52A is formed over the low step portion 55A from the high step portion 54A. The electrode 52A is formed in an electrode formation region 53A illustrated in Figs. 16(a) and 16(b). The electrode formation region 53A is a hatched region in Fig. 17(a).

In a case of the present embodiment, a bottom 51A of the blade portion 50A is a lowest portion of the low step portion 55A.

In a case of the present embodiment, the blade portion 50A has one high step portion 54A and one low step portion 55A. In a case of the present embodiment, the high step portion 54A has a flat portion 54aA that is a flat surface. A bottom portion of the low step portion 55A is a flat portion 55aA that is a flat surface. The flat portion 54aA and the flat portion 55aA are located parallel to each other. The flat portion 54aA and the flat portion 55aA are respectively located parallel to the axial direction of the shaft member 61A. In addition, the flat portion 54aA and the flat portion 55aA are located parallel to the virtual straight line L1.

In addition, a projecting direction end edge 43A (to be described later) of the distal claw portion 40A is also located parallel to the virtual straight line L1.

In a case of the present embodiment, when the high frequency treatment device knife 100A is viewed in the axial direction of the rotary shaft of the pair of shearing scissors 10A (that is, when viewed in a direction of Figs. 13 and 14 or in a direction opposite thereto), an angle α formed between a proximal edge side (hereinafter, a proximal side edge side 41A (Figs. 16(a) and 16(b))) in the distal claw portion 40A and the virtual straight line L1 is equal to or smaller than 90 degrees.

According to this configuration, the biological tissue can be gripped by the distal claw portion 40A with a sufficient gripping force.

More specifically, in a case of the present embodiment, the angle α is 90 degrees.

However, the angle α may be smaller than 90 degrees. That is, the proximal side edge side 41 may overhang the virtual straight line L1.

An angle β formed between a distal side edge side (hereinafter, a distal side edge side 42A (Figs. 16(a) and 16(b))) in the distal claw portion 40A and the virtual straight line L1 may be equal to or larger than 90 degrees, or may be equal to or smaller than 90 degrees.

In a case of the present embodiment, the angle β is approximately 90 degrees.

In addition, in a case of the present embodiment, the distal portion 43aA and the proximal portion 43bA of a projecting direction end edge of the distal claw portion 40A (hereinafter, a projecting direction end edge 43A (Figs. 16(a) and 16(b))) respectively have an R-chamfered shape.

In this manner, the biological tissue can be softly gripped by the distal claw portion 40A.

That is, in a case of the present embodiment, the biological tissue can be softly gripped by the distal claw portion 40A with a sufficient gripping force.

A stopper portion 11A is formed in at least one shearing scissors 10A of the pair of shearing scissors 10A. The closing operation of the pair of shearing scissors 10A is restricted by the stopper portion 11A coming into contact with the blade portion 50A of the other shearing scissors 10A.

In a case of the present embodiment, the stopper portion 11A is formed in each of the pair of shearing scissors 10A. The stopper portion 11A of the shearing scissors 10aA comes into contact with the blade portion 50A of the shearing scissors 10bA, and the stopper portion 11 of the shearing scissors 10bA comes into contact with the blade portion 50A of shearing scissors 10aA, thereby restricting the closing operation of the pair of shearing scissors 10A.

The stopper portion 11A is formed on the inner surface illustrated in Fig. 16(b) in the shearing scissors 10A. As illustrated in Fig. 17(b), in the stopper portion 11A, a portion facing the other shearing scissors 10A side is a flat surface 11aA. The flat surface 11aA is located parallel to the flat portion 54aA and the flat portion 55aA.

The stopper portion 11A is located in a portion having the high step portion 54A, out of a portion having the high step portion 54A and a portion having the low step portion 55A in the distal piece 30A. Then, when the pair of shearing scissors 10A is closed as illustrated in Fig. 13, the flat surface 11aA comes into surface contact with the flat portion 54aA of the other shearing scissors 10A, thereby restricting the closing operation of the pair of shearing scissors 10A.

A first shaft support hole 21A that penetrates the proximal piece 20A in the thickness direction is formed in the distal portion of the proximal piece 20A. A common shaft member 61A is inserted into the first shaft support hole 21A of the pair of shearing scissors 10A, and the pair of shearing scissors 10A is axially supported.

A second shaft support hole 22A that penetrates the proximal piece 20A in the thickness direction is formed in the proximal portion of the proximal piece 20A. The shaft member 63A is inserted into the second shaft support hole 22A of one shearing scissors 10aA and the distal portion of the link piece 65A, and the shearing scissors 10aA and the link piece 65A are rotatably and axially supported by each other. Similarly, the shaft member 62A is inserted into the second shaft support hole 22A of the other shearing scissors 10bA and the distal portion of the link piece 66A, and the shearing scissors 10bA and the link piece 66A are rotatably and axially supported by each other.

As illustrated in Fig. 16(a), a step portion 23A is formed on the outer surface of the proximal piece 20A. In the proximal piece 20A, the proximal side portion of the step portion 23A is thinner than the distal side portion of the step portion 23A.

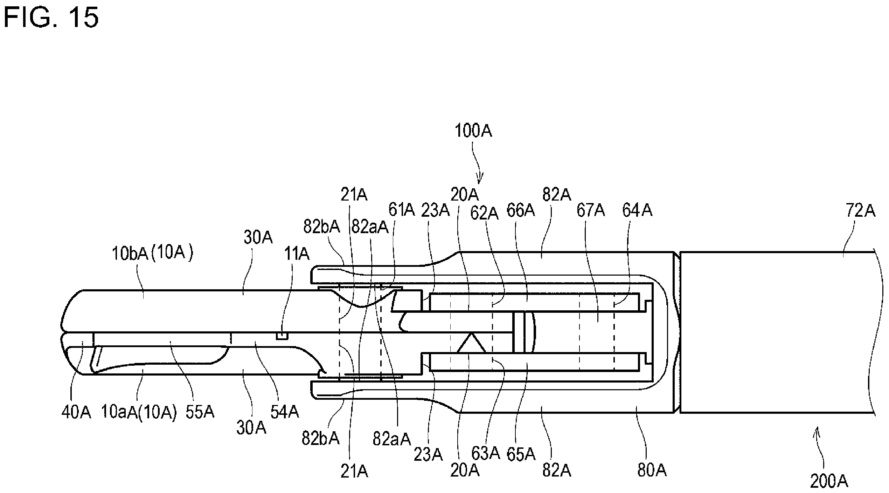

As illustrated in Fig. 15, a thickness difference between the proximal side portion and the distal side portion of the step portion 23A in the proximal piece 20A is set to be slightly larger than the thickness of the link pieces 65A and 66A, or is set to be equal to the thickness of the link pieces 65A and 66A.

As illustrated in Fig. 15, the pair of brackets 82A of the holding frame 80A pinches the proximal piece 20A of the pair of shearing scissors 10A from both sides in the axial direction of the rotary shaft (shaft member 61) of the pair of shearing scissors 10A, and axially supports the proximal piece 20A in the rotary shaft. The pair of brackets 82A pinches and axially supports the proximal piece 20A in the distal portion of the bracket 82A.

An outer surface 82b that is a rear surface with respect to each facing surface 82aA of the pair of brackets 82A is formed to be flat in the distal portion of the pair of brackets 82A. Moreover, a distance between the outer surfaces 82bA of the respective distal portions of the pair of brackets 82 is shorter than a distance between the outer surfaces of the proximal portions of the brackets 82A. That is, the distal portion of the bracket 82A has a flat shape so that the outer surface side is cut.

Therefore, interference between the bracket 82A and the biological tissue or a peripheral wall of the forceps hole can be suppressed. In addition, an advantageous effect can be achieved in that coating the bracket 82A is facilitated.

According to the fourth embodiment described above, based on the virtual straight line L1, the height of the highest position in the formation region (electrode formation region 53A) of the electrode 52A in the blade portion 50A is lower than the height of the distal claw portion 40A. Accordingly, when the pair of shearing scissors 10A is closed, an operation of the blade portion 50A for pushing back the biological tissue is suppressed. Therefore, when the pair of shearing scissors 10A is closed, the distal claw portion 40A can be quickly bitten into the biological tissue, and the biological tissue can be more reliably gripped by the pair of shearing scissors 10A.

### [Fifth Embodiment]

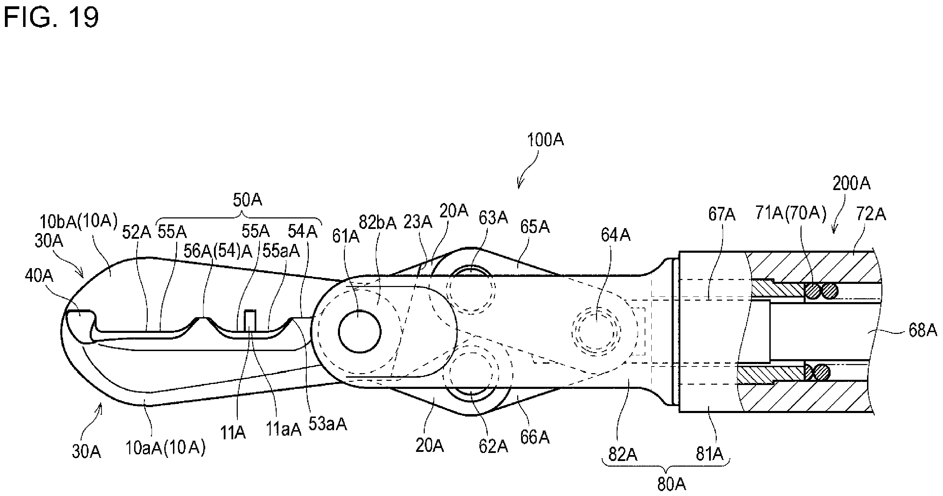

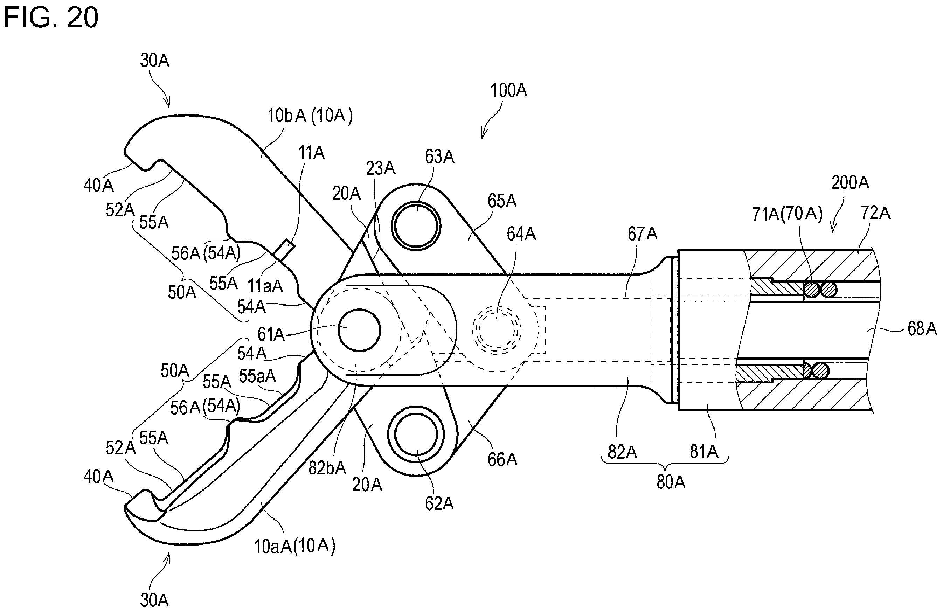

Next, a fifth embodiment will be described with reference to Figs. 19 and 20.

The high frequency treatment device 200A and the high frequency treatment device knife 100A according to the present embodiment are different from the high frequency treatment device 200A and the high frequency treatment device knife 100A according to the above-described fourth embodiment in the following points. Other points are configured to be the same as those of the high frequency treatment device 200A and the high frequency treatment device knife 100A according to the above-described fourth embodiment.

As illustrated in Figs. 19 and 20, in a case of the present embodiment, the blade portion 50A has a plurality of low step portions 55A and an intermediate high step portion 56A which is the high step portion 54A located between the low step portions 55A.

According to the present embodiment, the biological tissue can be gripped by the intermediate high step portion 56A of the pair of shearing scissors 10A. Therefore, the biological tissue can be gripped with a more sufficient gripping force.

In a case of the present embodiment, the blade portion 50A has two low step portions 55A. One intermediate high step portion 56A is located between the two low step portions 55A. In addition, a high step portion 54A is located on the proximal side in addition to the low step portion 55A on the proximal side.

In a case of the present embodiment, for example, an electrode proximal position 53aA is a distal position of a proximal position (high step portion 54A on the proximal side (high step portion 54A which is not the intermediate high step portion 56A)) of the low step portion 55A on the proximal side.

In a case of the present embodiment, the stopper portion 11A is located in a portion where the low step portion 55A on the proximal side is formed in the distal piece 30A. Then, when the pair of shearing scissors 10A is closed as illustrated in Fig. 19, the flat surface 11aA comes into surface contact with the flat portion 55aA of the low step portion 55A on the proximal side of the other shearing scissors 10A, thereby restricting the closing operation of the pair of shearing scissors 10A.

The depths of the plurality (two in a case of the present embodiment) of low step portions 55A may be equal to each other, or may be different from each other. In a case of the present embodiment, the depths of the two low step portions 55A are equal to each other, and the bottom of these two low step portions 55A is located on the virtual straight line.

### [Sixth Embodiment]

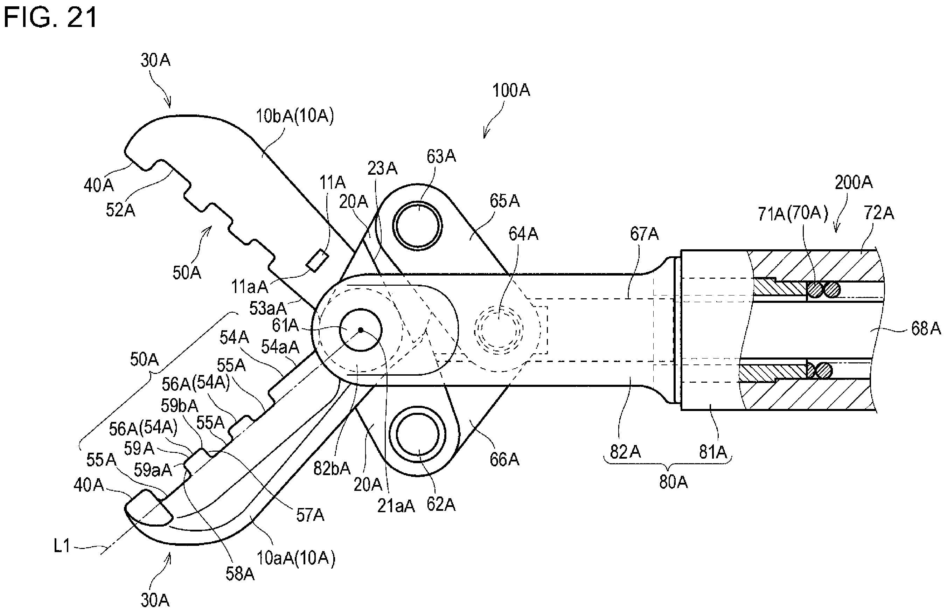

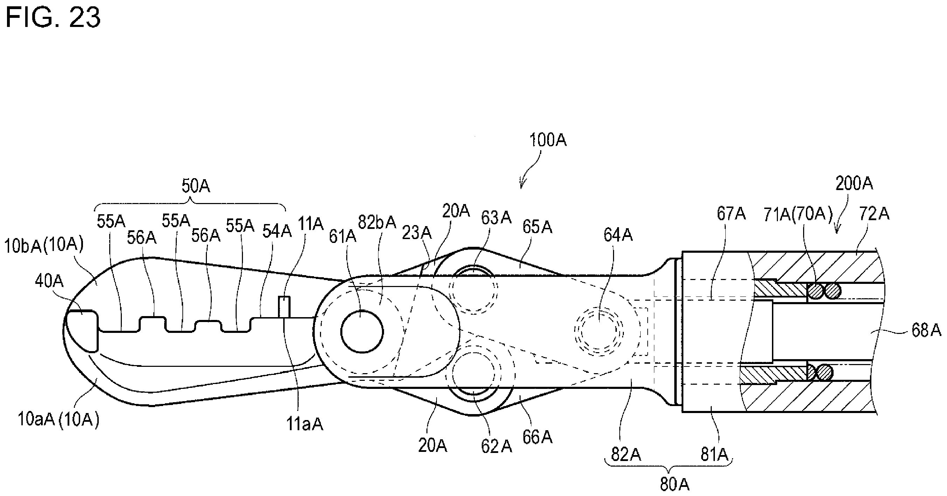

Next, a sixth embodiment will be described with reference to Figs. 21 to 23.

The high frequency treatment device 200A and the high frequency treatment device knife 100A according to the present embodiment are different from the high frequency treatment device 200A and the high frequency treatment device knife 100A according to the above-described fourth embodiment in the following points. Other points are configured to be the same as those of the high frequency treatment device 200A and the high frequency treatment device knife 100A according to the above-described fourth embodiment.

As illustrated in Figs. 21 to 23, in a case of the present embodiment, the blade portion 50A has the plurality of low step portions 55A and the intermediate high step portion 56A which is the high step portion 54A located between the low step portions 55A.

According to the present embodiment, the biological tissue can be gripped by the intermediate high step portion 56A of the pair of shearing scissors 10A. Therefore, the biological tissue can be gripped with a more sufficient gripping force.

In a case of the present embodiment, the blade portion 50A has a plurality of intermediate high step portions 56A. Out of the plurality of intermediate high step portions 56A, the intermediate high step portion 56A located closer to the proximal side has a low height based on the virtual straight line L1.

Therefore, timings at which the biological tissue is pinched between the intermediate high step portions 56A of the pair of shearing scissors 10A can be close to each other.

In a case of the present embodiment, the blade portion 50A has two intermediate high step portions 56A.

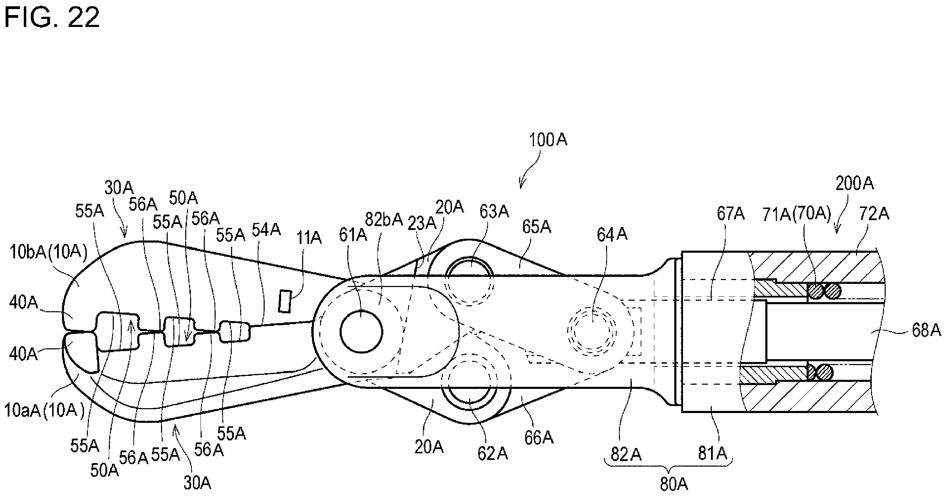

More specifically, as illustrated in Fig. 22, in a case of the present embodiment, the pair of shearing scissors 10A is closed. When the distal claw portions 40A of the pair of shearing scissors 10A start to overlap each other in the axial direction of the rotary shaft of the pair of shearing scissors 10A, the mutually corresponding intermediate high step portions 56A of the pair of shearing scissors 10A come into contact with each other.

Therefore, the timings at which the biological tissue is pinched between the intermediate high step portions 56A of the pair of shearing scissors 10A can be substantially the same timing.

In addition, as illustrated in Fig. 21, when the high frequency treatment device knife 100A is viewed in the axial direction of the rotary shaft, an angle formed between a proximal side edge side (hereinafter, a proximal side edge side 57A) of the intermediate high step portion 56A and the virtual straight line L1 is equal to or smaller than 90 degrees.

According to this configuration, the biological tissue can be gripped by the intermediate high step portion 56A with a more sufficient gripping force.

In a case of the present embodiment, with regard to each of the plurality of intermediate high step portions 56A, the angle formed between the proximal side edge side 57A and the virtual straight line L1 is equal to or smaller than 90 degrees.

More specifically, in a case of the present embodiment, the angle formed between the proximal side edge side 57A and the virtual straight line L1 is 90 degrees. However, this angle may be smaller than 90 degrees.

In addition, when the high frequency treatment device knife 100A is viewed in the axial direction of the rotary shaft, an angle formed between a distal side edge side (hereinafter, a distal side edge side 58) of the intermediate high step portion 56A and the virtual straight line L1 is equal to or smaller than 90 degrees.

According to this configuration, the biological tissue can be gripped by the intermediate high step portion 56A with a more sufficient gripping force.

In a case of the present embodiment, with regard to each of the plurality of intermediate high step portions 56A, the angle formed between the distal side edge side 58A and the virtual straight line L1 is equal to or smaller than 90 degrees.

More specifically, in a case of the present embodiment, the angle formed between the intermediate high step portion 56 and the virtual straight line L1 is 90 degrees. However, this angle may be smaller than 90 degrees.

In addition, the distal portion 59a and the proximal portion 59bA in the projecting direction end edge (hereinafter, projecting direction end edge 59A) of the intermediate high step portion 56A respectively have an R-chamfered shape.

In this manner, the biological tissue can be softly gripped by the intermediate high step portion 56A.

That is, in a case of the present embodiment, the biological tissue can be softly gripped with a sufficient gripping force by the intermediate high step portion 56A.

Hitherto, the fourth to sixth embodiments have been described with reference to the drawings. However, the embodiments are only examples of one or both of the second and third aspects of the present invention, and various configurations other than those examples described above can be adopted.

For example, in the above description, an example has been described in which the angle formed between the proximal side edge side 41A of the distal claw portion 40A and the virtual straight line L1 is equal to or smaller than 90 degrees. However, the angle may be equal to or larger than 90 degrees.

In addition, in the above description, an example has been described in which the low step portion 55A has the flat portion 55aA. However, the shape of the low step portion 55A when viewed in the direction of the rotary shaft of the shearing scissors 10A may be a notch shape having an arc shape.

In addition, the fourth to sixth embodiments can be appropriately combined with each other within the scope not departing from the gist of the second or third aspect of the present invention.

At least one form of the fourth to sixth embodiments includes the following technical concept.
(1) There is provided a high frequency treatment device knife disposed in a distal portion of the medical high frequency treatment device, and used by being inserted into a forceps hole of an endoscope so as to incise a biological tissue.
   The high frequency treatment device knife includes a pair of shearing scissors axially supported by a common rotary shaft, capable of opening and closing each other, and each having a blade portion for shearing the biological tissue.
   Each of the pair of shearing scissors has
   a proximal piece formed on a proximal side of the shearing scissors and axially supported by the rotary shaft,
   a distal claw portion formed in a distal end of the shearing scissors, and
   a blade portion formed between the distal claw portion and the proximal piece in the shearing scissors.
   An electrode is formed in the blade portion.
   Based on a virtual straight line connecting an axis center of the rotary shaft and a bottom of the blade portion to each other, a height of a highest position of the formation region of the electrode in the blade portion is lower than a height of the distal claw portion.
(2) In the high frequency treatment device knife according to (1), when the high frequency treatment device knife is viewed in the axial direction of the rotary shaft, an angle formed between a proximal side edge side in the distal claw portion and the virtual straight line is equal to or smaller than 90 degrees.
(3) In the high frequency treatment device knife according to (1) or (2), the distal portion and the proximal portion in a projecting direction end edge of the distal claw portion respectively have an R-chamfered shape.
(4) In the high frequency treatment device knife according to any one of (1) to (3), a stopper portion is formed in at least one of the pair of shearing scissors.
   A closing operation of the pair of shearing scissors is restricted by the stopper portion coming into contact with the blade portion of the other shearing scissor.
(5) In the high frequency treatment device knife according to any one of (1) to (4), the blade portion has a high step portion and a low step portion having a notch shape recessed toward a rear side of the high step portion.
   An electrode is formed over the low step portion from the high step portion.
   The bottom of the blade portion is a lowest portion of the low step portion.
(6) In the high frequency treatment device knife according to (5), the blade portion has a plurality of the low step portions and an intermediate high step portion that is the high step portion located between the low step portions.
(7) In the high frequency treatment device knife according to (6), the blade portion has a plurality of the intermediate high step portions.
   Out of the plurality of intermediate high step portions, the intermediate high step portion located on the proximal side has a lower height based on the virtual straight line.
(8) In the high frequency treatment device knife according to (6) or (7), when the pair of shearing scissors is closed and the distal claw portions of the pair of shearing scissors start to overlap each other in the axial direction of the rotary shaft, the mutually corresponding intermediate high step portions of the pair of shearing scissors come into contact with each other.
(9) In the high frequency treatment device knife according to any one of (6) to (8), when the high frequency treatment device knife is viewed in the axial direction of the rotary shaft, an angle formed between a proximal side edge side in the intermediate high step portion and the virtual straight line is equal to or smaller than 90 degrees.
(10) In the high frequency treatment device knife according to any one of (6) to (9), when the high frequency treatment device knife is viewed in the axial direction of the rotary shaft, an angle formed between a distal side edge side in the intermediate high step portion and the virtual straight line is equal to or smaller than 90 degrees.
(11) In the high frequency treatment device knife according to any one of (6) to (10), the distal portion and the proximal portion in a projecting direction end edge of the intermediate high step portion respectively have an R-chamfered shape.
(12) The high frequency treatment device knife according to any one of (1) to (11) includes a pair of brackets that pinches the proximal piece of the pair of shearing scissors from both sides in the axial direction of the rotary shaft, and that axially supports the proximal piece in the rotary shaft.
   The pair of brackets pinches and axially supports the proximal piece in the distal portion of the bracket.
   In each distal portion of the pair of brackets, an outer surface that is a rear surface with respect to each facing surface of the pair of brackets is formed to be flat.
   A distance between the outer surfaces of the respective distal portions of the pair of brackets is shorter than a distance between the outer surfaces of the proximal portions of the brackets.
(13) There is provided a medical high frequency treatment device, a distal portion of which has a high frequency treatment device knife according to any one of (1) to (12), and a proximal side of which has an operation unit for performing an opening and closing operation on the pair of shearing scissors.

### [Seventh Embodiment]

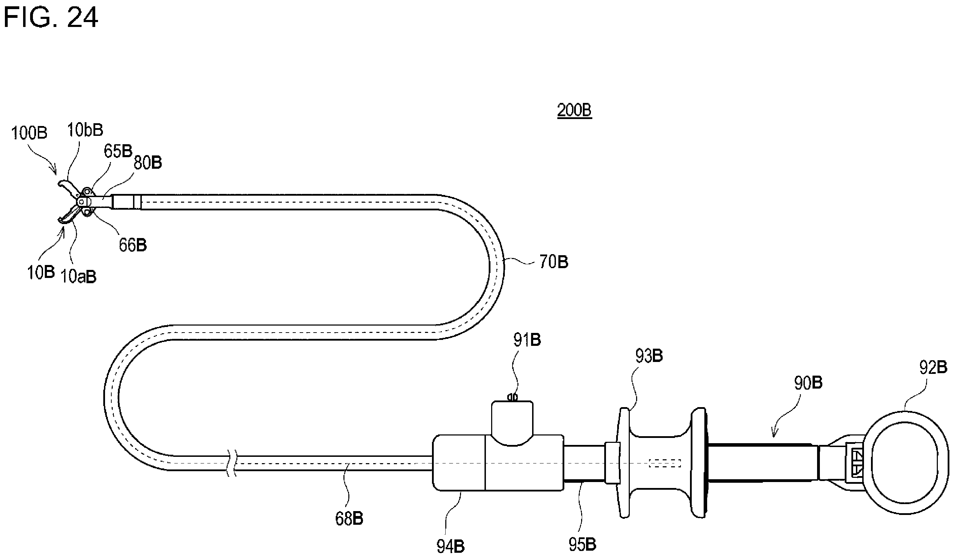

First, a seventh embodiment will be described with reference to Figs. 24 to 29.

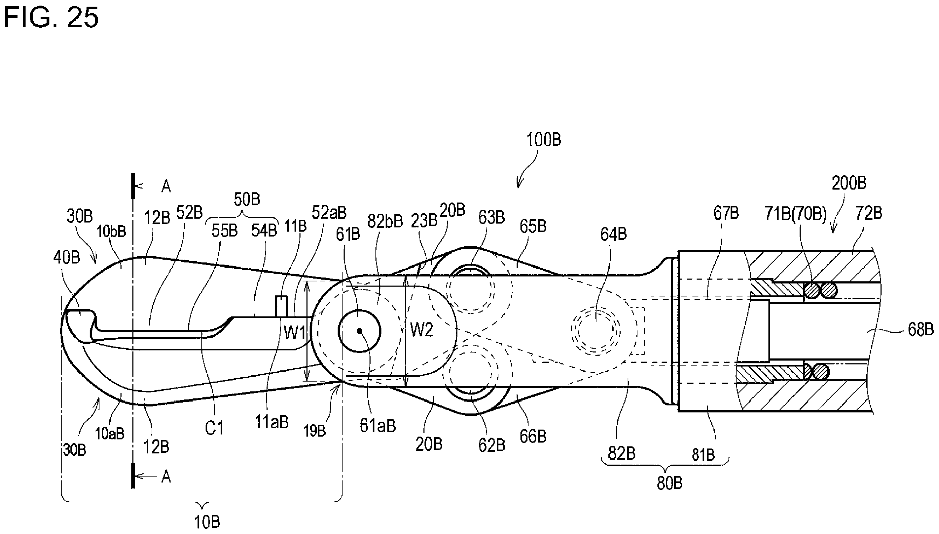

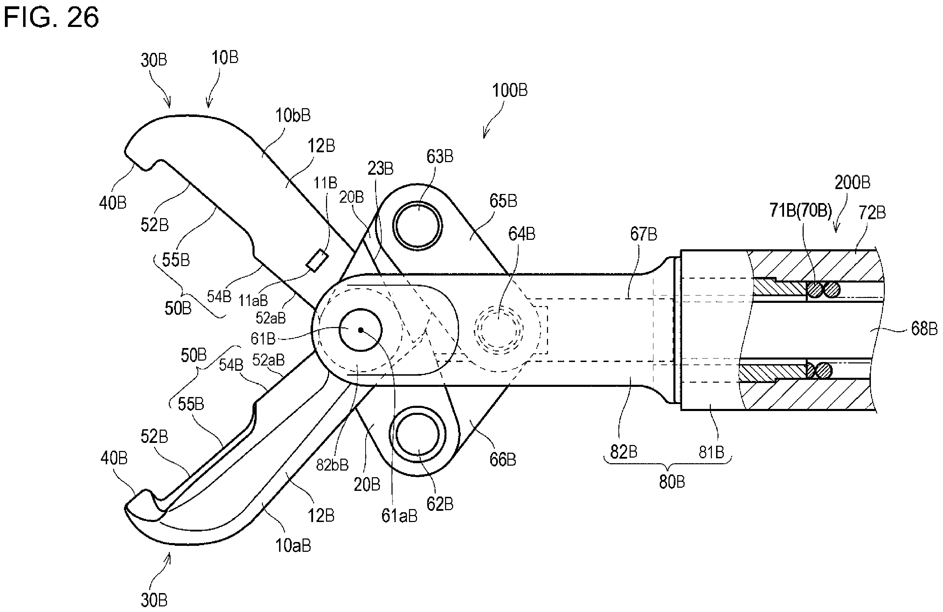

In side views illustrated in Figs. 25 and 26, a proximal side portion in an illustrated range in a sheath 70B indicates a side cross section taken along a center line.

In the following description, a distal direction of a distal treatment unit 10B is a direction from an axis center 61aB of a rotary shaft (shaft member 61B) toward a distal end (left end of the distal treatment unit 10B in Fig. 25) of the distal treatment unit 10B (opening and closing portions 10aB and 10bB), and a proximal direction of the distal treatment unit 10B is a direction opposite thereto. Therefore, a distal-proximal direction is a rightward-leftward direction in Fig. 25.

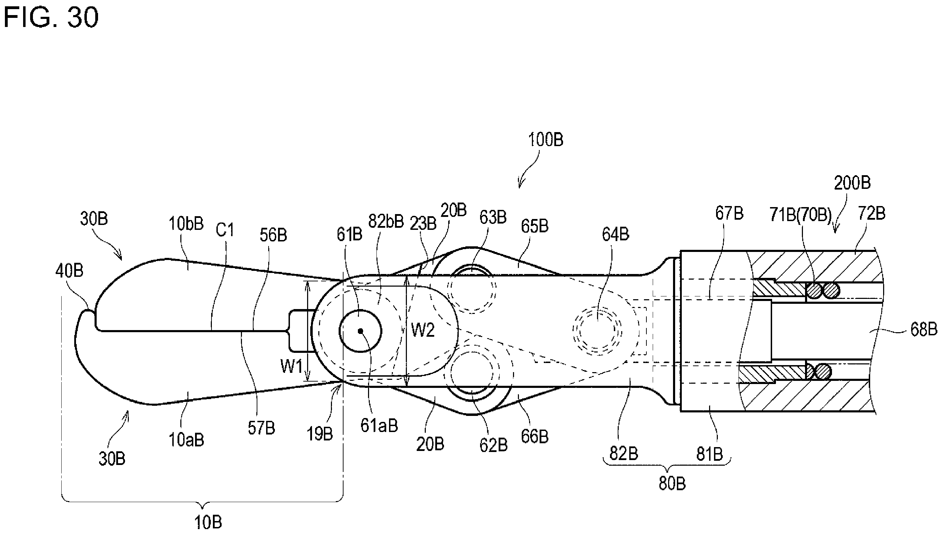

As illustrated in any of Figs. 24 to 27, a high frequency treatment device distal treatment instrument 100B according to the present embodiment is disposed in the distal portion of a medical high frequency treatment device 200B (hereinafter, simply referred to as a high frequency treatment device 200B in some cases), and is a high frequency treatment device distal treatment instrument 100B used by being inserted into a forceps hole (not illustrated) of an endoscope 300B (Fig. 30 illustrates a distal portion of a hood 310B in the distal portion of the endoscope 300B) so as to incise a biological tissue (not illustrated).

A biopsy forceps having a pair of cup-shaped opening and closing portions is excluded as the high frequency treatment device distal treatment instrument 100B according to the present embodiment.

The high frequency treatment device distal treatment instrument 100B includes the distal treatment unit 10B configured to have the pair of opening and closing portions 10aB and 10bB. Line-shaped (linear) electrodes 52B are respectively formed in the pair of opening and closing portions 10aB and 10bB. The line-shaped electrode 52B is not an annular or U-shaped electrode as viewed in the biopsy forceps having the pair of cup-shaped opening and closing portions. The electrode 52B may have a height difference in each portion, or may meander.

The pair of opening and closing portions 10aB and 10bB is axially supported by a common rotary shaft (shaft member 61B illustrated in Figs. 25 and 26), is capable of opening and closing each other, and performs high frequency excision by shearing or pinching the biological tissue.

The pair of opening and closing portions 10aB and 10bB has a plate shape or a rod shape, and does not have a cup shape.

In a state where the pair of opening and closing portions 10aB and 10bB is closed, a shape of the distal side portion of the distal treatment unit 10B when viewed in the axial direction (direction perpendicular to the paper surface in Fig. 25) of the rotary shaft is a shape which is narrowed after being widened from the distal end toward the proximal end. The term "widened or narrowed" as used herein means widened or narrowed in an opening direction (upward-downward direction in Fig. 25) of the pair of opening and closing portions 10aB and 10bB.

In other words, in a state where the pair of opening and closing portions 10aB and 10bB is closed, a shape of the distal side portion of the distal treatment unit 10B when viewed in the axial direction (direction perpendicular to the paper surface in Fig. 25) of the rotary shaft is a shape which is narrowed after being widened from the proximal end toward the distal end.

The distal treatment unit 10 is a portion located on the distal side of the rotary shaft (shaft member 61B) in the pair of opening and closing portions 10aB and 10bB. In a case of the present embodiment, in the pair of opening and closing portions 10aB and 10bB, a portion projecting from a bracket 82B of a holding frame 80B toward the distal side is the distal treatment unit 10B.

In addition, in the medical high frequency treatment device 200B according to the present embodiment, the distal portion has the high frequency treatment device distal treatment instrument 100B according to the present embodiment, and the proximal side has a hand operation unit (operation unit) 90B for performing an opening and closing operation on the pair of opening and closing portions 10aB and 10bB.

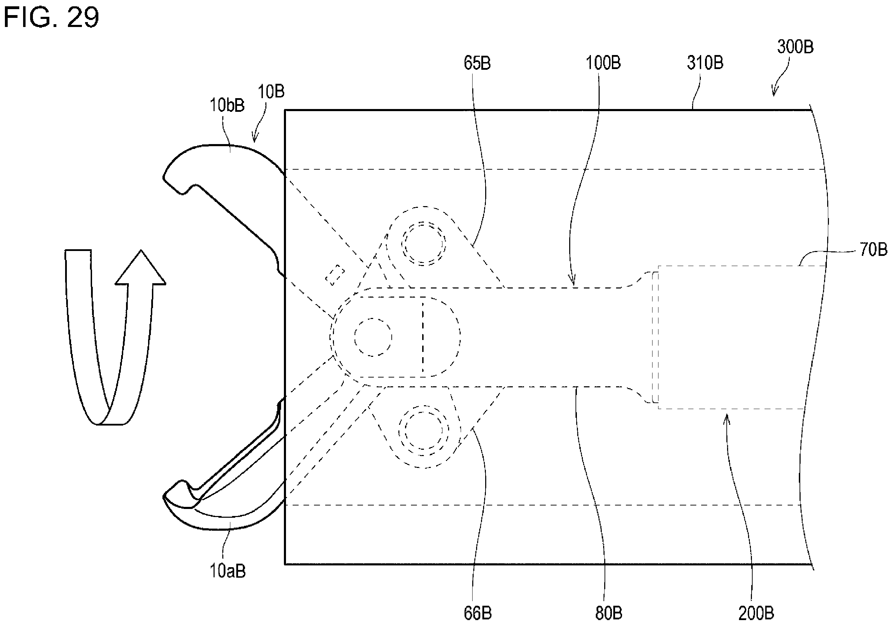

According to the present embodiment, in a state where the pair of opening and closing portions 10aB and 10bB is closed, the shape of the distal side portion of the distal treatment unit 10B when viewed in the axial direction of the rotary shaft is the shape which is narrowed after being widened from the distal end toward the proximal end. In this manner, as illustrated in Fig. 29, when the distal treatment unit 10B is projected from the distal end of the endoscope 300B so as to perform the treatment by opening the pair of opening and closing portions 10aB and 10bB, it is possible to suppress interference of the pair of opening and closing portions 10aB and 10bB with the distal end (for example, the distal end of the hood 310B) of the endoscope 300B.

In addition, the distal treatment unit 10B substantially comes into point contact with a peripheral wall of a forceps hole (not illustrated) of the endoscope 300B. Accordingly, an operation of moving the high frequency treatment device distal treatment instrument 100B forward into the forceps hole can be smoothly performed.

In addition, the pair of opening and closing portions 10aB and 10bB is rotationally operated in an open state. In this case, it is possible to suppress the interference between the pair of opening and closing portions 10aB and 10bB and the biological tissue located behind the pair of opening and closing portions 10aB and 10bB.

In a case of the present embodiment, the medical high frequency treatment device 200B is a pair of scissors forceps, and each of the pair of opening and closing portions 10aB and 10bB is thin plate-shaped shearing scissors. The axial direction of the shaft member 61B axially supporting the pair of opening and closing portions 10aB and 10bB extends in the thickness direction of the pair of opening and closing portions 10aB and 10bB.

Each of the pair of opening and closing portions 10aB and 10bB has a blade portion 50B for shearing a biological tissue.

More specifically, each of the pair of opening and closing portions 10aB and 10bB has a proximal piece 20B formed on each proximal side of the pair of opening and closing portions 10aB and 10bB and axially supported by the rotary shaft, a distal claw portion 40B formed in each distal end of the pair of opening and closing portions 10aB and 10bB, and the blade portion 50B formed between the distal claw portion 40B and the proximal piece 20B in each of the pair of opening and closing portions 10aB and 10bB. The line-shaped electrode 52B is formed in the blade portion 50B.

As illustrated in Fig. 24, the high frequency treatment device 200B includes an elongated operation wire 68B, the high frequency treatment device distal treatment instrument 100B disposed in the distal end of the operation wire 68B, a flexible sheath 70B that accommodates the operation wire 68B, and a hand operation unit 90B which is disposed on the proximal side of the sheath 70B and to which the proximal end of the operation wire 68B is connected.

The sheath 70B is an elongated and tubular member that accommodates the operation wire 68B. In a case of the present embodiment, the sheath 70B is a metal coil 71B (Figs. 25 and 26) manufactured by tightly winding a conductive wire such as a stainless wire. An insulating film 72B is tightly disposed on the outer surface of the sheath 70B. However, as the sheath 70B, an insulating tubular member (tube) may be used instead of the metal coil 71B.

The hand operation unit 90B illustrated in Fig. 24 includes a shaft portion 95B into which the operation wire 68B is inserted, a finger ring 92B disposed in the proximal portion of the shaft portion 95B, and a slider 93B to which the proximal end of the operation wire 68B is connected and which moves forward and rearward with respect to the shaft portion 95B. The operation wire 68B is slidably inserted into the shaft portion 95B. For example, a user inserts a thumb into the finger ring 92B, and pinches the slider 93B with other two fingers, thereby driving the slider 93B to move forward and rearward along the longitudinal direction of the shaft portion 95B. In this manner, the operation wire 68B moves forward or rearward with respect to the hand operation unit 90B. The proximal end of the sheath 70B is fixed to the hand operation unit 90B, and the operation wire 68B is inserted into the sheath 70B to be movable forward and rearward. Accordingly, the distal end of the operation wire 68 moves forward or rearward with respect to the sheath 70 in conjunction with the forward and rearward movement of the slider 93B. In this manner, as will be described later, a forward and rearward movement portion 67B (Figs. 25 and 26) of the high frequency treatment device distal treatment instrument 100B is driven to move forward and rearward, and the pair of opening and closing portions 10aB and 10bB is opened and closed.

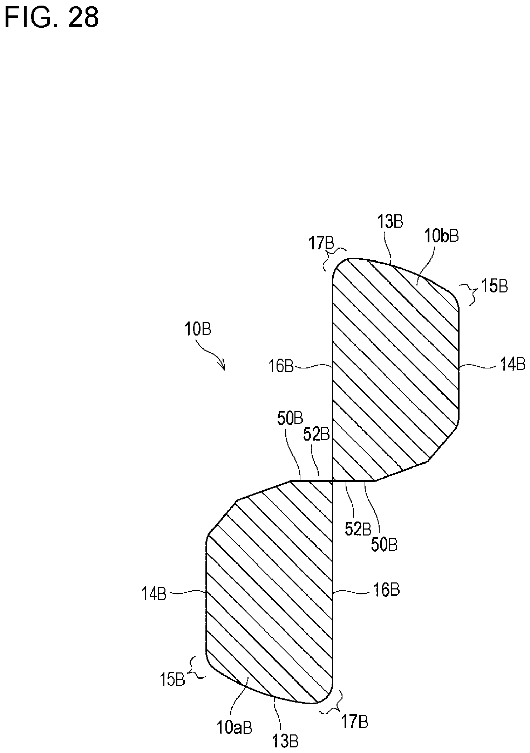

The axial direction of the rotary shaft of the pair of opening and closing portions 10aB and 10bB is a direction perpendicular to the plate surfaces of the pair of opening and closing portions 10aB and 10bB. When the pair of opening and closing portions 10aB and 10bB is opened and closed, portions of mutual facing surfaces 16 (b) (refer to Fig. 28) of the pair of opening and closing portions 10aB and 10bB slide on each other.

As illustrated in Fig. 24, the hand operation unit 90B includes a power supply unit 91B. The power supply unit 91B is a terminal for applying a high frequency current to the pair of opening and closing portions 10aB and 10bB, and a high frequency power source (not illustrated) is connected thereto through a power cable. The pair of opening and closing portions 10aB and 10bB, the link pieces 65B and 66B, and the forward and rearward movement portion 67B, which configure the high frequency treatment device distal treatment instrument 100B, are all manufactured using a conductive metal material. In addition, the operation wire 68B is also manufactured using the conductive metal material. Therefore, the high frequency current input to the power supply unit 91B is applied to the pair of opening and closing portions 10aB and 10bB.

As illustrated in Figs. 25 and 26, the high frequency treatment device distal treatment instrument 100B includes the pair of plate-shaped opening and closing portions 10aB and 10bB, the shaft member 61B that axially supports the pair of opening and closing portions 10aB and 10bB so as to be openable and closeable, the two link pieces 65B, 66B, the forward and rearward movement portion 67B, and the holding frame 80B.

The pair of opening and closing portions 10aB and 10bB is driven to open and close by pushing and pulling the operation wire 68B. The operation wire 68B is manufactured using a conductive metal material such as stainless steel.

The forward and rearward movement portion 67B is integrally connected to the distal end of the operation wire 68B. The two link pieces 65B and 66B are pivotally connected to the forward and rearward movement portion 67B by a shaft member 64B. Furthermore, the proximal piece 20B of one opening and closing portion 10aB is pivotally connected to the link piece 65B by a shaft member 63B, and the proximal piece 20B of the other opening and closing portion 10bB is pivotally connected to the link piece 66B by a shaft member 62B.

The axial direction of the respective shaft members 62B, 63B, and 64B is a direction parallel to the axial direction of the shaft member 61B.

The pair of opening and closing portions 10aB and 10bB and the link pieces 65B and 66B relatively pivot in a plane illustrated in Figs. 25 and 26 (in a plane perpendicular to the axial direction of the shaft member 61B).

The proximal piece 20B of the pair of opening and closing portions 10aB and 10bB and the link pieces 65B and 66B configure a four-joint link having a rhomboid shape.

The shaft members 62B and 63B are located on the distal side of the shaft member 64B, and the shaft member 61B is located on the distal side of the shaft members 62B and 63B.

The holding frame 80B is fixed to the distal end of the sheath 70B.

The holding frame 80B includes a proximal portion 81B fixed to the distal end of the sheath 70B, and a pair of brackets 82B projecting to the distal side from the proximal portion 81B.

For example, the bracket 82B is formed in a plate shape.

The pair of opening and closing portions 10aB and 10bB is axially supported by the shaft member 61B with respect to the distal portion of the pair of brackets 82B.

In a gap between the pair of brackets 82B, the proximal pieces 20B of the pair of opening and closing portions 10aB and 10bB and the link pieces 65 and 66 are respectively rotatable. A portion projecting to the distal side from the sheath 70B in the forward and rearward movement portion 67 is movable forward and rearward.

As described above, the high frequency treatment device distal treatment instrument 100B includes the pair of brackets 82B that pinches the proximal portion of the pair of opening and closing portions 10aB and 10bB from both sides in the axial direction of the rotary shaft (shaft member 61B), and that axially supports the proximal portion in the rotary shaft.

As illustrated in Fig. 25, when the operation wire 68B and the forward and rearward movement portion 67B are pulled toward the proximal side (rightward in Fig. 25), the pair of opening and closing portions 10aB and 10bB is in a closed state. Conversely, as illustrated in Fig. 26, when the operation wire 68B and the forward and rearward movement portion 67B are pushed to the distal side (leftward in Fig. 26), the pair of opening and closing portions 10aB and 10bB is in an open state.

Each of the pair of opening and closing portions 10aB and 10bB has a substantially L-shape (that is, a sickle shape) that is shallowly bent in a pivot plane in the vicinity of the shaft member 61B. The proximal piece 20B is a proximal side portion of the shaft member 61B in each of the pair of opening and closing portions 10aB and 10bB. On the other hand, in each of the pair of opening and closing portions 10aB and 10bB, a distal side portion of the shaft member 61B will be referred to as a distal piece 30B.

The shapes of the pair of opening and closing portions 10aB and 10bB may be the same as each other, or may be different from each other. In a case of the present embodiment, the pair of opening and closing portions 10aB and 10bB have mutually the same shape.

The distal claw portion 40B is formed in the distal end of each distal piece 30B of the opening and closing portions 10aB and 10bB. The distal claw portion 40B projects in a closing direction. The closing direction is a direction in which the pair of opening and closing portions 10aB and 10bB is closed, that is, a direction from one opening and closing portion 10aB to the other opening and closing portion 10bB, and a direction from the other opening and closing portion 10b to one opening and closing portion 10a. In addition, a direction in which the pair of opening and closing portions 10aB and 10bB is opened will be referred to as an opening direction.

The distal claw portion 40B is a projection that is bitten into the biological tissue.

The blade portion 50B is formed along an end edge (edge) on a side in the closing direction of the proximal side portion of the distal claw portion 40B in the distal piece 30B.

The biological tissue can be incised by the blade portion 50B of the pair of opening and closing portion 10aB and 10bB in a state where the biological tissue is picked and held by the distal claw portion 40B of the pair of opening and closing portion 10aB and 10bB so as to prevent the falling of the biological tissue.

An insulating film 12B (Figs. 25 and 26) is formed on each surface of the pair of opening and closing portions 10aB and 10bB. The insulating film 12B is formed on the entire surface of the distal piece 30B except for at least the formation region of the electrode 52B.

The insulating film 12B can be formed by coating the surface of the opening and closing portions 10aB and 10bB with an insulating material such as a fluororesin.

With regard to the film thickness of the insulating film 12B, the film thickness in the widest portion in the distal treatment unit 10B may be locally thicker than the film thickness in other portions. In this manner, even when the distal treatment unit 10B moves forward and rearward by substantially coming into point contact with the peripheral wall of the forceps hole (not illustrated) of the endoscope 300B, it is possible to suppress detachment of the insulating film 12B in the widest portion of the distal treatment unit 10B, and it is possible to suppress an exposure of a metal substrate in the widest portion.

The electrode 52B is a linear portion exposed from the insulating film 12B in the blade portion 50B. The pair of opening and closing portions 10aB and 10bB serves as a monopolar high frequency electrode when a high frequency voltage in the same phase is applied thereto from the power supply unit 91B. The high frequency current is applied to the pair of opening and closing portions 10aB and 10bB in a state where the biological tissue is gripped by the pair of opening and closing portions 10aB and 10bB. In this manner, the biological tissue is cauterized and incised. Instead of the present embodiment, a bipolar high frequency treatment device 200B may be used in which one of the pair of opening and closing portions 10aB and 10bB is used as an active electrode and the other is used as a return electrode.

The shape of the blade portion 50B is not particularly limited. In a case of the present embodiment, the blade portion 50B has a high step portion 54B and a low step portion 55B having a notch shape recessed toward a side in the opening direction from the high step portion 54B.

For example, the electrode 52B is continuously formed over a proximal side edge side in the distal claw portion 40B, the low step portion 55B, and electrode proximal positions 52aB located in proximal portion of the high step portion 54B.

In a case of the present embodiment, the "distal side portion of the distal treatment unit 10B", that is, the "portion in which the shape when viewed in the axial direction of the rotary shaft in a state where the pair of opening and closing portions 10aB and 10bB is closed is narrowed after being widened from the distal end toward the proximal end" is the distal side portion of the proximal end (electrode proximal position 52aB) of the formation region of the electrode 52B.

More specifically, in a case of the present embodiment, the "distal side portion of the distal treatment unit 10B" is a distal side portion of an intermediate position C1 (Fig. 25) in the distal-proximal direction of the distal treatment unit 10B. That is, the distal side portion obtained by dividing the distal treatment unit 10B into two in the distal-proximal direction in a state where the pair of opening and closing portions 10aB and 10bB is closed has the shape which is narrowed after being widened from the distal end toward the proximal end.

More specifically, the "distal side portion of the distal treatment unit 10B" has the shape which is gradually narrowed after being gradually widened from the distal end toward the proximal end (in both the upward direction and the downward direction in Fig. 25).

More specifically, the whole distal treatment unit 10B has the shape which is gradually narrowed after being gradually widened from the distal end toward the proximal end (in both the upward direction and the downward direction in Fig. 25). That is, in the distal treatment unit 10B, the proximal side portion of the widest portion in the upward-downward direction in Fig. 25 is gradually and monotonously narrowed.

A stopper portion 11B is formed in at least one of the pair of opening and closing portions 10aB and 10bB. A closing operation of the pair of opening and closing portions 10aB and 10bB is restricted by the stopper portion 11B coming into contact with the blade portion 50B of the other opening and closing portion (opening and closing portion 10aB or opening and closing portion 10bB).

In a case of the present embodiment, the stopper portion 11B is formed in each of the pair of opening and closing portions 10aB and 10bB. The stopper portion 11B of the opening and closing portion 10aB comes into contact with the blade portion 50B of the opening and closing portion 10bB, and the stopper portion 11B of the opening and closing portion 10bB comes into contact with the blade portion 50B of the opening and closing portion 10aB, thereby restricting the closing operation of the pair of opening and closing portions 10aB and 10bB.

In the stopper portion 11B, a portion facing the other opening and closing portion (opening and closing portion 10aB or 10bB) is a flat surface 11aB.

As illustrated in Figs. 25 to 27, a step portion 23B is formed on the outer surface of the proximal piece 20B. In the proximal piece 20B, a proximal side portion of the step portion 23B is thinner than a distal side portion of the step portion 23B.

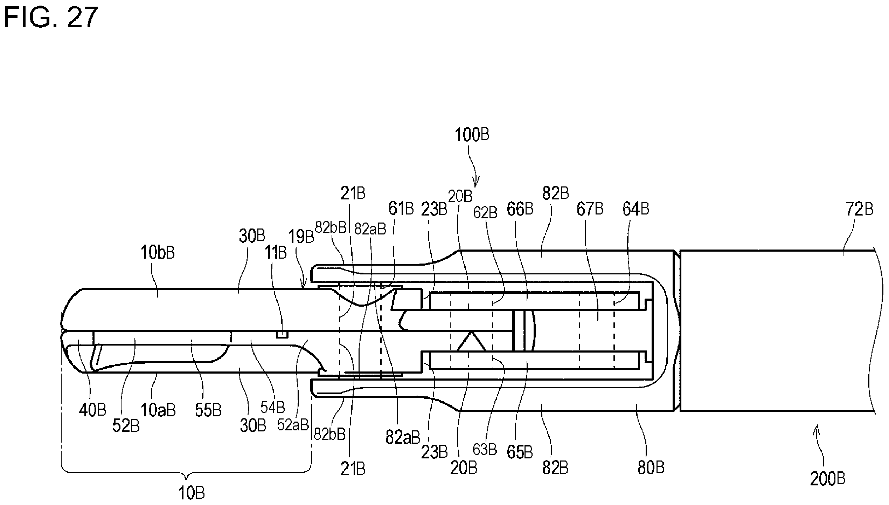

As illustrated in Fig. 27, in the proximal piece 20B, a thickness difference between the proximal side portion and the distal side portion of the step portion 23B is set to be slightly larger than the thickness of the link pieces 65B and 66B, or is set to be equal to the thickness of the link pieces 65B and 66B.

As illustrated in Fig. 25, in a state where the pair of opening and closing portions 10aB and 10bB is closed, the dimension of the proximal portion 19(b) of the distal treatment unit 10B is smaller than the dimension of the bracket 82B in a direction (that is, the upward-downward direction in Fig. 25) perpendicular to both the distal-proximal direction and the axial direction. That is, a dimension W1 illustrated in Fig. 25 is smaller than a dimension W2.

According to this configuration, when the distal treatment unit 10B is projected from the distal end of the endoscope 300B and the treatment is performed by opening the pair of opening and closing portions 10aB and 10bB, it is possible to further suppress the interference between the pair of opening and closing portions 10aB and 10bB and the distal end of the endoscope 300B.

Similarly, in a case where the pair of opening and closing portions 10aB and 10bB is rotationally operated in an open state, it is possible to further suppress the interference between the pair of opening and closing portions 10aB and 10bB and the biological tissue located behind the pair of opening and closing portions 10aB and 10bB.

As illustrated in Fig. 27, the pair of brackets 82BB of the holding frame 80B pinches the proximal piece 20B of the pair of opening and closing portions 10aB and 10bB from both sides in the axial direction of the rotary shaft (shaft member 61B) of the pair of opening and closing portions 10aB and 10b, and axially supports the proximal piece 20B in the rotary shaft. The pair of brackets 82B pinches and axially supports the proximal piece 20B in the distal portion of the bracket 82B.

An outer surface 82bB that is a rear surface with respect to each facing surface 82aB of the pair of brackets 82B is formed to be flat in each distal portion of the pair of brackets 82B. Moreover, a distance between the outer surfaces 82bB of the respective distal portions of the pair of brackets 82B is shorter than a distance between the outer surfaces of the proximal portions of the brackets 82B. That is, the distal portion of the bracket 82B has a shape a flat shape so that the outer surface side is cut.

Therefore, it is possible to suppress the interference between the bracket 82B and the biological tissue or the peripheral wall of the forceps hole. In addition, an advantageous effect can be achieved in that coating the bracket 82B is facilitated.

Here, as illustrated in Fig. 28, a boundary portion 15B between an edge portion 13B on a side in the opening direction of the pair of opening and closing portions 10aB and 10bB and an outer surface 14B has a chamfered shape.

In this manner, the distal treatment unit 10B can more smoothly slide on the peripheral wall of the forceps hole (not illustrated) of the endoscope 300B. Accordingly, it is possible to smoothly perform an operation of moving the high frequency treatment device distal treatment instrument 100B forward into the forceps hole.

It is preferable that the boundary portion 15B has an R-chamfered shape.

In addition, a boundary portion 17B between the edge portion 13B on the side in the opening direction of the pair of opening and closing portions 10aB and 10bB and the facing surface 16B also has the chamfered shape.

In this manner, the distal treatment unit 10B can more smoothly slide on the peripheral wall of the forceps hole (not illustrated) of the endoscope 300B. Accordingly, it is possible to smoothly perform an operation of moving the high frequency treatment device distal treatment instrument 100B forward into the forceps hole.

It is preferable that the boundary portion 17B also has the R-chamfered shape.

According to the seventh embodiment as described above, based on the virtual straight line L1, the height of the highest position in the formation region of the electrode 52B (electrode formation region 53B) in the blade portion 50B is lower than the height of the distal claw portion 40B. Accordingly, when the pair of opening and closing portions 10aB and 10bB is closed, an operation of the blade portion 50B for pushing back the biological tissue is suppressed. Therefore, when the pair of opening and closing portions 10aB and 10bB is closed, the distal claw portion 40B can be quickly bitten into the biological tissue, and the biological tissue can be more reliably gripped by the pair of opening and closing portions 10aB and 10bB.

### [Eighth Embodiment]

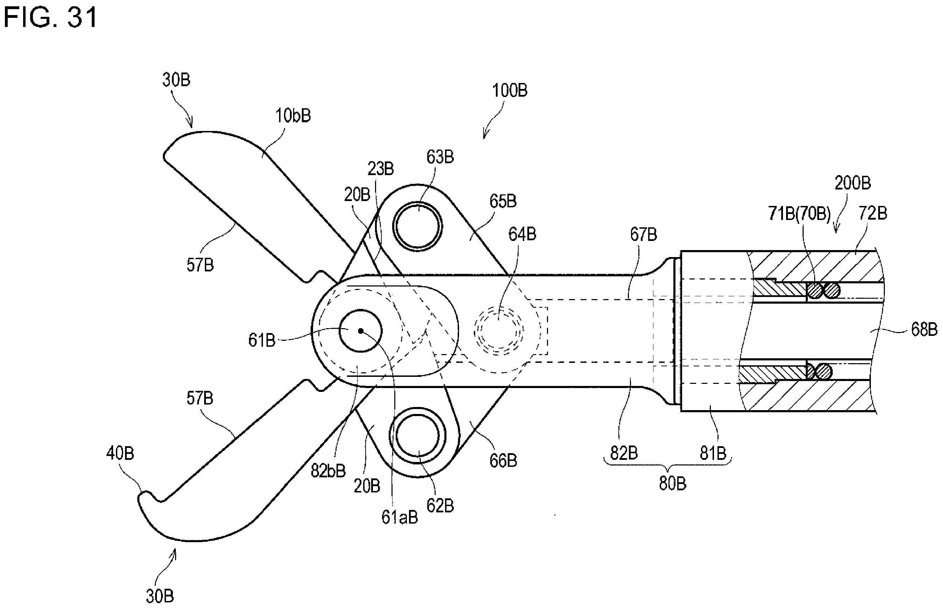

Next, an eighth embodiment will be described with reference to Figs. 30 to 32.

The high frequency treatment device 200B and the high frequency treatment device distal treatment instrument 100B according to the present embodiment are different from the high frequency treatment device 200B and the high frequency treatment device distal treatment instrument 100B according to the seventh embodiment in the following points. Other points are configured to be the same as those of the high frequency treatment device 200B and the high frequency treatment device distal treatment instrument 100B according to the above-described seventh embodiment.

In the above-described seventh embodiment, an example has been described in which each of the pair of opening and closing portions 10aB and 10bB is thin plate-shaped shearing scissors. However, in a case of the present embodiment, each of the pair of opening and closing portions 10aB and 10bB is formed in a rod shape, and includes facing surfaces 56 (b) and 57B that face each other.

In a state where the pair of opening and closing portions 10aB and 10bB is closed, the facing surface 56(b) and the facing surface 57B overlap each other in the opening and closing direction of the pair of opening and closing portions 10aB and 10bB.

The pair of opening and closing portions 10aB and 10bB is configured to pinch the biological tissue by using the facing surfaces 56(b) and 57B.

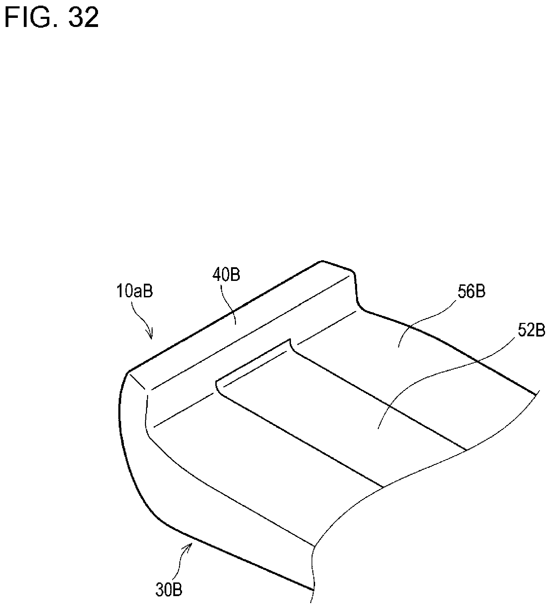

As illustrated in Fig. 32, for example, an electrode 52B extending in the distal-proximal direction is formed in a central portion in the width direction of the opening and closing portion 10aB. Similarly, although not illustrated, the electrode 52B extending in the distal-proximal direction is formed in a central portion in the width direction of the opening and closing portion 10bB.

For example, out of the opening and closing portion 10aB and the opening and closing portion 10bB, the distal claw portion 40B is formed in the distal portion of the opening and closing portion 10aB, and the distal claw portion 40B is not formed in the distal portion of the opening and closing portion 10bB.

The opening and closing portion 10bB does not have the distal claw portion 40B, and is formed to be shorter than the opening and closing portion 10aB correspondingly. As illustrated in Fig. 30, in a state where the pair of opening and closing portions 10aB and 10bB is closed, the proximal side surface in the distal claw portion 40B of the opening and closing portion 10aB comes into contact with or moves close to the distal surface of the opening and closing portion 10bB.

Even in a case of the present embodiment, in a state where the pair of opening and closing portions 10aB and 10bB is closed, the shape of the distal side portion of the distal treatment unit 10B when viewed in the axial direction of the rotary shaft (direction perpendicular to the paper surface in Fig. 30) is the shape which is narrowed after being widened from the distal end to the proximal end.

In addition, even in a case of the present embodiment, the distal side portion of the distal treatment unit 10B is the distal side portion from the proximal end of the formation region of the electrode 52B.

In addition, even in a case of the present embodiment, the distal side portion of the distal treatment unit 10B is the distal side portion of the intermediate position in the distal-proximal direction of the distal treatment unit 10B.

According to the present embodiment, the same advantageous effect as that of the seventh embodiment can be achieved.

Hitherto, the seventh and eighth embodiments have been described with reference to the drawings. However, the embodiments are examples of the fourth or fifth aspect of the present invention, and various configurations other than the above-described examples can be adopted.

In addition, the seventh or eighth embodiment can be appropriately combined within the scope not departing from the gist of the present invention.

At least one embodiment of the fourth and fifth aspects includes the following technical concept.
(1) There is provided a high frequency treatment device distal treatment instrument disposed in a distal portion of a medical high frequency treatment device and used by being inserted into a forceps hole of an endoscope so as to incise a biological tissue.
   The high frequency treatment device distal treatment instrument includes a distal treatment unit having a pair of opening and closing portions each having a line-shaped electrode, axially supported by a common rotary shaft, capable of opening and closing each other, performing high frequency excision by shearing or pinching the biological tissue.
   In a state where the pair of opening and closing portions is closed, a shape of a distal side portion of the distal treatment unit when viewed in an axial direction of the rotary shaft is a shape which is narrowed after being widened from a distal end toward a proximal end.
(2) In the high frequency treatment device distal treatment instrument according to (1), the distal side portion of the distal treatment unit is a distal side portion from a proximal end of a formation region of the electrode.
(3) The high frequency treatment device distal treatment instrument according to (1) or (2), the distal side portion of the distal treatment unit is a distal side portion of an intermediate position of the distal treatment unit in a distal-proximal direction.
(4) The high frequency treatment device distal treatment instrument according to any one of (1) to (3) includes a pair of brackets that pinches the proximal portion of the pair of opening and closing portions from both sides in the axial direction of the rotary shaft, and that axially supports the proximal portion in the rotary shaft.
   In a state where the pair of opening and closing portions is closed, a dimension of the proximal portion of the distal treatment unit is smaller than a dimension of the bracket in a direction perpendicular to both the distal-proximal direction and the axial direction.
(5) In the high frequency treatment device distal treatment instrument according to any one of (1) to (4), a boundary portion between an edge portion on a side in an opening direction of the pair of opening and closing portions and an outer surface has a chamfered shape.
(6) The high frequency treatment device distal treatment instrument according to any one of (1) to (5) includes a pair of brackets that pinches the proximal portion of the pair of opening and closing portions from both sides in the axial direction of the rotary shaft, and that axially supports the proximal portion in the rotary shaft.
   The pair of brackets pinches and axially supports the pair of opening and closing portions in the distal portion of the brackets.
   In each distal portion of the pair of brackets, an outer surface that is a rear surface with respect to each facing surface of the pair of brackets is formed to be flat.
   A distance between the outer surfaces of the respective distal portions of the pair of brackets is shorter than a distance between the outer surfaces of the proximal portions of the brackets.
(7) There is provided a medical high frequency treatment device, a distal portion of which has a high frequency treatment device distal treatment instrument according to any one of (1) to (6), and a proximal side of which has an operation unit for performing an opening and closing operation on a pair of opening and closing portions.

### [Ninth Embodiment]

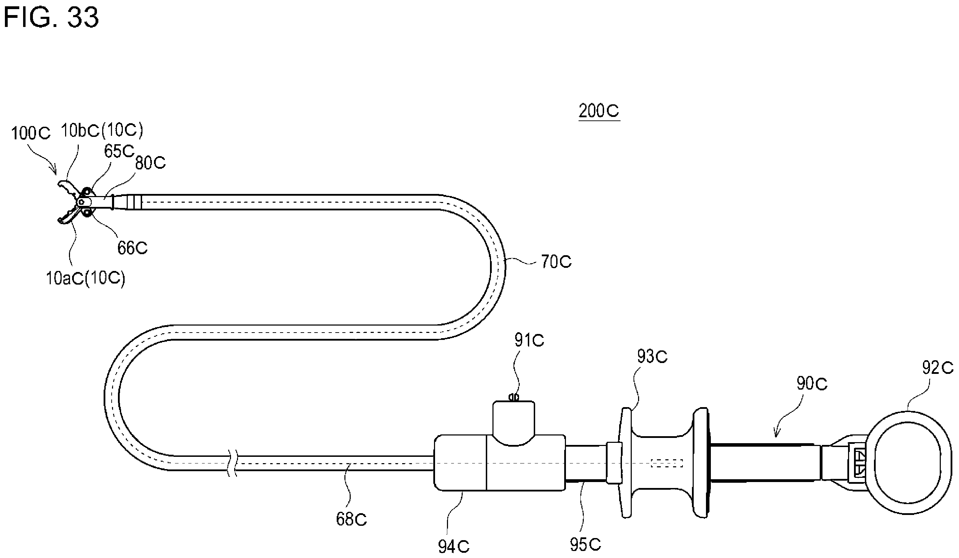

First, a ninth embodiment will be described with reference to Figs. 33 to 40.

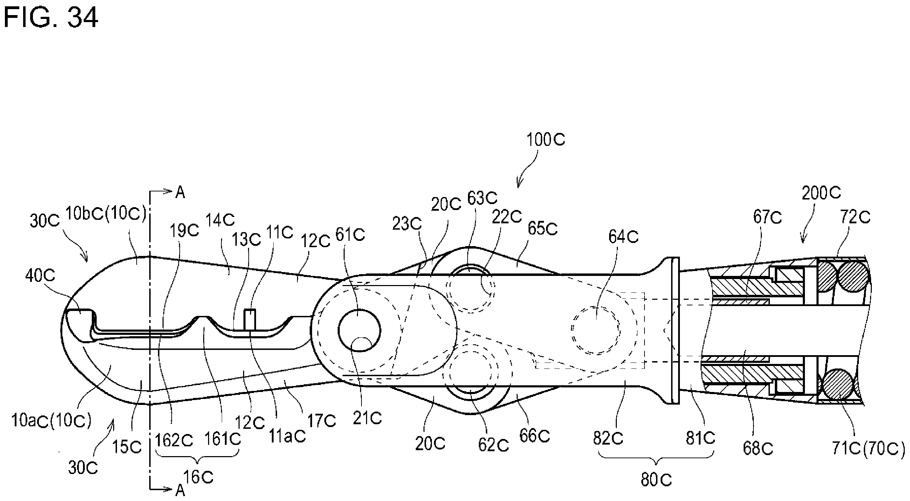

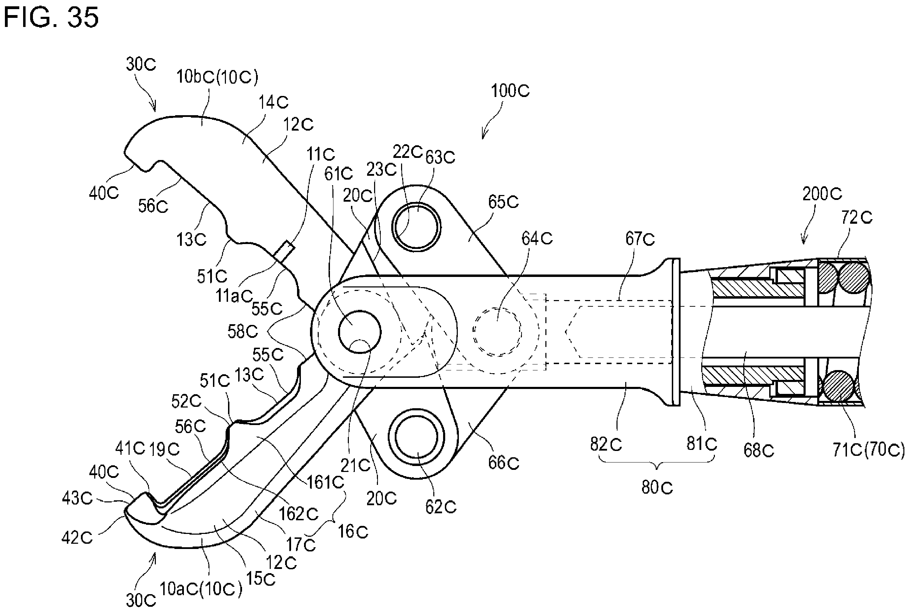

In side views illustrated in Figs. 34 and 35, a proximal side portion in an illustrated range in a sheath 70C indicates a side cross section taken along a center line.

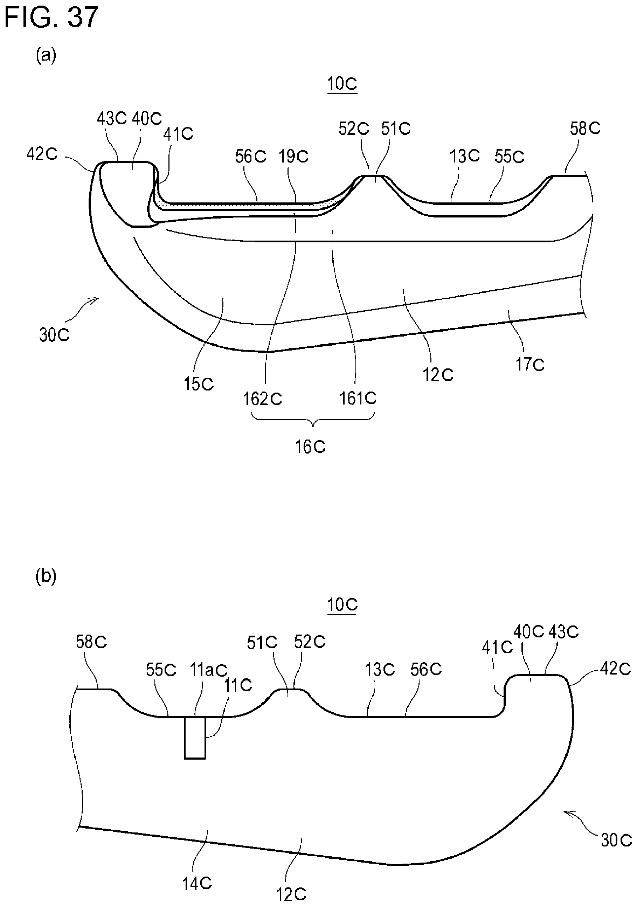

In each of Figs. 37(a), 38(a), 38(b), 39(a), and 39(b), a formation region of an electrode (electrode region 19) in scissors 10C is hatched in a dot shape. In each of Figs. 37(a), 38(a), 38(b), 39(a), and 39(b), a region which is not hatched in the dot shape is a formation region of an insulating film 12C (non-conductive layer) . However, the insulating film 12C may not be formed inside a first shaft support hole 21C and inside a second shaft support hole 22C.

As illustrated in Fig. 33, the high frequency treatment device 200C according to the present embodiment is a medical high frequency treatment device 200C. The high frequency treatment device 200C includes a high frequency treatment device knife 100C having the pair of scissors 10C so as to incise a biological tissue in a distal portion of the high frequency treatment device 200C.

The high frequency treatment device 200C is used by inserting the high frequency treatment device knife 100C of the high frequency treatment device 200C into a forceps hole of an endoscope (not illustrated).

One of the pair of scissors 10C will be referred to as scissors 10aC, and the other will be referred to as scissors 10bC.

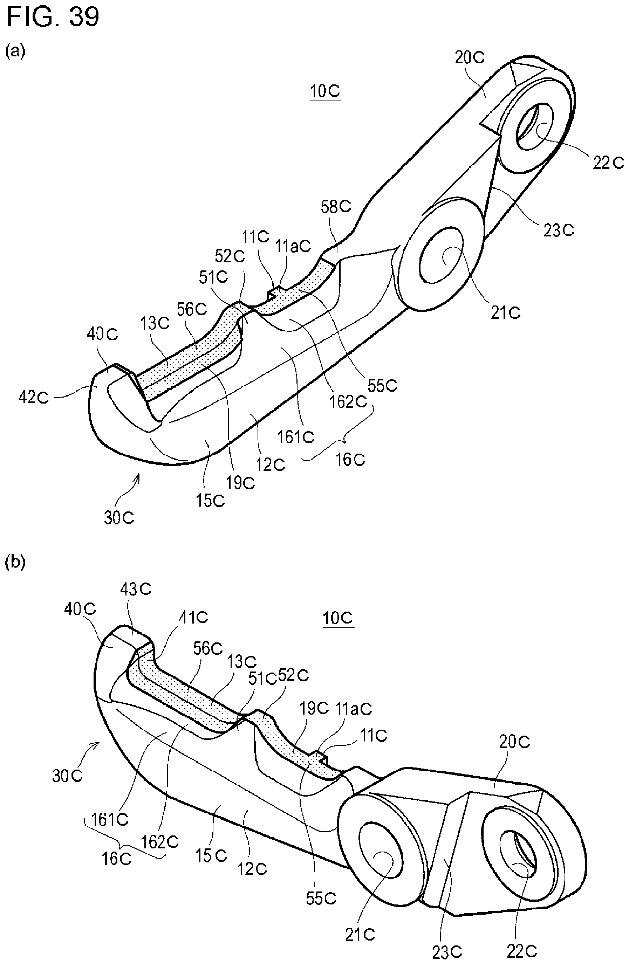

Each of the pair of scissors 10C is formed in an elongated plate shape (refer to Figs. 39(a) and 39(b)).

As illustrated in Figs. 34 to 36, proximal portions of the pair of scissors 10C are axially supported by each other in a pivot shaft (shaft member 61C) intersecting a plate surface direction of the scissors 10C.

The pair of scissors 10C is configured to be capable of shearing the biological tissue by pivoting in a direction closer to each other.

Each of the pair of scissors 10C has a blade surface 13C, a sliding contact surfaces 14C that comes into sliding contact with each other, an outer surface 15C that is a rear surface with respect to the sliding contact surface 14C, an inclined surface 16C located between the outer surface 15C and the blade surface 13C, and a rear surface 17C that is a surface opposite to the blade surface 13C.

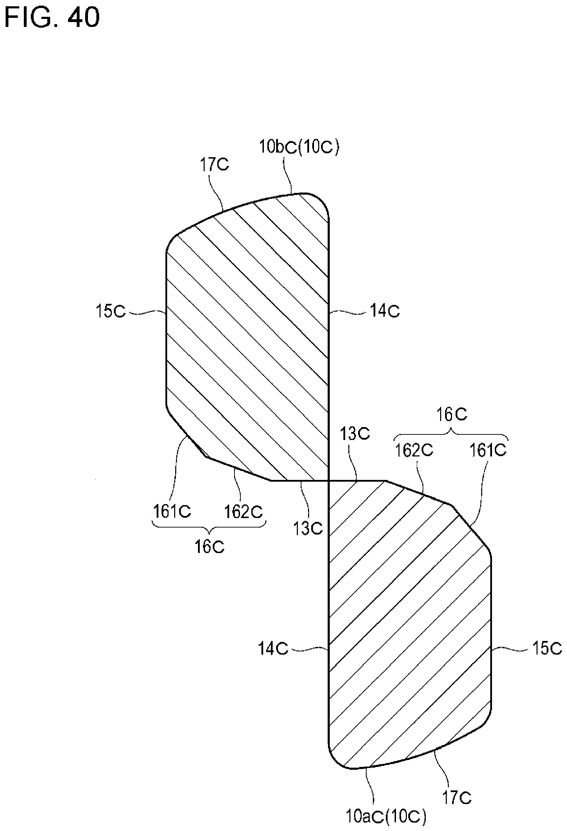

The inclined surface 16C is inclined from the sliding contact surface 14C side toward the outer surface 15C side in a direction away from the other scissors 10C. That is, as illustrated in Fig. 40, the inclined surface 16C of the scissors 10aC is inclined downward from the sliding contact surface 14C side (left side in Fig. 40) of the scissors 10aC toward the outer surface 15C side (right side in Fig. 40). The inclined surface 16C of the scissors 10bC is inclined upward from the sliding contact surface 14C side (right side in Fig. 40) of the scissors 10bC toward the outer surface 15C (left side in Fig. 40).

In other words, the inclined surface 16C is inclined from the blade surface 13C side to the outer surface 15C side in a direction away from the other scissors 10C. That is, as illustrated in Fig. 40, the inclined surface 16C of the scissors 10aC is inclined downward from the blade surface 13C side of the scissors 10aC toward the outer surface 15C side, and the inclined surface 16C of the scissors 10bC is inclined upward from the blade surface 13C side of the scissors 10bC toward the outer surface 15C.

Each surface of the pair of scissors 10C includes a formation region of a non-conductive layer (insulating film 12C) and an electrode region 19C where the non-conductive layer is not formed.

Then, for at least one of the pair of scissors 10C, the electrode region 19C is formed on the blade surface 13C and the inclined surface 16C. In a case of the present embodiment, for each of the pair of scissors 10C, the electrode region 19C is formed on the blade surface 13C and the inclined surface 16C.

According to the high frequency treatment device 200C in the present embodiment, for at least one of the pair of scissors 10C, the electrode region 19C is formed not only on the blade surface 13C but also on the inclined surface 16C. Therefore, when the biological tissue is incised, a current can be applied to the biological tissue from the electrode region 19C of the inclined surface 16C which comes into contact with the biological tissue together with the blade surface 13C. Accordingly, hemostatic capability for the biological tissue is improved.

As illustrated in Figs. 39(a) and 39(b), each of the pair of scissors 10C has a proximal piece 20C which is a proximal side portion in the scissors 10C, and a distal piece 30C which is a distal side portion in the scissors 10C.

The distal portion of the proximal piece 20C has a first shaft support hole 21C that penetrates the proximal piece 20C in the thickness direction. A common shaft member 61C (Figs. 34 to 36) is inserted into the first shaft support hole 21C of the pair of scissors 10C, and the pair of scissors 10C is axially supported therein.

The distal piece 30C is a distal side portion of the first shaft support hole 21C in the scissors 10C.

The proximal portion of the proximal piece 20C has a second shaft support hole 22C that penetrates the proximal piece 20C in the thickness direction.

As illustrated in Fig. 33, the high frequency treatment device 200C includes an elongated operation wire 68C, the high frequency treatment device knife 100C disposed in the distal end of the operation wire 68C, and a flexible sheath 70C that accommodates the operation wire 68C, and a hand operation unit 90C which is disposed on the proximal side of the sheath 70C and to which the proximal end of the operation wire 68C is connected.

The sheath 70C is an elongated and tubular member that accommodates the operation wire 68C. In a case of the present embodiment, the sheath 70C is configured to have a metal coil 71C (Figs. 34 and 35) manufactured by tightly winding a conductive wire such as a stainless steel wire. An insulating film 72C (Figs. 34 and 35) is tightly disposed on the outer surface of the sheath 70C. However, as the sheath 70C, an insulating tubular member (tube) may be used instead of the metal coil 71C.

The hand operation unit 90C is used to perform an opening and closing operation on the pair of scissors 10C, and is located on the proximal side in the high frequency treatment device 200C.

For example, the hand operation unit 90C includes a shaft portion 95C into which the operation wire 68C is inserted, a finger ring 92C disposed in the proximal portion of the shaft portion 95C, a slider 93C to which the proximal end of the operation wire 68C is connected and which moves forward and rearward with respect to the shaft portion 95C, and a rotational operation unit 94C. The operation wire 68C is slidably inserted into the shaft portion 95C. For example, a user inserts a thumb into the finger ring 92C, and pinches the slider 93C with other two fingers, thereby driving the slider 93C to move forward and rearward along the longitudinal direction of the shaft portion 95C. In this manner, the operation wire 68C moves forward or rearward with respect to the hand operation unit 90C. The proximal end of the sheath 70C is fixed to the hand operation unit 90C, and the operation wire 68C is inserted into the sheath 70C to be movable forward and rearward. Accordingly, the distal end of the operation wire 68C moves forward or rearward with respect to the sheath 70C in conjunction with the forward and rearward movement of the slider 93C. In this manner, as described later, a forward and rearward movement portion 67C (Figs. 34 and 35) of the high frequency treatment device knife 100C is driven to move forward and rearward, and the pair of scissors 10C is opened and closed.

The axial direction of the rotary shaft of the pair of scissors 10C is a direction perpendicular to the plate surface of the scissors 10C (thickness direction of scissors 10C). When the pair of scissors 10C is opened and closed, the sliding contact surfaces 14C of the pair of scissors 10C slide on each other.

As illustrated in Fig. 33, the hand operation unit 90C includes a power supply unit 91C. The power supply unit 91C is a terminal for applying a high frequency current to the pair of scissors 10C. A high frequency power source (not illustrated) is connected to the power supply unit 91C through a power cable. The pair of scissors 10C, the link pieces 65C and 66C (to be described later), and the forward and rearward movement portion 67C (to be described later), which configure the high frequency treatment device knife 100C, are all manufactured using a conductive metal material. The operation wire 68C is also manufactured using the conductive metal material. Therefore, the high frequency current input to the power supply unit 91C is applied to the pair of scissors 10C.

The operation wire 68C is connected to the rotational operation unit 94C, and the rotational operation unit 94C is axially rotated around the shaft portion 95C. In this manner, the operation wire 68C whose proximal end is fixed to the slider 93C is rotated inside the sheath 70C. In this manner, the high frequency treatment device knife 100C can be oriented in a desired direction.

The rotational operation unit 94C is rotatably attached to the power supply unit 91C. In a state where a power cable (not illustrated) connecting the power supply unit 91C and a high frequency power source (not illustrated) to each other is hung downward, the rotational operation unit 94C can be rotationally operated around the shaft portion 95C.

Instead of the present embodiment, the slider 93C may be configured to be axially rotatable around the shaft portion 95C so that the slider 93C also has a function of the rotational operation unit 94C. That is, a configuration may be adopted as follows. The slider 93C is driven to move the slider 93C forward and rearward along the longitudinal direction of the shaft portion 95C. In this manner, the operation wire 68C is moved forward and rearward to perform the opening and closing operation on the high frequency treatment device knife 100C. In addition, the slider 93C is rotated around the shaft portion 95C. In this manner, the high frequency treatment device knife 100C is rotated and oriented in the desired direction.

In addition, the rotational operation unit 94C may be configured to be rotatable with respect to the power supply unit 91C by disposing the rotational operation unit 94C in the shaft portion 95C. In this case, the slider 93C may be configured to be axially rotatable around the shaft portion 95C.

As illustrated in Figs. 34 to 36, the high frequency treatment device knife 100C includes the pair of plate-shaped scissors 10C, the shaft member 61C that axially supports the scissors 10C to be openable and closable, the two links pieces 65C and 66C, the forward and rearward movement portion 67C, and a holding frame 80C.

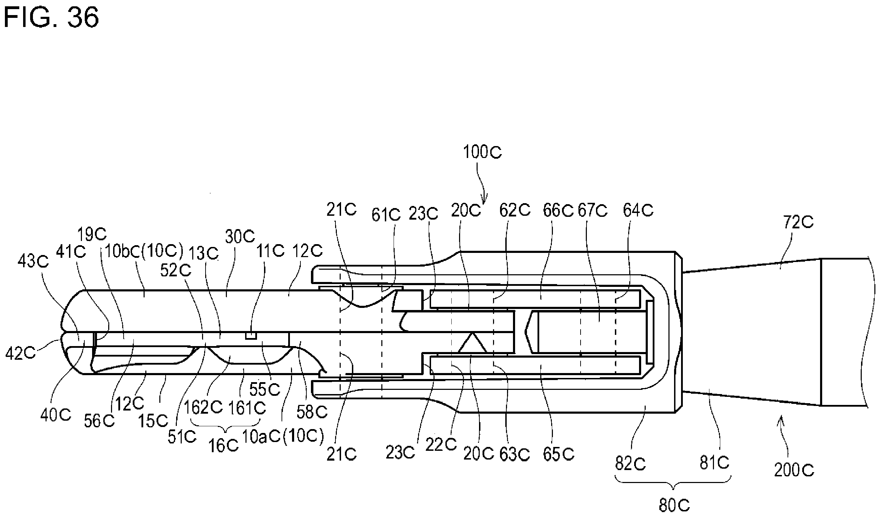

The axial direction of the shaft member 61C is a direction perpendicular to the paper surface in Figs. 34 and 35, and is the upward-downward direction in Fig. 36. The axial direction of the shaft member 61C is a direction in which the pair of scissors 10C overlaps with each other, in other words, the thickness direction of the pair of scissors 10C.

The pair of scissors 10C is driven to be opened and closed by pushing and pulling the operation wire 68C. The operation wire 68C is manufactured using a conductive metal material such as stainless steel.

The forward and rearward movement portion 67C is connected to the distal end of the operation wire 68C integrally with the operation wire 68C. The proximal portions of the two link pieces 65C and 66C are pivotally connected to the forward and rearward movement portion 67C by a shaft member 64C. Furthermore, the proximal piece 20C of one scissors 10C (scissors 10aC) is pivotally connected to the distal portion of the link piece 65C by a shaft member 63C. That is, the shaft member 63C is inserted into the second shaft support hole 22C of the one scissors 10aC and the distal portion of the link piece 65C. In this manner, the scissors 10aC and the link piece 65C are rotatably and axially supported by each other. Similarly, the proximal piece 20C of the other scissors 10C (scissors 10bC) is pivotally connected to the distal portion of the link piece 66C by a shaft member 62C. That is, the shaft member 62C is inserted into the second shaft support hole 22C of the other scissors 10bC and the distal portion of the link piece 66C. In this manner, the scissors 10bC and the link piece 66C are rotatably and axially supported by each other.

The axial direction of the respective shaft members 62C, 63C, and 64C is a direction parallel to the axial direction of the shaft member 61C.

The pair of scissors 10C and the link pieces 65C and 66C relatively pivot in a plane illustrated in Figs. 34 and 35 (in a plane perpendicular to the axial direction of the shaft member 61C) .

The proximal piece 20C of the pair of scissors 10C and the link pieces 65C and 66C configure a four-joint link having a rhomboid shape.

The shaft members 62C and 63C are located on the distal side of the shaft member 64C, and the shaft member 61C is located on the distal side of the shaft members 62C and 63C.

As illustrated in Figs. 39 (a) and 39 (b), a step portion 23C is formed on the outer surface of the proximal piece 20C. In the proximal piece 20C, the proximal side portion of the step portion 23C is thinner than the distal side portion of the step portion 23C.

As illustrated in Fig. 36, in the proximal piece 20C, a thickness difference between the proximal side portion and the distal side portion of the step portion 23C is set to be slightly larger than the thickness of the link pieces 65C and 66C, or is set to be equal to the thickness of the link pieces 65C and 66C.

The holding frame 80C is fixed to the distal end of the sheath 70C.

The holding frame 80C includes a proximal portion 81C fixed to the distal end of the sheath 70C, and a pair of brackets 82C projecting to the distal side from the proximal portion 81C.

For example, each of the pair of brackets 82C is formed in a plate shape.

The pair of scissors 10C is axially supported by the shaft member 61C with respect to the distal portion of the pair of brackets 82C. That is, the shaft member 61C is inserted into the first shaft support hole 21C of each proximal piece 20C of the pair of scissors 10C and the pair of brackets 82C. In this manner, the proximal piece 20C of the pair of scissors 10C is axially supported by the pair of brackets 82C.

In a gap between the pair of brackets 82C, the proximal piece 20C of the pair of scissors 10C and the link pieces 65C and 66C are respectively rotatable. A portion projecting to the distal side from the sheath 70C in the forward and rearward movement portion 67C is movable forward and rearward.

Furthermore, the bracket 82C is rotatable around the axis of the sheath 70C with respect to the proximal portion 81C, or the bracket 82C is rotatable around the axis of the sheath 70C with respect to the sheath 70C.

As illustrated in Fig. 34, when the operation wire 68C and the forward and rearward movement portion 67C are pulled toward the proximal side (rightward in Fig. 34), the pair of scissors 10C is in a closed state. Conversely, as illustrated in Fig. 35, when the operation wire 68C and the forward and rearward movement portion 67C are pushed to the distal side (leftward in Fig. 35), the pair of scissors 10C is in an open state.

An insulating film 12C (non-conductive layer) is formed on each surface of the pair of scissors 10C. The insulating film 12C is formed on the entire surface of the distal piece 30C except for at least the formation region of the electrode region 19C.

For example, the insulating film 12C can be formed by coating the surface of the scissors 10C with an insulating material such as a fluororesin, polyether ether ketone (PEEK), diamond-like carbon (DLC), or a ceramic material (ceramic material such as titanium oxide or silicon).

The electrode region 19C is a line-shaped portion where the insulating film 12C is not formed in the distal piece 30C. The pair of scissors 10C serves as a monopolar high frequency electrode when a high frequency voltage in the same phase is applied thereto from the power supply unit 91C. The high frequency current is applied to the pair of scissors 10C in a state where the biological tissue is gripped by the pair of scissors 10C. In this manner, the biological tissue is cauterized and incised. Instead of the present embodiment, a bipolar high frequency treatment device 200C may be used in which one of the pair of scissors 10C is used as an active electrode and the other is used as a return electrode.

The shapes of the pair of scissors 10C may be the same as each other, or may be different from each other. In a case of the present embodiment, the pair of scissors 10C has mutually the same shape.

Hereinafter, the shape of the scissors 10C will be described in detail with reference to Figs. 37(a) to 40.

As described above, the scissors 10C has a blade surface 13C, a sliding contact surface 14C, an outer surface 15C that is a rear surface with respect to the sliding contact surface 14C, and an inclined surface 16C located between the outer surface 15C and the blade surface 13C (refer to Fig. 40).

For example, the sliding contact surface 14C and the outer surface 15C are located parallel to each other.

In a case of the present embodiment, for example, the blade surface 13C is perpendicular to both the sliding contact surface 14C and the outer surface 15C. That is, the blade surface 13C is located parallel to the axial direction of the shaft member 61C that is the rotary shaft of the scissors 10C.

The inclined surface 16C is inclined with respect to both the blade surface 13C and the outer surface 15C.

In a case of the present embodiment, the inclined surface 16C is configured to include a first inclined surface 161C located on the outer surface 15C side and a second inclined surface 162C located on the blade surface 13C side.

An angle formed between the blade surface 13C and the first inclined surface 161C is larger than an angle formed between the blade surface 13C and the first inclined surface 161C. In addition, an angle formed between the outer surface 15C and the first inclined surface 161C is larger than an angle formed between the outer surface 15C and the second inclined surface 162C.

A distal claw portion 40C is formed in a distal portion (left end portion of the distal piece 30C in Fig. 37(a)) of the distal piece 30C of the scissors 10C. The distal claw portion 40C projects in a closing direction. The closing direction is a direction from one scissors 10C toward the other scissors 10bC, and a direction opposite thereto will be referred to as an opening direction. The distal claw portion 40C projects upward in Fig. 37(a).

The distal claw portion 40C is a projection that is bitten into a biological tissue.

The blade surface 13C is formed along an end edge (edge) on a side in the closing direction in the proximal side portion (right side in Fig. 37(a)) of the distal claw portion 40C in the distal piece 30C, that is, along an upper edge of the distal piece 30C in Fig. 37(a).

In a state where the biological tissue is pinched by the distal claw portion 40C of the pair of scissors 10C to suppress the falling of the biological tissue, the biological tissue can be sheared and incised by the blade surface 13C of the pair of scissors 10C.

In a case of the present embodiment, as illustrated in Fig. 37(a), the scissors 10C includes an intermediate projecting portion 51C projecting toward the other scissors 10C in an intermediate portion in the longitudinal direction of the scissors 10C, and recessed portions 55C and 56C respectively located adjacent to the proximal side and the distal side of the intermediate projecting portion 51C in the longitudinal direction of the scissors 10C and recessed toward a side away from the other scissors. A portion which is located adjacent to the proximal side of the recessed portion 55C on the proximal side and which is in a higher step than the recessed portion 55C will be referred to as a proximal side high step portion 58C.

The distal claw portion 40C, the recessed portion 56C, the intermediate projecting portion 51, the recessed portion 55C, and the proximal side high step portion 58C are located sequentially from the distal side of the distal piece 30C in the end edge on the side in the closing direction of the distal piece 30C.

The blade surface 13C includes a top surface 52C of the intermediate projecting portion 51C and surfaces of the recessed portions 55C and 56C. More specifically, in a case of the present embodiment, the blade surface 13C is continuously formed over the recessed portion 56C, the intermediate projecting portion 51C, and the recessed portion 55C.

The electrode region 19C is formed over the entire region of the blade surface 13C (entire region of the surface of the recessed portion 55C, the top surface 52C of the intermediate projecting portion 51C, and the surface of the recessed portion 56C) .

Each of the distal claw portion 40C, the recessed portion 56C, the intermediate projecting portion 51C, the recessed portion 55C, and the proximal side high step portion 58C has a predetermined width in the thickness direction of the scissors 10C.

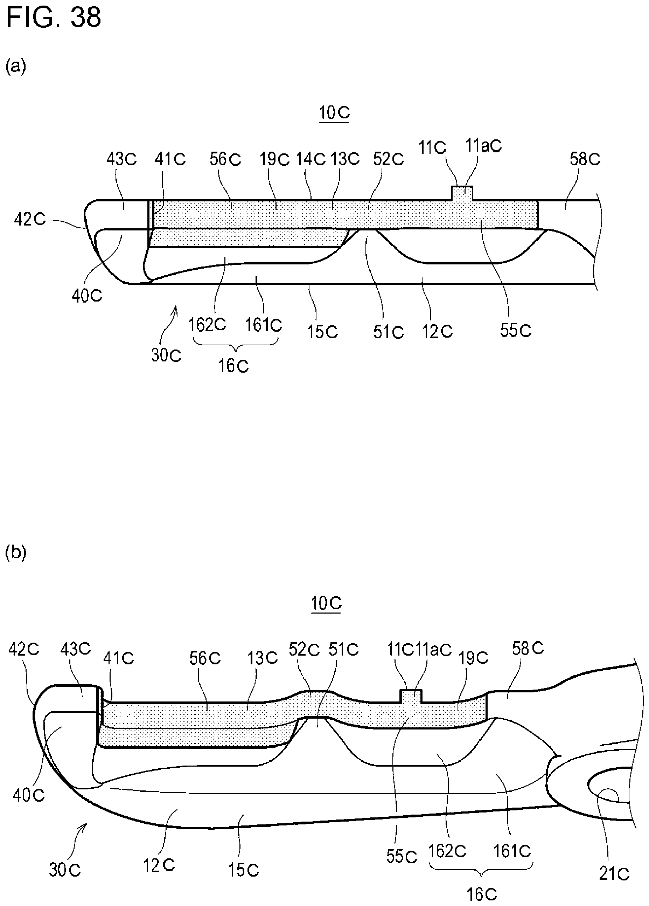

For example, the width dimension of the scissors 10C in the thickness direction is substantially constant in a range extending over the distal claw portion 40C, the recessed portion 56C, the intermediate projecting portion 51C, and the recessed portion 55C (refer to Figs. 38(a) and 38(b)).

Each of the recessed portion 56C and the recessed portion 55C is formed to be elongated in the distal-proximal direction of the distal piece 30C (rightward-leftward direction in Fig. 38(a)).

For example, the blade surface 13C is formed to be flat on each of the bottom surface of the recessed portion 55C, the bottom surface of the recessed portion 56C, and the top surface 52C of the intermediate projecting portion 51C. For example, the bottom surface of the recessed portion 55C, the bottom surface of the recessed portion 56C, and the top surface 52C of the intermediate projecting portion 51C are located substantially parallel to each other. The bottom surface of the recessed portion 56C and the bottom surface of the recessed portion 55C extend in the distal-proximal direction of the distal piece 30C.

Furthermore, the electrode region 19C is also formed on the inclined surface 16C between the recessed portion 56C and the outer surface 15C (refer to Figs. 38(a) to 39(b)). That is, the electrode region 19C is formed on the inclined surface 16C between the recessed portion 56C on the distal side of the scissors 10C from the intermediate projecting portion 51C and the outer surface 15C.

More specifically, for example, out of the inclined surface 16C between the recessed portion 56C and the outer surface 15C, the electrode region 19C is formed in a portion (portion on the recessed portion 56C side) of the second inclined surface 162C on the recessed portion 56C side (blade surface 13C side).

In addition, in a case of the present embodiment, the electrode region 19C is continuously formed over the blade surface 13C and the inclined surface 16C.

The electrode region 19C of the second inclined surface 162C between the recessed portion 56C and the outer surface 15C is continuously located over the entire region in the longitudinal direction of the recessed portion 56C.

On the other hand, the electrode region 19C is not formed on the inclined surface 16C between the recessed portion 55C and the outer surface 15C.

That is, out of the inclined surface 16C on the distal side of the scissors 10C from the top surface 52C of the intermediate projecting portion 51C and the inclined surface 16C on the proximal side of the scissors 10C from the top surface 52C of the intermediate projecting portion 51C, the electrode region 19C is selectively formed on the inclined surface 16C on the distal side of the scissors 10C from the top surface 52C of the intermediate projecting portion 51C.

The distal side portion of the scissors 10C is used to excise the biological tissue by entering a mucous membrane when the biological tissue is excised. Accordingly, this configuration can meet requirements for achieving an advantageous effect of improving hemostatic capability by increasing a current flowing from the electrode region 19C to the biological tissue.

For example, the second inclined surface 162C is formed between the recessed portion 56C and the outer surface 15C and between the recessed portion 55C and the outer surface 15C, and is not formed between the top surface 52C and the outer surface 15C. That is, for example, only the first inclined surface 161C is formed between the top surface 52C and the outer surface 15C.

On the other hand, the first inclined surface 161C is continuously present between the recessed portion 56C and the outer surface 15C, between the top surface 52C and the outer surface 15C, and between the recessed portion 55C and the outer surface 15C.

In addition, the insulating film 12C is formed on the inclined surface 16C on a side surface on the outer surface 15C side of the intermediate projecting portion 51C, and the electrode region 19C is not formed.

That is, the inclined surface 16C is also formed on the intermediate projecting portion 51C. The non-conductive layer (insulating film 12C) is formed on the inclined surface 16C of the intermediate projecting portion 51C, and the electrode region 19C is not formed.

In addition, for example, the insulating films 12C are also respectively formed on a distal surface 42C that is a surface facing the distal side in the distal claw portion 40C, the top surface 43C of the distal claw portion 40C, and the side surface of the distal claw portion 40C. The electrode region 19C is not formed on the distal surface 42C, the top surface 43C, and the side surface of the distal claw portion 40C.

On the other hand, the insulating film 12C is not formed on a proximal surface 41C that is a surface facing the proximal side in the distal claw portion 40C, and the electrode region 19C is formed. The electrode region 19C on the proximal surface 41C is continuous with the electrode region 19C of the recessed portion 56C (refer to Fig. 39(b)).

A stopper portion 11C is formed in at least one scissors 10C of the pair of scissors 10C. A closing operation of the pair of scissors 10C is restricted by the stopper portion 11C coming into contact with the blade surface 13C of the other scissors 10C.

In a case of the present embodiment, the stopper portion 11C is formed in each of the pair of scissors 10C. The stopper portion 11C of the scissors 10aC comes into contact with the blade surface 13C of the scissors 10bC, and the stopper portion 11C of the scissors 10bC comes into contact with the blade surface 13C of the scissors 10aC, thereby restricting the closing operation of the pair of scissors 10C.

The stopper portion 11C is formed on the sliding contact surface 14C illustrated in Fig. 37B, in the scissors 10C. In the stopper portion 11C, a portion facing the other scissors 10C side is a flat surface 11aC.

For example, the stopper portion 11C is located in an intermediate portion in the longitudinal direction of the recessed portion 55C.

For example, the flat surface 11aC is located to be flush with the bottom surface of the recessed portion 55C. Then, when the pair of scissors 10C is closed, the flat surface 11aC comes into surface contact with the bottom surface of the recessed portion 55C of the other scissors 10C, thereby restricting the closing operation of the pair of scissors 10C.

For example, the insulating film 12C is not formed on the flat surface 11aC, and the flat surface 11aC is also a portion of the electrode region 19C.

The flat surface 11aC of the stopper portion 11C is not included in the blade surface 13C.

The insulating film 12C is formed on the entire surface of the distal piece 30C except for the proximal surface 41C of the distal claw portion 40C, the surface of the recessed portion 56C, the top surface 52C of the intermediate projecting portion 51C, the surface of the recessed portion 55C, a portion of the second inclined surface 162C between the recessed portion 56C and the outer surface 15C, and the flat surface 11aC of the stopper portion 11C.

The inclined surface on the distal side in the intermediate projecting portion 51C is assumed to be a portion of the surface of the recessed portion 56C, and the inclined surface on the proximal side in the intermediate projecting portion 51C is assumed to be a portion of the surface of the recessed portion 55C.

According to the ninth embodiment as described above, for at least one of the pair of scissors 10C, the electrode region 19C is formed not only on the blade surface 13C but also on the inclined surface 16C. Therefore, when the biological tissue is incised, a current can be applied to the biological tissue from the electrode region 19C of the inclined surface 16C which comes into contact with the biological tissue together with the blade surface 13C. Accordingly, hemostatic capability for the biological tissue is improved.

Moreover, the outer surface 15C that is likely to touch the biological tissue is covered with the insulating film 12C. Accordingly, the outer surface 15C is sufficiently insulated, and it is possible to preferably suppress a possibility that the biological tissue may be erroneously cauterized by the outer surface 15C.

### [Tenth Embodiment]

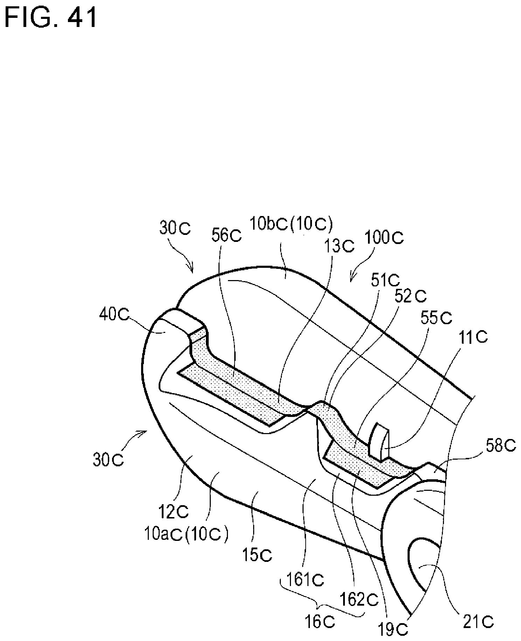

Next, a tenth embodiment will be described with reference to Fig. 41.

A high frequency treatment device according to the present embodiment is different from the high frequency treatment device 200C according to the ninth embodiment in the following points. Other points are configured to be the same as those of the high frequency treatment device 200C according to the above-described ninth embodiment.

In Fig. 41, an electrode formation region (electrode region 19C) in the scissors 10C is hatched in a dot shape. In Fig. 41, a region which is not hatched in the dot shape in the scissors 10C is the formation region of the insulating film 12C (non-conductive layer) . However, the insulating film 12C may not be formed inside the first shaft support hole 21C.

As illustrated in Fig. 41, in a case of the present embodiment, the electrode region 19C is formed on both the inclined surface 16C on the distal side of the scissors 10C from the top surface 52C of the intermediate projecting portion 51C and the inclined surface 16C on the proximal side of the scissors 10C from the top surface 52C of the intermediate projecting portion 51C.

More specifically, not only the electrode region 19C is formed on the second inclined surface 162C between the recessed portion 56C and the outer surface 15C, but also the electrode region 19C is formed on the second inclined surface 162C between the recessed portion 55C and the outer surface 15C.

### [Eleventh Embodiment]

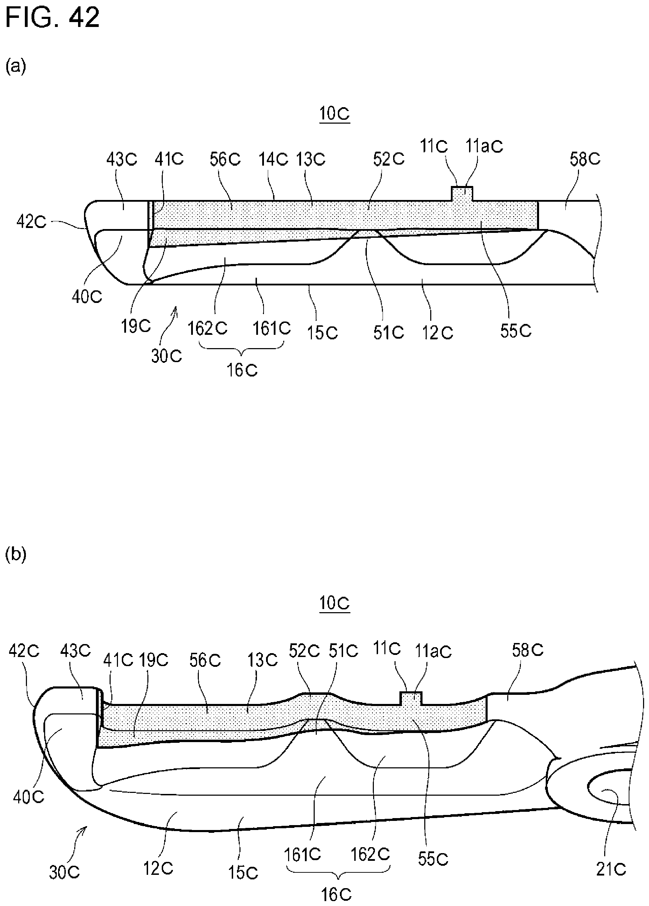

Next, an eleventh embodiment will be described with reference to Figs. 42(a) and 42(b).

A high frequency treatment device according to the present embodiment is different from the high frequency treatment device 200C according to the ninth embodiment in the following points. Other points are configured to be the same as those of the high frequency treatment device 200C according to the above-described ninth embodiment.

In each of Figs. 42(a) and 42(b), an electrode formation region (electrode region 19C) in the scissors 10C is hatched in a dot shape. In each of Figs. 42(a) and 42(b), a region which is not hatched in the dot shape in scissors 10C is the formation region of the insulating film 12C (non-conductive layer). However, the insulating film 12C may not be formed inside the first shaft support hole 21C.

As illustrated in Fig. 42(a), in a case of the present embodiment, the width dimension of the electrode region 19C on the inclined surface 16C is wider toward the distal side of the scissors 10C. The width dimension of the electrode region 19C is the width dimension of the electrode region 19C in a direction parallel to the pivot shaft of the scissors 10C (width dimension of the electrode region 19C in the thickness direction of the scissors 10C).

More specifically, as illustrated in Figs. 42(a) and 42(b), not only the electrode region 19C is formed on the second inclined surface 162C between the recessed portion 56C and the outer surface 15C, but also the electrode region 19C is formed on the inclined surface 16C (first inclined surface 161C) between the top surface 52C and the outer surface 15C and the second inclined surface 162C between the recessed portion 55C and the outer surface 15C.

Hitherto, the ninth to eleventh embodiments have been described with reference to the drawings. However, the embodiments are examples of the sixth aspect of the present invention, and various configurations other than those examples described above can be adopted.

For example, in the ninth to eleventh embodiments, an example has been described in which the electrode region 19C is continuously formed over the blade surface 13C and the inclined surface 16C. However, the sixth aspect of the present invention is not limited to the example, and the electrode region 19C of the blade surface 13C and the electrode region 19C of the inclined surface 16C may be located not to be continuous with each other.

In addition, the ninth to eleventh embodiments can be appropriately combined with each other within the scope not departing from the gist of the sixth aspect of the present invention.

At least one form of the ninth to eleventh embodiments includes the following technical concept.
(1) There is provided a medical high frequency treatment device, a distal portion of which includes a high frequency treatment device knife having a pair of scissors so as to incise a biological tissue.
   Each of the pair of scissors is formed in an elongated plate shape.
   Proximal portions of the pair of scissors are axially supported by each other in a pivot shaft intersecting a plate surface direction of the scissors.
   The pair of scissors is configured to be capable of shearing the biological tissue by pivoting in a direction closer to each other.
   Each of the pair of scissors has a blade surface, a sliding contact surface that comes into sliding contact with each other, an outer surface that is a rear surface with respect to the sliding contact surface, and an inclined surface that is located between the outer surface and the blade surface.
   The inclined surface is inclined from the sliding contact surface side toward the outer surface side in a direction away from the other scissor.
   Each surface of the pair of scissors includes a formation region of a non-conductive layer, and an electrode region where the non-conductive layer is not formed.
   With regard to at least one of the pair of scissors, the electrode region is formed on the blade surface and the inclined surface.
(2) In the high frequency treatment device according to (1), the electrode region is continuously formed over the blade surface and the inclined surface.
(3) In the high frequency treatment device according to (1) or (2),
   the scissors includes an intermediate projecting portion projecting toward the other scissors side in an intermediate portion in the longitudinal direction of the scissors, and
   recessed portions respectively located adjacent to the proximal side and the distal side of the intermediate projecting portion in the longitudinal direction of the scissors and recessed toward a side away from the other scissors.
   The blade surface includes a top surface of the intermediate projecting portion and a surface of the recessed portion.
   The electrode region is formed on the inclined surface between the recessed portion on the distal side of the scissors from the intermediate projecting portion and the outer surface.
(4) In the high frequency treatment device according to (3), out of the inclined surface on the distal side of the scissors from the top surface of the intermediate projecting portion and the inclined surface on the proximal side of the scissors from the top surface of the intermediate projecting portion, the electrode region is selectively formed on the inclined surface on the distal side of the scissors from the top surface of the intermediate projecting portion.
(5) In the high frequency treatment device according to (3), the electrode region is formed on both the inclined surface on the distal side of the scissors from the top surface of the intermediate projecting portion and the inclined surface on the proximal side of the scissors from the top surface of the intermediate projecting portion.
(6) In the high frequency treatment device according to any one of (3) to (5), the inclined surface is also formed in the intermediate projecting portion.
   The non-conductive layer is formed on the inclined surface of the intermediate projecting portion, and the electrode region is not formed.
(7) In the high frequency treatment device according to any one of (1) to (6), the width dimension of the electrode region on the inclined surface is wider toward the distal side of the scissors.

Priority is claimed to Japanese Patent Application No. 2018-076777, filed on April 12, 2018, Japanese Patent Application No. 2017-174238, filed on September 11, 2017, Japanese Patent Application No. 2017-174239, filed on September 11, 2017, and Japanese Patent Application No. 2018-076776, filed on April 12, 2018, the entire content of each of which is incorporated herein by reference.

## Claims

1. A medical high frequency treatment device, a distal portion of which includes a high frequency treatment device knife having a pair of scissors so as to incise a biological tissue,
wherein each of the pair of scissors is formed in an elongated plate shape,
wherein proximal portions of the pair of scissors are axially supported by each other on a pivot shaft intersecting a plate surface direction of the scissors,
wherein the pair of scissors is configured to be capable of shearing the biological tissue by pivoting in a direction closer to each other,
wherein each of the pair of scissors has a blade surface, wherein each surface of the pair of scissors includes a formation region of a non-conductive layer, and an electrode region where the non-conductive layer is not formed on a surface of the blade surface, and
wherein with regard to at least one of the pair of scissors, a width dimension of the electrode region in a plate thickness direction of the scissors varies depending on a position of the scissors in a longitudinal direction.

2. The high frequency treatment device according to claim 1,
wherein the scissors have a projecting portion projecting toward the other scissors side in an intermediate portion in the longitudinal direction of the scissors, and a recessed portion located adjacent to the projecting portion in the longitudinal direction of the scissors, and recessed toward a side away from the other scissors,
wherein the blade surface includes a top surface of the projecting portion and a surface of the recessed portion, and
wherein the electrode regions are respectively formed on the top surface of the projecting portion and the surface of the recessed portion.

3. The high frequency treatment device according to claim 2,
wherein the recessed portion is recessed in an arc shape.

4. The high frequency treatment device according to claim 2 or 3,
wherein in addition to the blade surface, each of the pair of scissors has a sliding contact surface that comes into sliding contact with each other, and an outer surface that is a rear surface with respect to the sliding contact surface, and
wherein the surface of the recessed portion is inclined from the sliding contact surface side to the outer surface side in a direction away from the other scissors.

5. The high frequency treatment device according to claim 3 or 4,
wherein the scissors have a plurality of recessed portions located with the projecting portion as a boundary, and
wherein the recessed portion on the distal side has a larger width dimension of the electrode region.

6. The high frequency treatment device according to any one of claims 2 to 5,
wherein the scissors have a plurality of the projecting portions located at mutually different positions in the longitudinal direction of the scissors, and
wherein widths of the electrode regions on the top surfaces of the plurality of projecting portions are equal to each other.

7. The high frequency treatment device according to any one of claims 2 to 6,
wherein a width of the electrode region on the top surface of the projecting portion is equal to or smaller than a width of a narrowest portion in the electrode region other than the projecting portion.

8. The high frequency treatment device according to any one of claims 1 to 5,
wherein the electrode region is formed to be wider toward the distal side of the scissors.

9. The high frequency treatment device according to claim 8,
wherein the scissors have a projecting portion projecting toward the other scissors side in an intermediate portion in the longitudinal direction of the scissors, and
wherein the electrode region is formed to be wider in a distal side portion of the projecting portion than in a proximal side portion of the projecting portion on the blade surface.

10. The high frequency treatment device according to claim 9,
wherein the scissors have a plurality of the projecting portions located at mutually different positions in the longitudinal direction of the scissors, and
wherein the electrode region is formed to be wider toward the distal side in a stepwise manner from each of the plurality of projecting portions serving as a boundary.

11. The high frequency treatment device according to claim 9 or 10,
wherein the electrode region is wider toward the distal side on the surface of the projecting portion.

12. The high frequency treatment device according to any one of claims 1 to 11,
wherein the blade surface is wider on the distal side of the scissors than on the proximal side, and
wherein the electrode region is wider in a relatively wide portion on the blade surface than in a relatively narrow portion on the blade surface.

13. The high frequency treatment device according to claim 12,
wherein the scissors have a projecting portion projecting toward the other scissors side in an intermediate portion in the longitudinal direction of the scissors, and
wherein the blade surface is formed to be wider in a distal side portion of the projecting portion than in a proximal side portion of the projecting portion.

14. The high frequency treatment device according to claim 13,
wherein the width dimensions of the blade surfaces are respectively and substantially constant in the distal side portion of the projecting portion and in the proximal side portion of the projecting portion.

15. The high frequency treatment device according to claim 12 or 13,
wherein the width dimension of the blade surface is changed to be continuously wider from the proximal side toward the distal side of the scissors.

16. A high frequency treatment device knife disposed in a distal portion of a medical high frequency treatment device, and used by being inserted into a forceps hole of an endoscope so as to incise a biological tissue, the knife comprising:
a pair of shearing scissors axially supported by a common rotary shaft, capable of opening and closing each other, and each having a blade portion for shearing the biological tissue,
wherein each of the pair of shearing scissors has
a proximal piece formed on a proximal side of the shearing scissors and axially supported by the rotary shaft,
a distal claw portion formed in a distal end of the shearing scissors, and
a blade portion formed between the distal claw portion and the proximal piece in the shearing scissors,
wherein an electrode is formed in the blade portion, and
wherein based on a virtual straight line connecting an axis center of the rotary shaft and a bottom of the blade portion to each other, a height of a highest position of the formation region of the electrode in the blade portion is lower than a height of the distal claw portion.

17. The high frequency treatment device knife according to claim 16,
wherein when the high frequency treatment device knife is viewed in the axial direction of the rotary shaft, an angle formed between a proximal side edge side in the distal claw portion and the virtual straight line is equal to or smaller than 90 degrees.

18. The high frequency treatment device knife according to claim 16 or 17,
wherein the distal portion and the proximal portion in a projecting direction end edge of the distal claw portion respectively have an R-chamfered shape.

19. The high frequency treatment device knife according to any one of claims 16 to 18,
wherein a stopper portion is formed in at least one of the pair of shearing scissors, and
wherein a closing operation of the pair of shearing scissors is restricted by the stopper portion coming into contact with the blade portion of the other shearing scissor.

20. The high frequency treatment device knife according to any one of claims 16 to 19,
wherein the blade portion has a high step portion and a low step portion having a notch shape recessed toward a rear side of the high step portion,
wherein an electrode is formed over the low step portion from the high step portion, and
wherein the bottom of the blade portion is a lowest portion of the low step portion.

21. The high frequency treatment device knife according to claim 20,
wherein the blade portion has a plurality of the low step portions and an intermediate high step portion that is the high step portion located between the low step portions.

22. The high frequency treatment device knife according to claim 21,
wherein the blade portion has a plurality of the intermediate high step portions, and
wherein out of the plurality of intermediate high step portions, the intermediate high step portion located on the proximal side has a lower height based on the virtual straight line.

23. The high frequency treatment device knife according to claim 21 or 22,
wherein when the pair of shearing scissors is closed and the distal claw portions of the pair of shearing scissors start to overlap each other in the axial direction of the rotary shaft, the mutually corresponding intermediate high step portions of the pair of shearing scissors come into contact with each other.

24. The high frequency treatment device knife according to any one of claims 21 to 23,
wherein when the high frequency treatment device knife is viewed in the axial direction of the rotary shaft, an angle formed between a proximal side edge side in the intermediate high step portion and the virtual straight line is equal to or smaller than 90 degrees.

25. The high frequency treatment device knife according to any one of claims 21 to 24,
wherein when the high frequency treatment device knife is viewed in the axial direction of the rotary shaft, an angle formed between a distal side edge side in the intermediate high step portion and the virtual straight line is equal to or smaller than 90 degrees.

26. The high frequency treatment device knife according to any one of claims 21 to 25,
wherein the distal portion and the proximal portion in a projecting direction end edge of the intermediate high step portion respectively have an R-chamfered shape.

27. The high frequency treatment device knife according to any one of claims 16 to 26, further comprising:
a pair of brackets that pinches the proximal piece of the pair of shearing scissors from both sides in the axial direction of the rotary shaft, and that axially supports the proximal piece in the rotary shaft,
wherein the pair of brackets pinches and axially supports the proximal piece in the distal portion of the bracket,
wherein in each distal portion of the pair of brackets, an outer surface that is a rear surface with respect to each facing surface of the pair of brackets is formed to be flat, and
wherein a distance between the outer surfaces of the respective distal portions of the pair of brackets is shorter than a distance between the outer surfaces of the proximal portions of the brackets.

28. A medical high frequency treatment device, a distal portion of which has the high frequency treatment device knife according to any one of claims 16 to 27, and a proximal side of which has an operation unit for performing an opening and closing operation on the pair of shearing scissors.

29. A high frequency treatment device distal treatment instrument disposed in a distal portion of a medical high frequency treatment device, and used by being inserted into a forceps hole of an endoscope so as to incise a biological tissue, the instrument comprising:
a distal treatment unit having a pair of opening and closing portions each having a line-shaped electrode, axially supported by a common rotary shaft, capable of opening and closing each other, performing high frequency excision by shearing or pinching the biological tissue,
wherein in a state where the pair of opening and closing portions is closed, a shape of a distal side portion of the distal treatment unit when viewed in an axial direction of the rotary shaft is a shape which is narrowed after being widened from a distal end toward a proximal end.

30. The high frequency treatment device distal treatment instrument according to claim 29,
wherein the distal side portion of the distal treatment unit is a distal side portion from a proximal end of a formation region of the electrode.

31. The high frequency treatment device distal treatment instrument according to claim 29 or 30,
wherein the distal side portion of the distal treatment unit is a distal side portion of an intermediate position of the distal treatment unit in a distal-proximal direction.

32. The high frequency treatment device distal treatment instrument according to any one of claims 29 to 31, further comprising:
a pair of brackets that pinches the proximal portion of the pair of opening and closing portions from both sides in the axial direction of the rotary shaft, and that axially supports the proximal portion in the rotary shaft,
wherein in a state where the pair of opening and closing portions is closed, a dimension of the proximal portion of the distal treatment unit is smaller than a dimension of the bracket in a direction perpendicular to both the distal-proximal direction and the axial direction.

33. The high frequency treatment device distal treatment instrument according to any one of claims 29 to 32,
wherein a boundary portion between an edge portion on a side in an opening direction of the pair of opening and closing portions and an outer surface of the pair of opening and closing portions has a chamfered shape.

34. The high frequency treatment device distal treatment instrument according to any one of claims 29 to 33, further comprising:
a pair of brackets that pinches the proximal portion of the pair of opening and closing portions from both sides in the axial direction of the rotary shaft, and that axially supports the proximal portion in the rotary shaft,
wherein the pair of brackets pinches and axially supports the pair of opening and closing portions in the distal portion of the brackets,
wherein in each distal portion of the pair of brackets, an outer surface that is a rear surface with respect to each facing surface of the pair of brackets is formed to be flat, and
wherein a distance between the outer surfaces of the respective distal portions of the pair of brackets is shorter than a distance between the outer surfaces of the proximal portions of the brackets.

35. A medical high frequency treatment device, a distal portion of which has a high frequency treatment device distal treatment instrument according to any one of 29 to 34, and a proximal side of which has an operation unit for performing an opening and closing operation on a pair of opening and closing portions.

36. A medical high frequency treatment device, a distal portion of which includes a high frequency treatment device knife having a pair of scissors and used to incise a biological tissue,
wherein each of the pair of scissors is formed in an elongated plate shape,
wherein proximal portions of the pair of scissors are axially supported by each other on a pivot shaft intersecting a plate surface direction of the scissors,
wherein the pair of scissors is configured to be capable of shearing the biological tissue by pivoting in a direction closer to each other,
wherein each of the pair of scissors has a blade surface, a sliding contact surface that comes into sliding contact with each other, an outer surface that is a rear surface with respect to the sliding contact surface, and an inclined surface that is located between the outer surface and the blade surface,
wherein the inclined surface is inclined from the sliding contact surface side toward the outer surface side in a direction away from the other scissor,
wherein each surface of the pair of scissors includes a formation region of a non-conductive layer, and an electrode region where the non-conductive layer is not formed, and
wherein with regard to at least one of the pair of scissors, the electrode region is formed on the blade surface and the inclined surface.

37. The high frequency treatment device according to claim 36,
wherein the electrode region is continuously formed over the blade surface and the inclined surface.

38. The high frequency treatment device according to claim 36 or 37,
wherein the scissors have
an intermediate projecting portion projecting toward the other scissors side in an intermediate portion in the longitudinal direction of the scissors, and
recessed portions respectively located adjacent to the proximal side and the distal side of the intermediate projecting portion in the longitudinal direction of the scissors and recessed toward a side away from the other scissors,
wherein the blade surface includes a top surface of the intermediate projecting portion and a surface of the recessed portion, and
wherein the electrode region is formed on the inclined surface between the recessed portion on the distal side of the scissors from the intermediate projecting portion and the outer surface.

39. The high frequency treatment device according to claim 38,
wherein out of the inclined surface on the distal side of the scissors from the top surface of the intermediate projecting portion and the inclined surface on the proximal side of the scissors from the top surface of the intermediate projecting portion, the electrode region is selectively formed on the inclined surface on the distal side of the scissors from the top surface of the intermediate projecting portion.

40. The high frequency treatment device according to claim 38,
wherein the electrode region is formed on both the inclined surface on the distal side of the scissors from the top surface of the intermediate projecting portion and the inclined surface on the proximal side of the scissors from the top surface of the intermediate projecting portion.

41. The high frequency treatment device according to any one of claims 38 to 40,
wherein the inclined surface is also formed in the intermediate projecting portion, and
wherein the non-conductive layer is formed on the inclined surface of the intermediate projecting portion, and the electrode region is not formed.

42. The high frequency treatment device according to any one of claims 36 to 41,
wherein the width dimension of the electrode region on the inclined surface is wider toward the distal side of the scissors.
